# EUROPEAN PATENT SPECIFICATION

(11) **EP 2 449 103 B1**
(45) Date of publication and mention of the grant of the patent: **03.08.2016**
(21) Application number: 10800274.2
(22) Date of filing: 28.06.2010
(51) Int. Cl.: C12N 15/10, C12N 15/12, C07K 14/725, C12Q 1/68

(54) **ISOLATION OF UNKNOWN REARRANGED T-CELL RECEPTORS FROM SINGLE CELLS**
ISOLIERUNG VON UNBEKANNTEN NEU ANGEORDNETEN T-ZELLEN-REZEPTOREN AUS EINZELNEN ZELLEN
ISOLEMENT DE RÉCEPTEURS DE LYMPHOCYTES T RÉAGENCÉS INCONNUS À PARTIR DE CELLULES UNIQUES

(30) Priority: 29.06.2009 US 221505 P
(43) Date of publication of application: 09.05.2012
(73) Proprietor: California Institute of Technology, Pasadena, CA 91125 (US)
(72) Inventor: BALAZS, Alejandro, Benjamin, Berkeley CA 94707 (US); TSAI, Jonathan, Michael, Saratoga CA 95070 (US); BALTIMORE, David, Pasadena CA 91106 (US)
(74) Representative: Beck Greener
(86) International application number: PCT/US2010/040245
(87) International publication number: WO 2011/008502

(56) References cited:
- KR-A- 20050 034 724
- US-A- 5 840 304
- US-A- 6 114 516
- US-A1- 2002 110 807
- US-B2- 7 294 712
- DONGEN VAN J J M ET AL: "Design and standardization of PCR primers and protocols for detection of clonal immunoglobulin and T-cell receptor gene recombinations in suspect lymphoproliferations: Report of the BIOMED-2 Concerted Action BMH4-CT98-3936", LEUKEMIA, MACMILLAN PRESS LTD, US, vol. 17, no. 12, 1 December 2003 (2003-12-01), pages 2257-2317, XP008093070, ISSN: 0887-6924, DOI: 10.1038/SJ.LEU.2403202
- S. SEITZ ET AL: "Reconstitution of paired T cell receptor - and beta-chains from microdissected single cells of human inflammatory tissues", PROCEEDINGS OF THE NATIONAL ACADEMY OF SCIENCES, vol. 103, no. 32, 31 July 2006 (2006-07-31), pages 12057-12062, XP55013528, ISSN: 0027-8424, DOI: 10.1073/pnas.0604247103
- YUAN OU ET AL: "An improved design of PCR primers for detection of human T cell receptor Î chain repertoire", MOLECULAR BIOLOGY REPORTS ; AN INTERNATIONAL JOURNAL ON MOLECULAR AND CELLULAR BIOLOGY, KLUWER ACADEMIC PUBLISHERS, DO, vol. 36, no. 1, 21 August 2008 (2008-08-21), pages 145-152, XP019647230, ISSN: 1573-4978
- BORIA ILENIA ET AL: "Primer sets for cloning the human repertoire of T cell Receptor Variable regions", BMC IMMUNOLOGY, BIOMED CENTRAL, LONDON, GB, vol. 9, no. 1, 29 August 2008 (2008-08-29) , page 50, XP021042325, ISSN: 1471-2172, DOI: 10.1186/1471-2172-9-50
- MOYSEY R ET AL: "Amplification and one-step expression cloning of human T cell receptor genes", ANALYTICAL BIOCHEMISTRY, ACADEMIC PRESS INC, NEW YORK, vol. 326, no. 2, 15 March 2004 (2004-03-15), pages 284-286, XP004493127, ISSN: 0003-2697, DOI: 10.1016/J.AB.2003.12.011
- JUNKER ANDREAS ET AL: "Multiple sclerosis: T-cell receptor expression in distinct brain regions", BRAIN, vol. 130, no. Part 11, November 2007 (2007-11), pages 2789-2799, XP002698294, ISSN: 0006-8950
- VERGIER B ET AL: "Combined analysis of T cell receptor gamma and immunoglobulin heavy chain gene rearrangements at the single-cell level in lymphomas with dual genotype", JOURNAL OF PATHOLOGY, vol. 198, no. 2, October 2002 (2002-10), pages 171-180, XP002691561, ISSN: 0022-3417

## Description

### BACKGROUND OF THE INVENTION

### Field of the Invention

The human immune system is comprised of innate and adaptive mechanisms that clear foreign particles from the body. The adaptive immune response includes the humoral response, which involves B lymphocytes and antibody secretion, and a cell-mediated response, which involves T lymphocytes. The cell-mediated immune response functions by activating macrophages, natural killer cells, and CD8+ cytotoxic T-lymphocytes (CTLs) that act to destroy pathogens, as well as CD4+ helper T-cells that activate the humoral immune response. The specificity of the cell-mediated response comes from the T-cell receptors (TCRs) found on the T-cell surface. These TCRs recognize a specific combination of antigen and major histocompatibility complex (MHC) molecules and trigger T-cell function. TCR recognition on CD8+ CTLs can lead to the induction of apoptosis of the target cell, and the initiation of the humoral immune response from CD4+ T-helper cells.

A single TCR consists of two unique peptide chains (alpha and beta), each of which is produced by a genomic recombination of two segments known as variable and joining regions (V and J, respectively). A TCR includes one of 44 alpha variable (V) regions, 76 alpha junctional (J) regions, 54 beta V regions, and 14 beta J regions. Thus, amplification of this region is complex, and requires at least 188 unique oligonucleotides. There are 3344 different alpha chain combinations and 216 beta combinations, leading to 722,304 possible chain pairings. This diversity is greatly amplified by additional mutagenesis occurring from DNA repair following the RAG1-RAG2 recombination process. As a consequence, simply identifying the different V and J regions is not sufficient information to isolate a functional TCR.

This complexity and variability make difficult the isolation of an unknown TCR from an inhomogeneous population of cells. Isolation of the TCR from a single cell has been suggested as a high throughput solution to the problem of alpha-beta mispairing. However, unlike plasma cells, T-cells do not express high transcript levels of TCR, making it difficult to isolate sufficient cDNA template for reliable amplification.

Because there are 722,304 possible chain pairings, previous protocols have not permitted the isolation of a TCR sequence using a single PCR reaction using all 188 oligonucleotides simultaneously. Instead, prior art methods have utilized an array of simultaneous PCR reactions, each using a different, restricted pool of primers, followed by additional iterations of PCR using progressively smaller pools of primers.

### SUMMARY OF THE INVENTION

Methods for isolating and identifying the sequence of the recombinant region of the alpha and/or beta chains of a T cell receptor (TCR) from a single T cell are provided.

In one aspect, methods of isolating DNA encoding the variable regions of a T cell receptor (TCR) alpha or beta chain are provided. The methods comprise isolating genomic DNA from a single T cell, wherein said isolating step comprises whole genome amplification, and then amplifying a gene segment encompassing the TCR alpha or beta chain variable region by an enrichment amplification reaction to produce a first enrichment product. The first enrichment product comprises the V and J regions of the TCR alpha or beta chain from the single cell. The amplification reaction comprises incubating the isolated genomic DNA with a set of forward outer primers and a set of outer reverse primers, wherein each outer forward primer anneals to a sequence 5 to 40 base pairs upstream of the signal sequence junction of the V region, the set of outer forward primers comprising at least one primer for each of the possible V regions of the TCR alpha chain or TCR beta chain, and wherein each outer reverse primer anneals to a sequence 5 to 40 base pairs downstream of the exon/intron junction of the J region, the set of outer reverse primers comprising at least one primer for each of the possible J regions of the TCR alpha chain or TCR beta chain. The amplification reaction may be a PCR reaction, such as a touchdown PCR reaction.

In some embodiments the first enrichment product is further amplified in an isolation amplification reaction to produce a second isolation product. The isolation amplification reaction may comprise incubating the first enrichment product with a set of inner forward primers and a set of inner reverse primers, wherein each inner forward primer anneals to a sequence at the start of the first amino acid downstream of the signal sequence of the V region, the set of inner forward primers comprising at least one primer for each of the possible V regions of the TCR alpha chain or TCR beta chain, and wherein each inner reverse primer anneals to a sequence at the downstream end of the J region, the set of inner reverse primers comprising at least one primer for each of the possible J regions of the TCR alpha chain or TCR beta chain. The isolation product may be further amplified in a cloning amplification reaction to facilitate cloning into a vector. Further the product of the cloning amplification reaction may be further amplified in a homologous amplification reaction to increase yield of the desired product.

### BRIEF DESCRIPTION OF THE DRAWINGS

Figure 1 is a representation of the structure of the genes encoding the TCR alpha and beta chains. The relative positions of outer, inner, cloning, and homologous primer annealing sites are also shown.
Figure 2 shows the results of Jurkat cell TCR-specific PCR with or without WGA. Genomic DNA was isolated from Jurkat cells diluted from 10,000 to single cells. A Jurkat TCR alpha chain-specific PCR was run on samples with and without WGA. Alpha chains were isolated from single genomes only when these genomes were WGA-amplified.
Figure 3 shows the results of the design and testing of primers against all alpha and beta V and J regions in the genome. Figure 3a is the relative positions of inner and outer primers. Figure 3b is a test of the ability of each of the oligonucleotides from 3a to function under identical PCR conditions.
Figure 4 illustrates the results of Jurkat TCR PCR with increasing numbers of oligonuclotides in the pool. Figure 4a shows the number of Forward/Reverse primers in each pool for TCR alpha chain-specific PCR. Figure 4b shows the number of Forward/Reverse primers in each pool for TCR beta chain-specific PCR.
Figure 5 shows the isolation of melanoma-specific T cells. Three alpha-beta pairs were isolated from a strip of eight NY-ESO+ T-cells. Cells were obtained from a patient sample and stained with an NY-ESO specific tetramer.
Figure 6 shows the isolation of NA-17 specific T cells. Four alpha chains and five beta chains were isolated from a strip of eight NA-17+ T-cells. Cells were obtained from a patient sample and stained with an NA-17specific tetramer.

### DETAILED DESCRIPTION OF THE INVENTION

Methods are provided for identifying the alpha and beta chains of specific TCRs from individual T-cells. A single T cells is isolated and the genomic DNA of that T cell is amplified using whole genome amplification (WGA). An optional screening assay, for example a PCR-based assay, can be used to screen the product of the WGA to verify that genomic DNA encoding the T cell receptor is present. This can be accomplished, for example, by amplifying constant regions of the T cell receptor. If the appropriate genomic DNA is present, separate amplification reactions for the alpha and beta chain of the TCR are then performed. Several rounds of PCR may be carried out using different primer sets, as described in detail below, in order to isolate and amplify the DNA encoding the alpha and beta chains of the TCR. Generally, the product of each round of PCR is used as the template for the subsequent round of PCR.

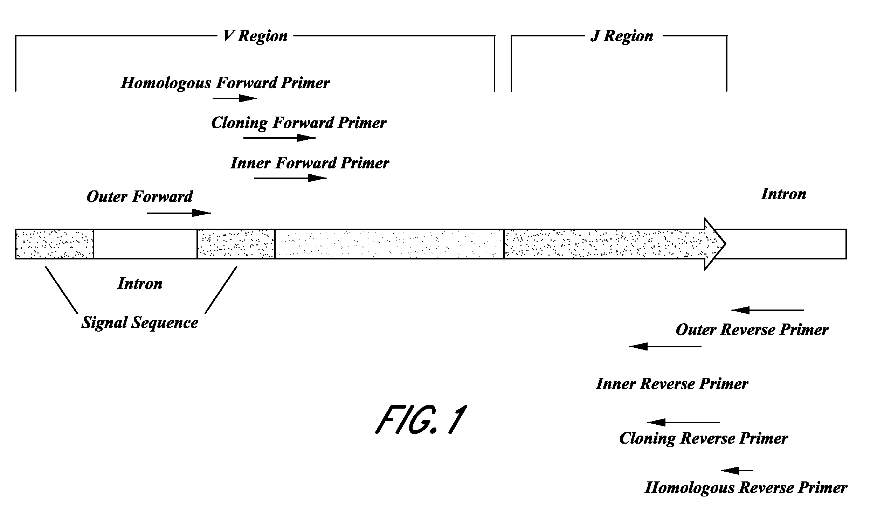

Figure 1 summarizes various sets of primers that can be used in each round of amplification. The first round of amplification is typically an enrichment round in which outer forward and reverse primers are used on amplified genomic DNA to enrich for DNA sequences encompassing the TCR alpha or beta chain. The second round of PCR is an isolation round. Inner forward and reverse primers are used on the enriched product of round 1 to isolate TCR sequences from non-specific product. An optional third round of PCR is a cloning round. Cloning primers that overlap the inner primers are used to generate TCR sequences that contain ends with homology to a cloning vector, which may also function as an expression vector. An optional fourth round of PCR is a homologous "clean-up" round. Homologous primers are used to isolate TCR sequences with vector homology on their ends from non-specific product.

Following isolation, the amplified nucleic acid can be sequenced to determine the identity of the alpha and beta chains of the TCR from the single isolated T-cell. Furthermore, it can cloned into an expression vector for subsequent expression in a cell of interest.

### Analysis of Populations of T Cells

In some embodiments, TCR sequences are isolated from a single T-cell that has itself been isolated from a population of T Cells. The population of T cells may be selected for a certain activity or set of activities, and TCRs isolated from one or more individual cells in the population. For example, populations of T cells with specificity for disease antigens such as melanoma antigens (for example MART1, NY-ESO, NA-17, GP-100, or Tyrosinase) can be isolated using FACs of patient T cells stained with tetramers for these antigens (see Example 3). In some embodiments, the population of T cells is a subpopulation of T cells with a certain activity; the subpopulation may be selected using an activity, a marker, and/or a set of markers.

Individual T cells are isolated from the population and the TCR alpha and beta chain sequences of the individual T cells are separately isolated by PCR. By way of example, a single cell may be isolated from tissue, from bodily fluid, or from cell culture. Examples of methods for isolating T Cells include Fluorescence Activated Cell Sorting (FACs), and serial dilutions. Other methods are known in the art.

### Whole Genome Amplification:

Once an individual T-cell has been isolated, whole genome amplification (WGA) may be carried out. WGA has been used in a variety of applications, for example, large-scale genotyping (e.g. SNP typing, RFLP analysis), comparative genome hybridization (e.g. Southern blotting), and molecular cloning. In the present embodiments, WGA is used to amplify the genomic DNA of the single isolated T cell.

One skilled in the art will appreciate that any number of WGA methods or kits can be used to perform the WGA step. In some embodiments, a PCR-based method of WGA is used (See, e.g. Zhang et al (1992). Proc Natl Acad Sci USA 89: 5847-51). In other embodiments, an Omniplex method of WGA is used (Langmore, J.T. (2002). Genome Res. 14: 901-07). Other methods of amplification of genomic DNA known in the art may be used.

In some embodiments, a multiple displacement amplification (MDA) method of WGA is used: Briefly, DNA synthesis is initiated by the addition of random hexamers to DNA, which prime the reaction. The Phi29 polymerase is used to elongate from each hexamer and continues until it reaches a downstream synthesis reaction. The upstream reaction then displaces the downstream reaction and continues replication and displacement. Additional priming and synthesis is able to occur on the displaced strands in a branching pattern, allowing for mass amplification from low levels of template. Phi29 is a highly processive enzyme, able to replicate long stretches of DNA (up to 100kb). The polymerase also contains a 3' to 5' proofreading mechanism, making it 100 times more accurate than conventional PCR enzymes such as Taq (Telenius, H et al (1992). Genomics 13: 718-25). Dean et al ((2002). Proc. Natl. Acad. Sci. USA 99: 5261-66) disclose a method of MDA which comprises an isothermal strand-displacing reaction, that can amplify 1-10 copies of human genomic DNA to produce 20-30 µg of product. MDA may be used to amplify DNA from crude sources, for example whole blood cells or whole tissue culture cells (*Id.* at 5263). Dean et al report that relative to PCR-based WGA methods, MDA increases genomic coverage, reduces amplification bias, and generates longer products (>10kb for MDA versus ~1kb for PCR-based amplification) (*Id.* at 5265-66).

One skilled in the art will appreciate that in some embodiments, the invention can comprise a method of WGA other than PCR, MDA, or Omniplex.

In the some embodiments, after a single T cell is isolated, the cell is lysed. The lysis is performed using methods known in the art. For example, each cell can be sorted into 1.5ul of alkaline lysis buffer (ALB), incubated at -20°C for 30 minutes, and then incubated at 65°C for 10 minutes to lyse that cell. After lysis, WGA is performed using a commercial kit, such as a Qiagen Repli-g Midi kit (catalog number 150043), which comprises an MDA method of WGA (See Qiagen "REPLI-g® Mini/Midi Handbook" Feb. 2008).

### Screening of WGA products:

Following WGA, or other amplification of genomic DNA, a screening assay is performed on the amplification products to verify that the appropriate genomic DNA was amplified and that the reaction was not contaminated. For example, such a screening assay can verify there was no bacterial contamination in the WGA reaction. In some embodiments, the screening assay comprises a PCR reaction. In some embodiments, the PCR primers are directed against a known un-rearranged human gene, for example ataxia telangiectasia mutated (ATM) (see Example 2). In other embodiments, the screening PCR primers are directed to the constant regions of the alpha and beta chains of the TCR. For example, the screening primers can comprise the primers described in Table 1. In the presence of amplified genomic DNA template, the sizes of the alpha and beta constant region products generated by these primers are 420bp and 530bp respectively. Other primer combinations can also be used to identify the presence of the TCR constant regions.

| **SEQ ID NO:** | **Table 1: Example Constant Region Diagnostic Primer Sequences** | |
|---|---|---|
| | **Primer** | **Sequence** |
| 1 | Fwd TCR-Alpha Constant Screening | CAGAACCCTGACCCTGCCGTGTACC |
| 2 | Rev TCR-Alpha Constant Screening | GCCATTCCTGAAGCAAGGAAACAGC C |
| 3 | Fwd TCR-Beta Constant Screening | GGCCACACTGGTGTGCCTGGCC |
| 4 | Rev TCR-Beta Constant Screening | CGGCGCTGACGATCTGGGTGAC |

In some embodiments, other techniques may be used to perform the screening assay on WGA amplification products, for example DNA comparative genome hybridization such as microarray screening or Southern blotting.

### Design of primers for isolating TCR alpha and beta chains:

The generalized structure of the gene encoding the TCR receptor alpha or beta chains is depicted in Figure 1. In some embodiments, the alpha and beta chains of the TCR are isolated using a series of amplification reactions, preferably PCR reactions, comprising two or more sets of nested primers. In order to identify both the alpha and beta chains, two series of amplifications may be performed in parallel-one for the alpha chain and one for the beta chain-to isolate the genes encoding the TCR alpha and beta chains of a single T cell. In other embodiments, the TCR alpha or beta chain may be isolated individually or in sequence.

In some embodiments, each series of amplifications comprises two rounds of amplification reactions: First, parallel enrichment reactions are performed to enrich previously-amplified genomic DNA for sequences encompassing the variable regions of the TCR alpha and beta chains. If both the TCR alpha and beta regions are to be identified, one reaction is carried out using primers to enrich for sequences encoding the variable region of the TCR alpha chain and a separate reaction is carried out to enrich for sequences encoding the variable region of the TCR beta chain. These may be referred to as the TCR alpha enrichment reaction and the TCR beta enrichment reaction, respectively. In some embodiments these reactions are carried out in parallel. The enrichment reactions comprise forward and reverse primers that anneal to substantially all variable regions of the TCR alpha or beta chain. That is, in each enrichment reaction, the genomic DNA is incubated with a set of primers comprising at least one primer complementary to each of the V and J regions of the TCR alpha chain or TCR beta chain. The primers for this step are referred to as outer primers (Fig. 1) and are described in more detail below.

Second, an isolation reaction is performed on the product of the enrichment reactions to isolate sequence encoding the variable region of the TCR alpha and beta chains. These reactions may be referred to as the TCR alpha isolation reaction and TCR beta isolation reaction, respectively. As with the enrichment reactions, each of the isolation reactions may be performed using a set of primers that anneal to substantially all variable regions of the TCR alpha or beta chain. Thus, the product of the enrichment reaction may be incubated with a set of forward and reverse primers comprising at least one primer complementary to each of the V and J regions of the TCR alpha chain or TCR beta chain. In other embodiments a subset of primers is utilized. The primers for this step are referred to as inner primers (Fig. 1) and are described in more detail below.

In some embodiments, the amplification reaction series also comprises a third cloning amplification reaction, such as a PCR reaction, in which end sequences with homology to a cloning vector are added to the isolated TCR alpha and/or beta chain sequence. These reactions may be referred to as the TCR alpha cloning reaction and TCR beta cloning reaction, respectively. As with the enrichment and isolation reactions, each of the cloning reactions may be performed using a set of primers that anneal to substantially all variable regions of the TCR alpha or beta chain. Thus, the product of the isolation reaction may be incubated with a set of forward and reverse primers comprising at least one primer complementary to each of the V and J regions of the TCR alpha chain or TCR beta chain, where the primers additionally comprise sequences with homology to a cloning vector. In other embodiments a subset of primers is utilized. The primers for this step are referred to as cloning primers (Fig. 1) and are described in more detail below.

In some embodiments, the amplification reaction series also comprises a fourth homologous amplification reaction, such as a fourth PCR reaction, to further isolate and amplify DNA segments comprising end sequences homologous to a cloning vector. This reaction may be referred to as the homologous reaction. The primers used in the homologous reaction are preferably homologous to the cloning vector sequences. The product of the cloning reaction is incubated with these forward and reverse primers. The primers for this step are referred to as homologous primers (Fig. 1) and are described in more detail below.

In some embodiments, the product of the isolation round using the inner primers is sequenced directly. In some embodiments, the product of the isolation round using the inner primers is cloned into a vector, for example TOPO-TA or TOPO-Blunt.

In other embodiments, the product of the amplification using the cloning primers is sequenced. This product may be cloned into a vector, for example an expression vector.

In other embodiments, the product of the amplification using the homologous primers is sequenced. It may be cloned into a vector, for example an expression vector.

Primers may be designed so that each set of inner and outer primers for the alpha chain contains at least one primer that anneals to each of the 44 possible alpha variable ("alpha V") regions and 76 possible alpha junctional ("alpha J") regions. Similarly, each set of inner and outer primers for the beta chain preferably contains at least one primer that anneals to each of the 54 possible beta variable ("beta V") regions and the 14 possible beta junctional ("beta J") regions. In some of these embodiments, one primer may anneal to two or more different possible regions. In some embodiments, the sequence of the primers is selected based on their predicted annealing temperatures to genomic sequences within the TCR gene.

*Alpha chain primers.* In some embodiments, the primer sets for isolating sequence encoding the TCR alpha chain comprise outer primers and inner primers, as described herein; in other embodiments the primers sets comprise outer primers, inner primers and cloning primers, as described herein; in still other embodiments, the primer sets comprise outer primers, inner primers, cloning primers and homologous primers, as described herein:

Outer primers: Outer primers comprise forward and reverse outer primers (Fig. 1), in order to amplify the V and J regions. Each outer forward primer anneals to sequence 5-40 base pairs upstream of the signal sequence junction of the alpha V region. In these embodiments, the outer primer set comprises at least one forward primer that anneals to each of the 44 possible alpha V regions. Each outer reverse primer anneals to sequence 5-40 base pairs downstream of the exon/intron junction of the alpha J region. In these embodiments, the outer primer set comprises at least one reverse primer that anneals to each of the 76 possible alpha J regions. By way of example, one possible set of alpha chain outer primers is disclosed in Tables 3-1 and 3-2.

Inner primers: Inner primers comprise forward and reverse inner primers (Fig. 1), in order to amplify the V and J regions. Preferably a set of inner primers is used comprising at least one primer complementary to each V and J region of the TCR alpha chain. Each inner forward primer anneals to sequence at the start of the first amino acid downstream of the signal sequence of the alpha V region. In these embodiments, the forward inner primer set comprises at least one inner primer that anneals to each of the 44 possible alpha V regions. Each inner reverse primer anneals to sequence at the downstream end of the alpha J region. In these embodiments, the inner reverse primer set comprises at least one reverse primer that anneals to each of the 76 possible alpha J regions. By way of example, one possible set of alpha chain inner primers is disclosed in Tables 3-5 and 3-6.

Cloning primers: Cloning primers comprise forward and reverse cloning primers (Fig. 1), in order to amplify the V and J regions. Each cloning forward primer is designed to anneal to sequence that overlaps the sequence of an inner forward primer. Thus, a set of cloning primers that comprises at least one primer complementary to each V and J regions of the TCR alpha chain are used. In the preferred embodiments, the 5' end of each cloning forward primer also comprises 10 to 50, more preferably 15 bases of homology to a desired vector, the "vector region," in order to facilitate cloning into a desired vector. For example, it may facilitate cloning by direct recombination into a vector, such as an expression vector, for example a lentiviral expression vector. Each cloning forward primer is designed to create a product that can be cloned directly into a vector. For an expression vector, the primers may be designed such that the product may be cloned within the correct reading frame, starting with the amino acid directly after the signal sequence. In other embodiments, the 5' end of each cloning forward primer instead comprises restriction sites that allow ligation into a desired vector, such as an expression vector. In some embodiments-for example embodiments in which TCR sequence is desired but functional TCR need not be expressed-the cloning forward primer does not necessarily produce an in-frame product, and the vector need not be an expression vector. Each cloning reverse primer is designed to anneal to sequence that overlaps the sequence of an inner reverse primer. In the preferred embodiments, the 5' end of each cloning reverse primer also comprises 10 to 50, more preferably 15 bases of homology to facilitate insertion into a vector, for example direct recombination into an expression vector, for example a lentiviral vector. In other embodiments, the 5' end of each cloning reverse primer comprises restriction sites that allow ligation into a vector, such as an expression vector. By way of example, one possible set of alpha chain cloning primers is disclosed in Tables 3-9 and 3-10.

Homologous primers: Homologous "clean-up" primers are designed to anneal to the vector region of the product of the cloning reaction. That is, the homologous primers are designed to anneal to the 5' ends of the product of the cloning primers that are homologous to the vector of choice. The homologous forward primer anneals to the homologous region from the cloning forward (V) primer. The homologous reverse primer anneals to the homologous region from the cloning reverse (J) primer. See Fig. 1. Preferably, the homologous primers are specific to only the 15 bases of homology at the end of the cloning product. In other embodiments-for example, embodiments wherein the primers contain 5' restriction sites for cloning into a vector-each homologous primer anneals to the 5' sequence added by the cloning primers. By way of example, one possible set of homologous primers is disclosed in Table 3-13.

*Beta chain primers.* In some embodiments, the primer sets for isolating sequence encoding the TCR beta chain comprise outer primers and inner primers as described herein; in other embodiments the primers sets comprise outer primers, inner primers and cloning primers as described herein; in other embodiments, the primer sets comprise outer primers, inner primers, cloning primers and homologous primers as described herein:

Outer primers: Outer primers comprise forward and reverse outer primers (Fig. 1), in order to amplify the V and J regions. Each outer forward primer anneals to sequence 5-40 base pairs upstream of the signal sequence junction of the beta V region. In these embodiments, the outer primer set comprises at least one forward primer that anneals to each of the 54 possible beta V regions. Each outer reverse primer anneals to sequence 5-40 base pairs downstream of the exon/intron junction of the beta J region. In these embodiments, the outer primer set comprises at least one reverse primer that anneals to each of the 14 possible beta J regions. By way of example, one possible set of beta chain outer primers is disclosed in Tables 3-3 and 3-4.

Inner primers: Inner primers comprise forward and reverse inner primers (Fig. 1), in order to amplify the V and J regions. Preferably a set of inner primers is used comprising at least one primer complementary to each V and J region of the TCR beta chain. Each inner forward primer anneals to sequence at the start of the first amino acid downstream of the signal sequence of the beta V region. In these embodiments, the forward primer set comprises at least one inner primer that anneals to each of the 54 possible beta V regions. Each inner reverse primer anneals to sequence at the downstream end of the beta J region. In these embodiments, the inner primer set comprises at least one reverse primer that anneals to each of the 14 possible beta J regions. By way of example, one possible set of beta chain inner primers is disclosed in Tables 3-7 and 3-8.

Cloning primers: Cloning primers comprise forward and reverse cloning primers (Fig. 1). Each cloning forward primer is designed to anneal to sequence that overlaps the sequence of an inner forward primer. Thus, a set of cloning primers that comprises at least one primer complementary to each V and J regions of the TCR beta chain are used. In the preferred embodiments, the 5' end of each cloning forward primer also comprises 10 to 50, more preferably 15 bases of homology to a desired vector, the "vector region," in order to facilitate cloning into a desired vector. The homologous region may facilitate direct recombination into an expression vector, for example a lentiviral vector. Each cloning forward primer is designed to create a product that can be cloned directly into a vector. For an expression vector, the primers may be designed such that the product may be cloned within the correct reading frame, starting with the amino acid directly after the signal sequence. In other embodiments, the 5' end of each cloning forward primer instead comprises restriction sites that allow ligation into a desired vector, such as an expression vector. In some embodiments-for example embodiments in which TCR sequence is desired but functional TCR need not be expressed-the cloning forward primer does not necessarily produce an in-frame product, and the vector need not be an expression vector. Each cloning reverse primer is designed to anneal to sequence that overlaps the sequence of an inner reverse primer. In the preferred embodiments, the 5' end of each cloning reverse primer also comprises 10 to 50, more preferably 15 bases of homology to facilitate insertion into a vector, for example direct recombination into an expression vector, for example a lentiviral vector. In other embodiments, the 5' end of each cloning reverse primer comprises restriction sites that allow ligation into a vector, such as an expression vector. By way of example, one possible set of beta chain cloning primers is disclosed in Tables 3-11 and 3-12.

Homologous primers. Homologous "clean-up" primers are designed to anneal to the vector region of the product of the cloning reaction. That is the homologous primers are designed to anneal to the 5' ends of the product of the cloning primers that are homologous to the vector of choice. The homologous forward primer anneals to the homologous region from the cloning forward (V) primer. The homologous reverse primer anneals to the homologous region from the cloning reverse (J) primer. See Figure 1. In other embodiments-for example, embodiments wherein the primers contain 5' restriction sites for cloning into an expression vector-each homologous primer anneals the 5' added by the cloning primers. By way of example, one possible set of homologous primers is disclosed in Table 3-13.

### Amplification of the TCR gene:

In some embodiments, template genomic DNA undergoes two or more rounds of amplification. Two series of amplification reactions may be performed in parallel, one for the alpha chain and one for the beta chain. However, in some embodiments, it is possible to perform only the reactions for identifying the alpha chain or only the reactions for identifying the beta chain.

In some embodiments, two rounds of amplification reactions, such as PCR reactions, are performed, the first to enrich genomic template for sequence encompassing a TCR alpha or beta chain, and the second to isolate sequence encoding the TCR alpha or beta chain. In some embodiments, a third round of amplification, such as PCR, is also performed to add sequence homologous to a cloning vector to the ends of the DNA segments encoding the TCR alpha or beta chain. In some embodiments, a fourth round of amplification, such as PCR, is also performed to isolate DNA segments that contain end sequence homologous to a cloning vector.

*First round (genomic TCR enrichment reaction):* A first round of amplification is performed to enrich genomic template for all TCR loci. A first enrichment product is produced. All of the outer forward and outer reverse primers for a TCR chain (alpha or beta chain) are pooled, the product of WGA is added as template, and amplification is performed.

In some embodiments amplification is performed using a touchdown PCR protocol (for a description of touchdown PCR, see Don et al (1991). Nucleic Acids Res. 19: 4008). The touchdown PCR protocol utilizes multiple iterations of a three-step cycle, each cycle comprising: first melting the template DNA at a melting temperature (TM)-for example 95 °C; second allowing primers to anneal at an annealing temperature; and third allowing polymerase to extend DNA at an extension temperature-for example, 70 °C. The annealing temperature may be the same as the extension temperature, or annealing temperature may be the different from the extension temperature. In the first cycle, a top annealing temperature is used-for example 77°C. In each subsequent cycle of touchdown PCR, the annealing temperature is incrementally decreased in until a bottom annealing temperature at or slightly below the optimal annealing temperature is reached. In some embodiments, the annealing temperature is decreased by an increment of less than 0.1, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.5, 4.6, 4.7, 4.8, 4.9, 5.0, or more than 5.0 °C in each cycle until the bottom annealing temperature is reached-for example 60°C. Once the bottom annealing temperature is reached, one or more cycles are performed using the bottom annealing temperature. By way of example only, a sample touchdown PCR protocol for the first round is described in Table 3-14.

One skilled in the art will appreciate that a number of other amplification protocols can be used, and the protocol for each round may be optimized along different parameters including, but not limited to, annealing temperature(s), annealing time(s), extension temperature(s), extension time(s), number of cycles, type and amount of polymerase used, concentrations of deoxyribonucleic acid trisphosphates (dNTP's), buffer composition, magnesium concentration, and the addition or removal of additional reagents, for example betaine, bovine serum albumin (BSA), dimethyl sulfoxide (DMSO), or preCES (a cocktail of additives).

*Second round (TCR Isolation reaction):* In some embodiments, a second round of amplification is performed to isolate specific alpha or beta chain sequence from the enriched genomic template. A second isolation product is produced. The inner forward and inner reverse primers for a TCR chain (alpha or beta) are pooled for the second round, and the enrichment product from the TCR enrichment reaction is added as a template. An amplification reaction, such as a touchdown PCR reaction, is then performed. By way of example only, a sample touchdown PCR protocol for the second round is described in Table 3-14. The skilled artisan will recognize that other amplification protocols can be used.

In some embodiments, products of the second TCR isolation round are used as template for DNA sequencing. In other embodiments, products of the second round are cloned directly into a vector. In other embodiments, products of the second round are used as template in a TCR cloning reaction, as described below.

*Third round (TCR cloning reaction):* In some embodiments, a third round of amplification is used to add sequence homologous to a cloning vector to the ends of DNA segments isolated in the second round (the isolation product). The cloning forward and reverse primers for a TCR chain (alpha or beta) are pooled for the third round, and isolation product from the second isolation round is added as a template. An amplification reaction, such as a touchdown PCR protocol, is then performed and a third cloning product is produced. By way of example only, a sample touchdown PCR protocol for the third round is described in Table 3-14. The skilled artisan will recognize that other amplification protocols can be used.

Products from the third round may be sequenced directly. They may also be cloned into a vector as desired, such as an expression vector. In some embodiments, products from the third round are purified via gel electrophoresis, for example on an agarose gel using techniques known in the art. In some embodiments, desired bands are 300-400bp in length.

*Fourth round (homologous reaction):* In the preferred embodiments, a fourth amplification reaction is performed to isolate and amplify DNA segments that contain end sequence homologous to a cloning vector. The homologous forward and reverse primers are pooled for the fourth round. Cloning product from the third amplification round is used as a template for a fourth amplification round to produce a fourth homologous product. In some embodiments a PCR protocol is performed. The PCR protocol comprises multiple iterations of a three-step cycles: first, melting the template DNA at a melting temperature (TM)-for example 95 °C; second, allowing primers to anneal at an annealing temperature-for example 65 °C; and third, allowing polymerase to extend DNA at an extension temperature-for example, 70 °C. By way of example only, a sample PCR protocol for the fourth round is described in Table 3-14. In other embodiments, a touchdown PCR protocol may be performed.

Homologous products from the fourth amplification round may be sequenced directly. In some embodiments, products from the fourth round are purified via gel electrophoresis, for example on an agarose gel using techniques known in the art. In some embodiments, desired bands are 300-400bp in length. In some embodiments, the isolated gene for each T cell receptor is subsequently cloned into a vector. In some embodiments, the vector comprises an expression vector, for example a lentiviral expression vector.

### Example 1:

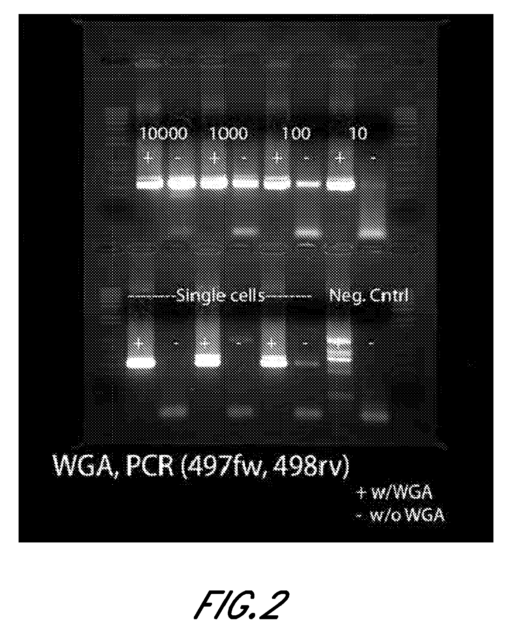

Cells from the well-characterized Jurkat T cell liner were used to validate the primer set and PCR protocol for isolating TCR alpha and beta chain sequences. Cells were serially diluted into two sets of wells of PBS containing 10000, 1000, 100, 10, and single cells. Both sets of cells were lysed but only one was amplified using WGA. PCR using oligonucleotides designed against Jurkat-specific alpha chains was run on both sets. In the absence of WGA, PCR amplification the TCR alpha chain (target size ~350bp) produced very low yields for templates with fewer than 100 cells. PCR amplification of samples processed using WGA produced high yields (Figure 2).

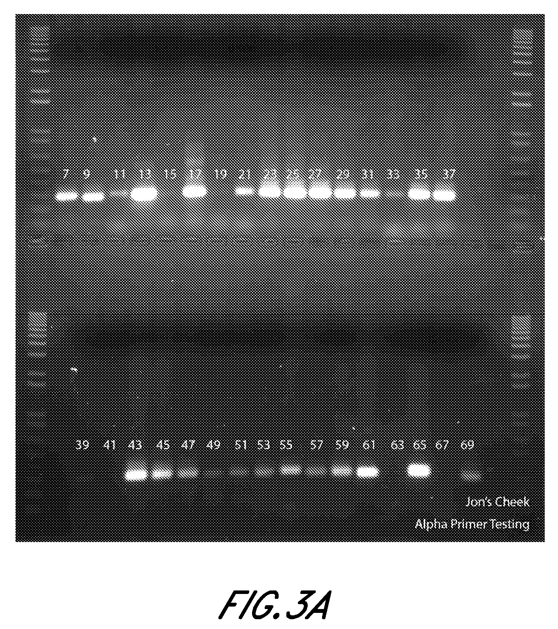

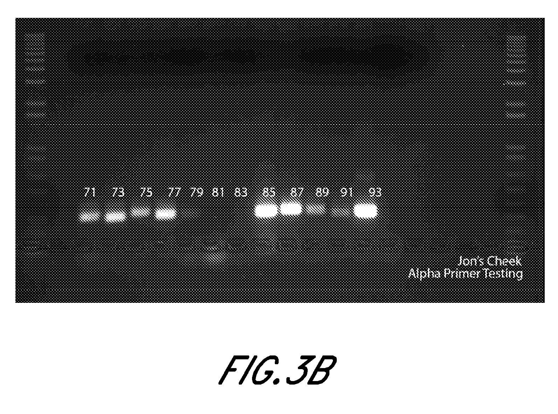

In order to isolate all possible rearrangements of the alpha and beta chains by nested PCR, two sets of oligonucleotides (inner and outer) were designed against all 188 V and J regions of both chains. Forward inner oligonucleotides in the V region were designed downstream of the V region signal sequence and intron. Care was taken to ensure that the primer would create a template capable of cloning into an expression vector while maintaining the correct reading frame starting with the amino acid directly after the signal sequence. Reverse inner oligonucleotides in the J region were designed at the ends of the J regions while also maintaining reading frame (Figure 3a). Outer oligonucleotides were designed 20-30 base pairs upstream of their corresponding inner oligonucleotides. Alpha and beta forward primers were designed with BbvCl and Sbfl restriction sites respectively at the ends to facilitate cloning. To confirm the ability of each of these oligonucleotides to function under identical PCR conditions, corresponding reverse primers were designed for each V and J region. PCR was performed using unrearranged genomic DNA as template using each pair of oligonucleotides. All oligonucleotides were functional under the same PCR conditions (Figure 3b).

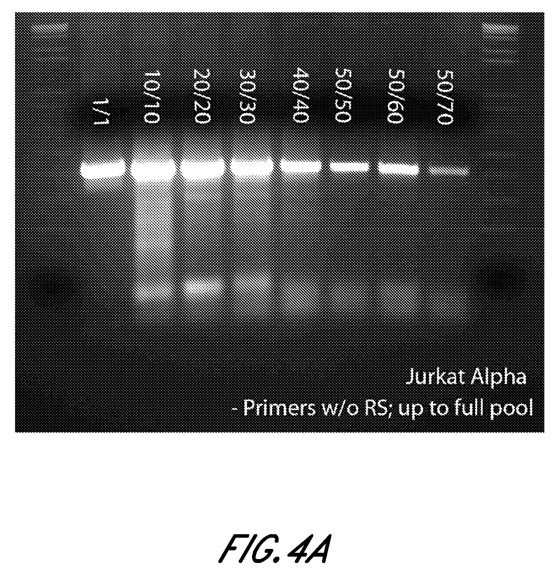

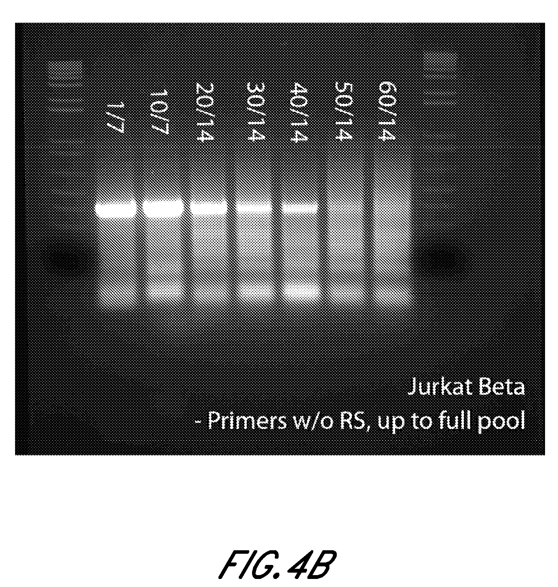

To minimize the number of reactions necessary for TCR amplification, the amplification protocol was multiplexed by pooling oligonucleotides into groups of V and J inner primers. Tests were run to determine the minimum number of forward and reverse pools of inner oligonucleotides (maximum number of oligonucleotides in each reaction) that could still isolate a single TCR. Multiplexed PCRs with increasing number of inner primers were performed on template genomic DNA isolated from cultured Jurkat cells to determine the maximum number of primers that still permitted successful TCR amplification. (Figures 4a,4b). Pools of 10 forward and reverse alpha primers (fw/rv) were added to PCR reactions until all primers against alpha loci were present in a single reaction. For example, a pool of 50 forward and 70 reverse primers allowed isolation of a single alpha chain. The Jurkat alpha chain could be isolated with all primers against alpha loci present in a single reaction (Figure 4a). Pools of 10 forward and reverse beta primers (forward/reverse) were added to PCR reactions until all primers against beta loci were present in a single reaction. The Jurkat beta chain was isolated with all primers in a single reaction (Figure 4b). For example a pool of 60 forward and 14 reverse primers allowed isolation of a single beta chain.

### Example 2:

In this example, TCR sequences were isolated from a specific population of T cells, CD25+ regulatory T-cells (T-regs). T-regs were obtained from a patient blood sample from the University of California, Los Angeles and are diluted to single cells. These cells were lysed, amplified by WGA, and confirmed to contain human genetic material by a PCR for a known unrearranged human gene, ataxia telangiectasia mutated (ATM). The TCR isolation protocol was run on these samples. Four different alpha chains and eight different beta chains were identified. Amplified chains were cloned into a TOPO shuttle cloning vector, and sequenced using techniques known in the art. They were found to be aV9-2J33, aV17J33, aV2J3, aV26-2J33, bV18J2-2, bV6-1J1-1, bV4-1J2-5, bV11-2J2-2, bV6-2J2-7, bV9J2-7, bV6-1J2-2, bV6-8J1-1. These sequences were aligned against the specific V and J regions of the genome and were 100% homologous except for the N region at the junction of the V and J regions.

### Example 3: NY-ESO TCR Isolation

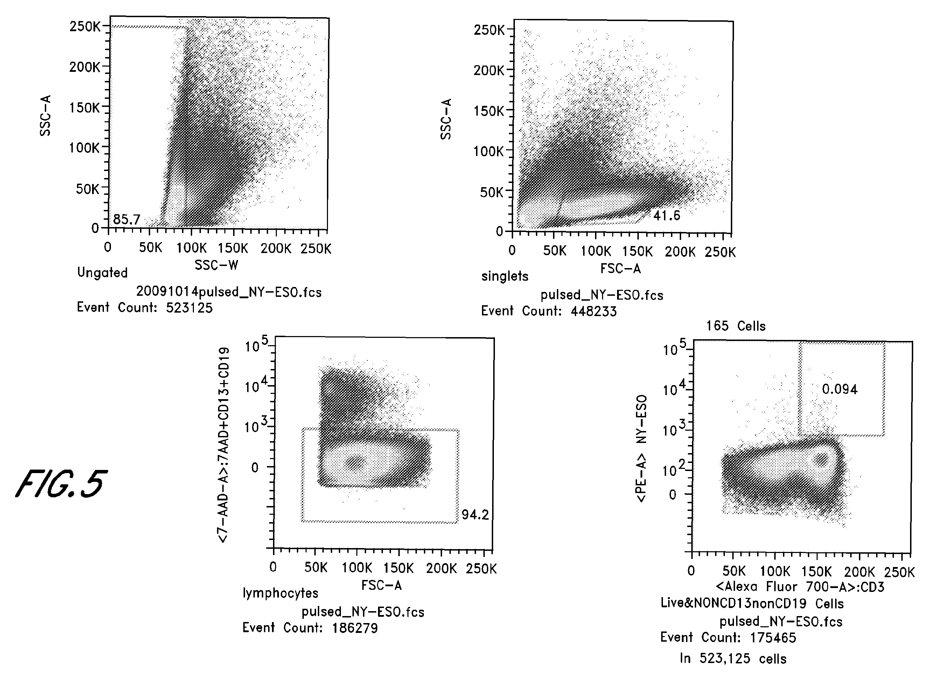

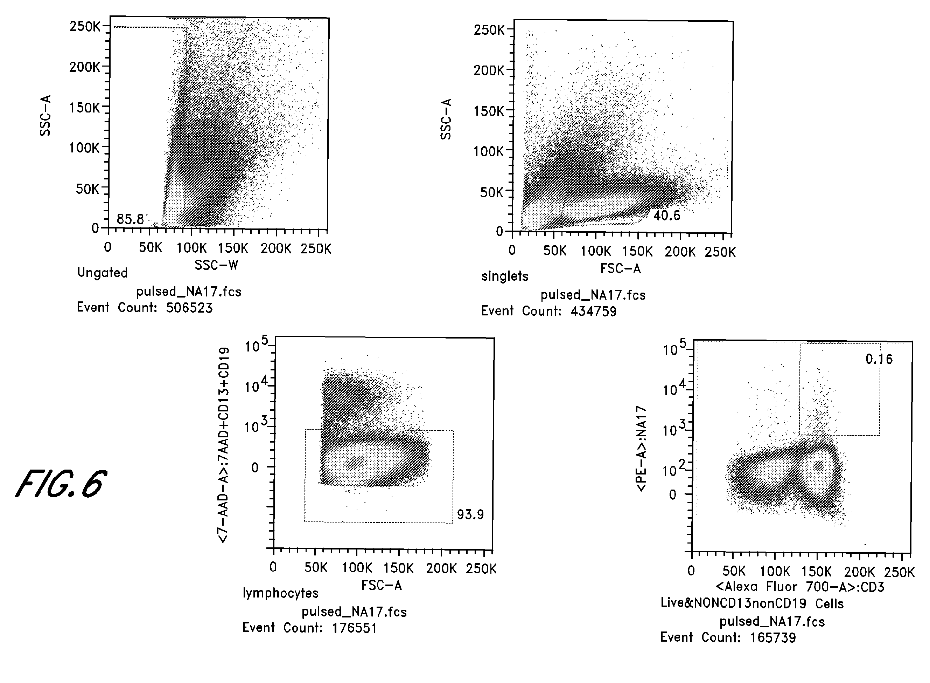

In this example, TCR sequences were isolated from a population of T cells with anti-melanoma activity. CD25+ regulatory T-cells (T-regs) were isolated Patient samples demonstrating clinical anti-melanoma activity were stained with antibodies against CD3, CD13, CD19, CD4, CD8. CD3+ patient T-cells were stained with melanoma antigen-specific tetramers, including NYSEO, MART1, NA-17, Tyrosinase and GP100. 7-AAD negative live cells were further gated to be CD13- CD19- CD3+ and tetramer positive (Figures 5, 6). Cells meeting the gating criteria were individually sorted into 1.5ul of alkaline lysis buffer (ALB), each cell added to a single well of a 96-well plate, and frozen at -20°C for transport.

WGA kits were obtained from Qiagen (Repli-G) and GE Healthcare Life Sciences (Illustra GenomiPhi V2 DNA Amplification Kit) and amplification was done according to Spits, C. et al (2006). Nature Protocols 1: 1965-70. Briefly, cells were sorted into 1.5ul of alkaline lysis buffer (ALB) and incubated at -20°C for 30 minutes. Reactions were incubated at 65°C for 10 minutes to lyse cells prior to addition of 9ul of GenomiPhi sample buffer, 9ul of GenomiPhi reaction buffer and 1ul of Phi29 enzyme. Reactions were run isothermally at 30°C for 2 hours and inactivated at 65°C for 10 minutes. The products were stored at 4 °C.

To minimize the number of reactions necessary for TCR amplification, the PCR amplification was multiplexed by pooling oligonucleotides into groups of V and J primers. The first round PCR reactions was performed in 20µl reactions comprising: 1µl of WGA product; 10µl of Novagen KOD Hot Start Master Mix; 4µl of DEP-C treated H₂O; 4µl of a 5x solution of preCES additive; 0.5 µl of pooled forward primers; 0.5µl of pooled reverse primers (pools of primers are created by combining 5µl of 100µM solution of each primer and creating 20µl aliquots). The primers pairs used for amplifying the alpha chain were the alpha V outer primers (Table 3-1) and the alpha J outer primers (Table 3-2). The primer pairs used for amplifying the beta chain were the beta V outer primers (Table 3-3) and the beta J outer primers (Table 3-4). The PCR amplification protocol for the first round is summarized in Table 3-14. One skilled in the art will appreciate that parameters of the PCR protocol, for example annealing temperatures and incubation times, can readily be altered to optimize the protocol for a certain set of primers and/or template.

In the second round of PCR, 1µl of product from the first round was added to a PCR cocktail comprising all of the inner primers for either the alpha or the beta chain. The primer pairs used for amplifying the alpha chain are the alpha V inner primers (Table 3-5) and the alpha J inner primers (Table 3-6). The primer pairs used for amplifying the beta chain are the beta V inner primers (Table 3-7) and the beta J inner primers (Table 3-8). The PCR cocktail is otherwise as described in the first round. The PCR amplification protocol for the second round is summarized in Table 3-14.

In the third round of PCR, 1µl of product from the second round is added to a PCR cocktail comprising all of the cloning primers for either the alpha or the beta chain. The primer pairs used for amplifying the alpha chain are the alpha V cloning primers (Table 3-9) and the alpha J cloning primers (Table 3-10). The primer pairs used for amplifying the beta chain are the beta V cloning primers (Table 3-11) and the beta J cloning primers (Table 3-12). The PCR cocktail is otherwise as described in the first round. The PCR amplification protocol for the third round is summarized in Table 3-14.

Product from the third round was added to a cocktail comprising the homologous primers (Table 3-13), and amplified using the protocol disclosed in Table 3-14. Three cells yielded both alpha and beta chains, and one cell yielded an alpha chain. The pairs were TRAV12-1J1, TRBV5-6J1-1; TRAV38-2DV8J53, TRBV5-6J1-2; and TRAV8-3J44, TRBV5-6J1-1. The unpaired alpha chain was TRAV21J47. Upon sequence analysis, it was found that all chains aligned perfectly with their corresponding genomic regions and regions of junction diversity could be readily identified. The receptor TRAV38-2DV8J53, TRBV5-6J1-2 was found to be a non-functional rearrangement with no open reading frames. It was found that two of the isolated TCRs have a common identical beta chain (TRBV5-6J1-1). Without limiting the invention to any one theory, this result could be explained by positive selection, where the beta chain first rearranges and is expressed on the surface with the pre-T-cell receptor alpha chain (pTalpha). If the beta chain is positively selected for, a subsequent alpha chain will undergo rearrangement.

| **SEQ ID NO:** | **Table 3-1: Alpha V Outer Primers** |
|---|---|
| 5 | 5' TCRaV1-2us; CCAATGGCTCAGGAACTGGGAATGC; |
| 6 | 5' TCRaV2us; GGAAAAAATGCAAAACAGGTAGTCTTAAATAAGCATTC; |
| 7 | 5' TCRaV3us; GACCCCCCCAATCCCGCCC; |
| 8 | 5' TCRaV4us; CAGACACAGCAAAAGAGCCTAGAACCTGG; |
| 9 | 5' TCRaV5us; GATAATATAGCTCTCTTGGCTGGAGATTGCAGGT; |
| 10 | 5' TCRaV6us; TAACACCTATCAAACTAAACAGAATGGCTTTTTGG; |
| 11 | 5' TCRaV7us; AAGAAACAAACAATAAAAGCTTTGTTTGGCTACATAATT; |
| 12 | 5' TCRaV8-1us; AAGACCTGGGTTCCAGCCACTTTCCTACT; |
| 13 | 5' TCRaV8-2us; CTCCTAGCTCCTGAGGCTCAGGACCCCTGGCTTC; |
| 14 | 5' TCRaV8-3us; ACACCTCTTGGTCTTGGTCTCTTCAGACACTT; |
| 15 | 5' TCRaV8-4us; TGTCCGCTCTGCTCAGGGCCCT; |
| 16 | 5' TCRaV9-1us; TTTTCCTCACACTAAGAAGACAAGACCCAAGG; |
| 17 | 5' TCRaV10us; TAGTGTTAAAAAAAAAGAGAAGATGTTGAATACACAAGTCAACT; |
| 18 | 5' TCRaV12-1us; ATTTCTTTTTGGATTGAAAATTTTAATCCTCAGTGAAC; |
| 19 | 5' TCRaV12-2us; AAATATCCATTCTAGGTGCATTTTTTAAGGGTTTAAAATTT; |
| 20 | 5' TCRaV13-1us; ACAACCTGATGATAGAAGTAACTCTTATAACTGGAGGTTG; |
| 21 | 5' TCRaV13-2us; CGATGATGGAAGTAGCTCTTATGGCTGGAGAT; |
| 22 | 5' TCRaV14D4us; CACCTCACAGTACAGAGTCCTGAAAATAAAGAAGAAGA; |
| 23 | 5' TCRaV9-2us; TTCTTCATGTTAAGGATCAAGACCATTATTTGGGTAA; |
| 24 | 5' TCRaV12-3us; AAATGAGAAACGTTTGTTATTATTTTTTTTTCGTGTTTAA; |
| 25 | 5' TCRaV8-6us; TGAGGCTCAGCGCCCTTGGCTTCTGTCCGCC; |
| 26 | 5' TCRaV16us; CAGAGTGTCTATGTGGCTGAATCGTTTCCAG; |
| 27 | 5' TCRaV17us; TCATCTGTGACTGAGGAGCCTTGCTCC; |
| 28 | 5' TCRaV18us; ACCTTTCGGTTTGGATATCTCTCAACAAAACC; |
| 29 | 5' TCRaV19us; AGACGGAGCACGGAACATTTCACTCAG; |
| 30 | 5' TCRaV20us; AAGAAGGTTGGAATTATCGTAATTTGTTTCTAGGCTG; |
| 31 | 5' TCRaV21us; CTTGTGAGCCATTCTCCATATTTCAGATATAAGATTTCAG; |
| 32 | 5' TCRaV22us; CCAAGGTTTAGTTAAATATATCTTATGGTGAAAATGCCC; |
| 33 | 5' TCRaV23D6us; GTTGGGAAGACTGGAAGACCACCTGG; |
| 34 | 5' TCRaV24us; TTCCACAGATTTTGGCTGAAAAACGTTTTTCT; |
| 35 | 5' TCRaV25us; GTACCAGGCAACCCATTTAGGAGAAGTTGG; |
| 36 | 5' TCRaV26-1us; AACCTAGAATCAGACACAAAAACTGAACTCTGGG; |
| 37 | 5' TCRaV8-7us; CCCACTCAGGAGATCTTCTAGAATAGAGCTCTCA; |
| 38 | 5' TCRaV27us; AGGAGCAGCTAAAGTCAGGGGCCATGT; |
| 39 | 5' TCRaV29DV5us; TGCAGCTTTCTAGGCAGGAGACAAGACAAT; |
| 40 | 5' TCRaV30us; GTTAAGGAAGCCCATTCAGAAGCTGACTGG; |
| 41 | 5' TCRaV26-2us; GACACAGAGTCTGAGTTCTGGGGCCTG; |
| 42 | 5' TCRaV34us; GCAAAGTAACTTCTGCTGGGGAAGCTCAT; |
| 43 | 5' TCRaV35us; GTGTCACTCTAAGCCCAAGAGAGTTTCTTGAAGC; |
| 44 | 5' TCRaV36DV7us; TTAAAGGTAGTGAATCACGTTTTGCCCAGG; |
| 45 | 5' TCRaV38-1us; AACCCATCAGAGCAGGAGACTTTTCACTCT; |
| 46 | 5' TCRaV38-2DV8us; ATACTCAAGGTTCAGATCAGAAGAGGAGGCTTCTC; |
| 47 | 5' TCRaV39us; CAACTTTCAAGGCTCCTAAATCTGAGTTTTCAGTG; |
| 48 | 5' TCRaV40us; AACTGTGAATCCTCACTTCAACAGTGATGCC; |
| 49 | 5' TCRaV41us; ATATATTCCGAAATCCTCCAACAGAGACCTGTG; |

| **SEQ ID NO:** | **Table 3-2: Alpha J Outer Primers** |
|---|---|
| 50 | 3' TCRaJ1 Intron; GGTCCCACCGAGGCTTTAGTGAGCA; |
| 51 | 3' TCRaJ2 Intron; AGAGAAAGGATTAGTGACACTGGCCCATGG; |
| 52 | 3' TCRaJ3 Intron; GCATTTTGGACAAAGAAGAAATAGTTGTCCGTC; |
| 53 | 3' TCRaJ4 Intron; CTGTTTTCTCATAGACAAGTGGTCAGTTCTTTTTGC; |
| 54 | 3' TCRaJ5 Intron; TTCATCATCTAAGAAAGCAGAGTAGGGCCTTTCT; |
| 55 | 3' TCRaJ6 Intron; TCTCACTACTACAGTATCCTTCCATAATATAATCTGTCTGCAA; |
| 56 | 3' TCRaJ7 Intron; TCTCCAGCACAGGGTAGCGATGGG; |
| 57 | 3' TCRaJ8 Intron; ACCAGAATAATTATATCCATATATGCCCAATATTGAGGATA; |
| 58 | 3' TCRaJ9 Intron; CCCCTAAAAAGAAAAAAATCAATGAAAACAGATGTTC; |
| 59 | 3' TCRaJ10 Intron; CACCTTTTCTTCCACTTATTGTCACCAGAATACATT; |
| 60 | 3' TCRaJ1 1 Intron; CAATACATATGGAAGCCTTAAACCAGATAAGGGG; |
| 61 | 3' TCRaJ12 Intron; TTTCCTGAGACATGAAGACATTTTACCCTCAATC; |
| 62 | 3' TCRaJ13 Intron; CAAATGTTACGGTCTGAGAGAAGACAACACAAG; |
| 63 | 3' TCRaJ14 Intron; AGTAAGTTTAGTGGGTCTCAGTAGCCACATTAAGCC; |
| 64 | 3' TCRaJ15 Intron; TCACCTGTGCAATATATGACTACAGGATAAGTACAAGC; |
| 65 | 3'TCRaJI6Intron; CTTAGATTTCCAAAAAAGCTTATTACTTGTCTCAAAAACTAATC; |
| 66 | 3' TCRaJ17 Intron; GCACATTGAATTGCAAATTGATGACAAGG; |
| 67 | 3' TCRaJ18 Intron; GAGTTAATTCATCTCCCCTTTTAATTTCTCCACAGTAATA; |
| 68 | 3' TCRaJI9 Intron; GAAGAAACTTTGCTCCCCTGGCCCT; |
| 69 | 3' TCRaJ20 Intron; TGCTGAAAAACCTACCCACCATTTTGCTTAA; |
| 70 | 3' TCRaJ21 Intron; AATACAGACTGAAAAGAAGAATTTAGCATAATGTGTTGGT; |
| 71 | 3'TCRaJ22Intron;CCCATTAAGTTACATGTACAGAATACATTTGTAGATTAGTAAATC AG; |
| 72 | 3' TCRaJ23 Intron; GGTCTAAATCAGCCCTTAATCCACAGACATTG; |
| 73 | 3' TCRaJ24 Intron; GCATGCAGGGCATGCCAAATACTAAGG; |
| 74 | 3' TCRaJ25 Intron; AAAGAGGGCAAGTTTTCCTCTTGGAGATAATCATA; |
| 75 | 3' TCRaJ26 Intron; AGCTTCTCCCCACATCAAGCACTGGACT; |
| 76 | 3' TCRaJ27 Intron; TTCAAACTAATGATTTGATTGATTGCCCCTG; |
| 77 | 3' TCRaJ28 Intron; AGCTTCTGCATGATGGAAGACAGGCTTCT; |
| 78 | 3' TCRaJ29 Intron; AAATAATTCAAGGGAAGAAGCCATTGCTGAG; |
| 79 | 3' TCRaJ30 Intron; CACTCTCAGCAGTTTGAACTCAGTGGGAGTTA; |
| 80 | 3' TCRaJ31 Intron; AAATCTCCACTAACTTCACGGGATTTATTTGTTTG; |
| 81 | 3' TCRaJ32 Intron; CGCTTCCTACTTGCCATGGACACAGAA; |
| 82 | 3' TCRaJ33 Intron; TGCTACACTTTGTGCATTATTCAACTAGTGTCTCCT; |
| 83 | 3' TCRaJ34 Intron; TTCATTTAAAAAAAAAAGAAAAAGAAAAAGAAAACACCTTTT; |
| 84 | 3' TCRaJ35 Intron; CATAAGAAGAACTGTTCTATATGATTTACGGACATAACAGC; |
| 85 | 3' TCRaJ36 Intron; GTGTCTGGGATGTGAGAACTTGTCATTACAGACTAA; |
| 86 | 3' TCRaJ37 Intron; GACAGAGAAGATTAAACAAAAAATGAACAGAGTGGATAAC; |
| 87 | 3' TCRaJ38 Intron; GGCAGTTTCTGAGATATTTCAAACTGCACAGAC; |
| 88 | 3' TCRaJ39 Intron; TCAGTGCTACGGCTTCCTTTTGAAATTAGAGC; |
| 89 | 3' TCRaJ40 Intron; GTCCCTCAAACATGAACACCAACAACCTTTAA; |
| 90 | 3' TCRaJ41 Intron; CAACAGGTCCCATTGGATTTCTTTCCAGA; |
| 91 | 3' TCRaJ42 Intron; ATTCTGTTGCCCAGAGTGACAAAGTACTGATGAT; |
| 92 | 3' TCRaJ43 Intron; CAGCACCATTGCTCACTCAGGTCAGC; |
| 93 | 3' TCRaJ44 Intron; TGCAGTATCCCCTGTTTTAAAGGAACACACAG; |
| 94 | 3' TCRaJ45 Intron; GGTTTAGAAATGTCCCCATGAGGACTGCA; |
| 95 | 3' TCRaJ46 Intron; CTCAAATGTCAGGCTAGAACAAATAATAGGAAAAGGC; |
| 96 | 3' TCRaJ47 Intron; AGCCAGAAAAAGTTTATTTAATATGCAATGAAACCCA; |
| 97 | 3' TCRaJ48 Intron; ATGTCTACTATGATCCCCAGAATCTTATGCAGGC; |
| 98 | 3' TCRaJ49 Intron; CTCTTTCTGCAGTTTAAAGGGTTTGCTCAACAC; |
| 99 | 3' TCRaJ50 Intron; CTAATGTATGAGACTGTTAGCCCCAGCGCA; |
| 100 | 3' TCRaJ51 Intron; CGGGGAAGGGAGCAAAAGTACATAAGGA; |
| 101 | 3' TCRaJ52 Intron; CTGACACTGGGGTGACATTCCAGAATTTC; |
| 102 | 3' TCRaJ53 Intron; GGAGGGGCAAGTAATTAAATCAGAAGTGTTTGAAT; |

| **SEQ ID NO:** | **Table 3-3: Beta V Outer Primers** |
|---|---|
| 103 | 5' TCRbV2us; CCACAGGACCAGATGCCTGAGCTAGG; |
| 104 | 5' TCRbV3-1us; CACTGCAGACCAGAATCCTGCCCTG; |
| 105 | 5' TCRbV4-1us; CAGCACCTCGCCCAAAGGACCC; |
| 106 | 5' TCRbV5-1us; GGAGGACCAAGCCCTGAGCACAGA; |
| 107 | 5' TCRbV6-1us; TATCACCGATGCACAGACCCAGAAGACC; |
| 108 | 5' TCRbV7-1us; TCCTACTCACAGTGACTCTGATCTGGTAAAGCTC; |
| 109 | 5' TCRbV4-2us; TCACCCAGAGGACCCCAGTCAGAGG; |
| 110 | 5' TCRbV6-2us; TCCCTTTTCACCAATGCACAGACCCA; |
| 111 | 5' TCRbV4-3us; ACCTCACCCAGAGGACCCCAGTCAGA; |
| 112 | 5' TCRbV6-3us; CCTTTTCACCAATGCACAGACCCAGAG; |
| 113 | 5' TCRbV7-2us; CTCACAGTGATCCTGATCTGGTAAAGCTCCC; |
| 114 | 5' TCRbV6-4us; GCCTTTCATCAACACACAGACCCAGAAGA; |
| 115 | 5' TCRbV7-3us; TCCTGCTCACAGTGACCCTGATCTGGTA; |
| 116 | 5' TCRbV5-3us; CCCAGGAGGACCAAGCCCTGAATC; |
| 117 | 5' TCRbV9us; GGAGCTTAGGAACTTCAGAATGCTTACTACAGAGA; |
| 118 | 5' TCRbV10-1us; CTTCAGTCTGCCCACAGCAGGGCT; |
| 119 | 5' TCRbV11-1us; CTCCTCTGCTCCTGTTCACAAGGACCCT; |
| 120 | 5' TCRbV10-2us; AATTTGCCCACAGCAGGGCTGG; |
| 121 | 5' TCRbV11-2us; TTTTGCTCACAGTGACCCTGATTGGG; |
| 122 | 5' TCRbV6-5us; CTGCTCCCCTTTCATCAATGCACAGATA; |
| 123 | 5' TCRbV7-4us; GCTCCTGCTCATAGTGACACTGACCTGGTA; |
| 124 | 5' TCRbv5-4us; CCCCAGGAGGACCAAGCCCTGAAT; |
| 125 | 5' TCRbV6-6us; CCTTTCATCAATGCACAGATACAGAAGACCC; |
| 126 | 5' TCRbV7-5us; GCTCCTGCTCACAGTGACACTGATCTGGTA; |
| 127 | 5' TCRbV5-5us; CCCAGGAGGACCAAGCCCTGAATC; |
| 128 | 5' TCRbV6-7us; CCCCTTTCATCAATGCACAGACCCAG; |
| 129 | 5' TCRbV7-6us; TGCTGCTGCTCACAGTGACACTGATCTG; |
| 130 | 5' TCRbV5-6us; TTCCCCAGGAGAACCAAGCCCTGA; |
| 131 | 5' TCRbV6-8us; CCCTTTTATCAATGCACAGACCCAGAAGAC; |
| 132 | 5' TCRbV7-7us; TCCGCTCCTGCTCACAGTGACACTGAT; |
| 133 | 5' TCRbV5-7us; TTTCCCAGGAGGACCAAGCCCTG; |
| 134 | 5' TCRbV6-9us; CCTTTCATCAATGCACAGACCCAGAAGAC; |
| 135 | 5' TCRbV7-8us; TTCTGTTCACAGTGACACTGATCTGGTAAAGCC; |
| 136 | 5' TCRbV5-8us; CCAGGAAGACCAAGCCCTGAATCAGG; |
| 137 | 5' TCRbV7-9us; CTGCTCACAGTGACCCTGATCTGGTAAAGC; |
| 138 | 5' TCRbV13us; CAAAAGCCCTGCTTTCTCACCCCAG; |
| 139 | 5' TCRbV10-3us; CTATTTCCCCAGGCAGGGCTGGG; |
| 140 | 5' TCRbV11-3us; CTGCTCCTGCTCACAGTGACCCTGATC; |
| 141 | 5' TCRbV12-3us; CTCACAGAGGGCCTGGTCTAGAATATTCCA; |
| 142 | 5' TCRbV12-4us; TTCTTTGCTCATGTTCACAGAGGGCCTG; |
| 143 | 5' TCRbV12-5us; TTCGTGCCCACAAGGGCCTCAT; |
| 144 | 5' TCRbv14us; CTCATACTTGTAAGCTCCTTCATCTGGAAATGTG; |
| 145 | 5' TCRbV15us; CAGAGCCTGAGACAGACAGATGCTTCATTC; |
| 146 | 5' TCRbV17us; TACTGCACATCAGAACCCATCGCTGG; |
| 147 | 5' TCRbV18us; TGCAGCAAGTGCCTTTGCCCTG; |
| 148 | 5' TCRbV19us; CATTCTCTTCCAACAAGTGCTTGGAGCTC; |
| 149 | 5' TCRbV20-1us; GAGGCAGTGGTCACAACTCTCCCCA; |
| 150 | 5' TCRbV22us; TCTCTCTCTCTTAGAGCCTGTGTCTGTAACTTCAG; |
| 151 | 5' TCRbV23-1us; CAGAAAGGGGATGAAAAAGCCTCATCC; |
| 152 | 5' TCRbV24-1us; ATGCCCTGCTTCCCTCAACATCCAG; |
| 153 | 5' TCRbV25-1us; CCCATCCTGCTTCCCCACTACTGG; |
| 154 | 5' TCRbV26us; ATCAGGGACTAAATTCATCACAGCACCAAGC; |
| 155 | 5' TCRbV27us; ACAGAAACCACCTGGAGCCCCCAG; |

| **SEQ ID NO:** | **Table 3-4: Beta J Outer Primers** |
|---|---|
| 156 | 3' TCRbJ1-1 Intron; TGGACCCACTTTTCCCTGTGACGG; |
| 157 | 3' TCRbJ1-2 Intron; CATTTCCCAGGACAGAGTCCTCCCTCAT; |
| 158 | 3' TCRbJ1-3 Intron; CTGGATTCCAGCCCCTTTTTGCAAG; |
| 159 | 3' TCRbJ1-4 Intron; GGTCCTCCTGGAACTCCGACCTTATGAT; |
| 160 | 3' TCRbJ1-5 Intron; AAGCAGAGAACTCTGCCTTCAAGGGACAA; |
| 161 | 3' TCRbJ1-6 Intron; AACTGATCATTGCAGTCAAACCCAGGC; |
| 162 | 3' TCRbJ2-1 Intron; CGTGCAGGCTGGGCTGCTCAC; |
| 163 | 3' TCRbJ2-2 Intron; GCCCATCCCGCCCTCTCGG; |
| 164 | 3' TCRbJ2-2P Intron; CAGACTCAGCTCGGGTCCTTCCCA; |
| 165 | 3' TCRbJ2-3 Intron; GGGCGCCCCCTCCCCAGT; |
| 166 | 3' TCRbJ2-4 Intron; GCACAAAAACCCGAGCGCAGTCTC; |
| 167 | 3' TCRbJ2-5 Intron; CAAAAACCAGACCCAAGCCGCC; |
| 168 | 3' TCRbJ2-6 Intron; CGCCGCCTTCCACCTGAATCC; |
| 169 | 3' TCRbJ2-7 Intron; CGACTCCGGGGACCGAGGG; |

| **SEQ ID NO:** | **Table 3-5: Alpha V Inner Primers** |
|---|---|
| 170 | 5' TCRaV1-2; GGACAAAACATTGACCAGCCCACTGAGAT; |
| 171 | 5' TCRaV2;AAGGACCAAGTGTTTCAGCCTTCCACAGTG; |
| 172 | 5' TCRaV3;CAGTCAGTGGCTCAGCCGGAAGATC; |
| 173 | 5' TCRaV4;AAGACCACCCAGCCCATCTCCATG; |
| 174 | 5' TCRaV5;GAGGATGTGGAGCAGAGTCTTTTCCTGAGTG; |
| 175 | 5' TCRaV6;CAAAAGATAGAACAGAATTCCGAGGCCCTG; |
| 176 | 5' TCRaV7;GAAAACCAGGTGGAGCACAGCCCTC; |
| 177 | 5' TCRaV8-1;CAGTCTGTGAGCCAGCATAACCACCAC; |
| 178 | 5' TCRaV8-2;CAGTCGGTGACCCAGCTTGACAGC; |
| 179 | 5' TCRaV8-3;CAGTCAGTGACCCAGCCTGACATCCAC; |
| 180 | 5' TCRaV8-4;CAGTCGGTGACCCAGCTTGGCAG; |
| 181 | 5' TCRaV9-1;GATTCAGTGGTCCAGACAGAAGGCCAAGT; |
| 182 | 5' TCRaV10;AAAAACCAAGTGGAGCAGAGTCCTCAGTCC; |
| 183 | 5' TCRaV12-1;CAACGGAAGGAGGTGGAGCAGGATC; |
| 184 | 5' TCRaV12-2;CAACAGAAGGAGGTGGAGCAGAATTCTGG; |
| 185 | 5' TCRaV13-1;GAGAATGTGGAGCAGCATCCTTCAACC; |
| 186 | 5' TCRaV13-2;GAGAGTGTGGGGCTGCATCTTCCTACC; |
| 187 | 5' TCRaV14D4;CAGAAGATAACTCAAACCCAACCAGGAATGTTC; |
| 188 | 5' TCRaV9-2;AATTCAGTGACCCAGATGGAAGGGCC; |
| 189 | 5' TCRaV12-3;CAACAGAAGGAGGTGGAGCAGGATCCT; |
| 190 | 5' TCRaV8-6;CAGTCTGTGACCCAGCTTGACAGCCA; |
| 191 | 5' TCRav16;CAGAGAGTGACTCAGCCCGAGAAGCTC; |
| 192 | 5' TCRaV17;CAACAGGGAGAAGAGGATCCTCAGGCC; |
| 193 | 5' TCRaV18;GACTCGGTTACCCAGACAGAAGGCCC; |
| 194 | 5' TCRaV19;CAGAAGGTAACTCAAGCGCAGACTGAAATTTCT; |
| 195 | 5' TCRaV20;GAAGACCAGGTGACGCAGAGTCCCG; |
| 196 | 5' TCRaV21;AAACAGGAGGTGACGCAGATTCCTGC; |
| 197 | 5' TCRaV22;ATACAAGTGGAGCAGAGTCCTCCAGACCTGA; |
| 198 | 5'TCRaV23DV6;CAACAGAAGGAGAAAAGTGACCAGCAGCA; |
| 199 | 5'TCRaV24;ATACTGAACGTGGAACAAAGTCCTCAGTCACTG; |
| 200 | 5'TCRaV25;CAACAGGTAATGCAAATTCCTCAGTACCAGC; |
| 201 | 5' TCRaV26-1 ;AAGACCACCCAGCCCCCCTCC; |
| 202 | 5' TCRaV8-7;CAGTCGGTGACCCAGCTTGATGGC; |
| 203 | 5' TCRaV27;CAGCTGCTGGAGCAGAGCCCTCAGT; |
| 204 | 5'TCRaV29DV5;CAACAGAAGAATGATGACCAGCAAGTTAAGCAA; |
| 205 | 5' TCRaV30;CAACAACCAGTGCAGAGTCCTCAAGCC; |
| 206 | 5' TCRaV26-2;AAGACCACACAGCCAAATTCAATGGAGAGTAAC; |
| 207 | 5' TCRaV34;CAAGAACTGGAGCAGAGTCCTCAGTCCTTG; |
| 208 | 5'TCRaV35;CAACAGCTGAATCAGAGTCCTCAATCTATGTTTATC; |
| 209 | 5'TCRaV36DV7;GAAGACAAGGTGGTACAAAGCCCTCTATCTCTG; |
| 210 | 5'TCRaV38-2DV8;CAGACAGTCACTCAGTCTCAACCAGAGATGTCT; |
| 211 | 5' TCRaV39;GAGCTGAAAGTGGAACAAAACCCTCTGTTC; |
| 212 | 5' TCRaV40;AATTCAGTCAAGCAGACGGGCCAAATAAC; |
| 213 | 5' TCRaV41;GCCAAAAATGAAGTGGAGCAGAGTCCTC; |

| **SEQ ID NO:** | **Table 3-6: Alpha J Inner Primers** |
|---|---|
| 214 | 5' TRAJ1;/5PHOS/AT GGGGAGAAGTGGAAACTCTGGTTCC; |
| 215 | 5' TRAJ2;/5PHOS/AT CAGATATAATGAATACATGGGTCCCTTTCCCA; |
| 216 | 5' TRAJ3;/5PHOS/AT TTGGCCGGATGCTGAGTCTGGTC; |
| 217 | 5' TRAJ4;/5PHOS/AT ATGGGTGTACAGCCAGCCTGGTCCC; |
| 218 | 5' TRAJ5;/5PHOS/AT TTGGTTGCACTTGGAGTCTTGTTCCACTC; |
| 219 | 5' TRAJ6;/5PHOS/AT ACGGATGAACAATAAGGCTGGTTCCTCTTC; |
| 220 | 5' TRAJ7;/5PHOS/AT TTGGTATGACCACCACTTGGTTCCCCTT; |
| 221 | 5' TRAJ8;/5PHOS/AT TTGGACTGACCAGAAGTCGGGTGCC; |
| 222 | 5' TRAJ9;/5PHOS/AT TTGCTTTAACAAATAGTCTTGTTCCTGCTCCAAAG; |
| 223 | 5' TRAJ10;/5PHOS/AT TGAGTTCCACTTTTAGCTGAGTGCCTGTCC; |
| 224 | 5' TRAJ11;/5PHOS/AT CTGGAGAGACTAGAAGCATAGTCCCCTTCCC; |
| 225 | 5' TRAJ12;/5PHOS/AT CAGGCCTGACCAGCAGTCTGGTCC; |
| 226 | 5' TRAJ13;/5PHOS/AT TTGGGATGACTTGGAGCTTTGTTCCAAT; |
| 227 | 5' TRAJ14;/5PHOS/AT CAGGTTTTACTGATAATCTTGTCCCACTCCCA; |
| 228 | 5' TRAJ15;/5PHOS/AT TGGAACTCACTGATAAGGTGGGTTCCCTTC; |
| 229 | 5' TRAJ16;/5PHOS/AT TAAGATCCACCTTTAACATGGTTCCCCTTG; |
| 230 | 5' TRAJ17;/5PHOS/AT TTGGTTTAACTAGCACCCTGGTTCCTCCTC; |
| 231 | 5' TRAJ18;/5PHOS/AT CAGGCCAGACAGTCAACTGAGTTCCTCTTC; |
| 232 | 5' TRAJ19;/5PHOS/AT TTGGAGTGACATTATGTTTGGATCCCTTTCC; |
| 233 | 5' TRAJ20;/5PHOS/AT TTGCTCTTACAGTTACTGTGGTTCCGGCTC; |
| 234 | 5' TRAJ21;/5PHOS/AT TTGGTTTTACATTGAGTTTGGTCCCAGATCC; |
| 235 | 5' TRAJ22;/5PHOS/AT CAGGTAAAACAGTCAATTGTGTCCCAGATCC; |
| 236 | 5' TRAJ23;/5PHOS/AT TGGGTTTCACAGATAACTCCGTTCCCTGT; |
| 237 | 5' TRAJ24;/5PHOS/AT CTGGGGTGACCACAACCTGGGTC; |
| 238 | 5' TRAJ25;/5PHOS/AT CTGGGGTGACCACAACCTGGGTC; |
| 239 | 5' TRAJ26;/5PHOS/AT AGGGCAGCACGGACAATCTGGTTC; |
| 240 | 5' TRAJ27;/5PHOS/AT TTGGCTTCACAGTGAGCGTAGTCCCATC; |
| 241 | 5' TRAJ28;/5PHOS/AT TTGGTATGACCGAGAGTTTGGTCCCCTT; |
| 242 | 5' TRAJ29;/5PHOS/AT TTGCAATCACAGAAAGTCTTGTGCCCTTTC; |
| 243 | 5' TRAJ30;/5PHOS/AT TGGGGAGAATATGAAGTCGTGTCCCTTTTC; |
| 244 | 5' TRAJ31;/5PHOS/AT TGGGCTTCACCACCAGCTGAGTTC; |
| 245 | 5' TRAJ32;/5PHOS/AT TTGGCTGGACAGCAAGCAGAGTGC; |
| 246 | 5' TRAJ33;/5PHOS/AT CTGGCTTTATAATTAGCTTGGTCCCAGCG; |
| 247 | 5' TRAJ34;/5PHOS/AT TTGGAAAGACTTGTAATCTGGTCCCAGTCC; |
| 248 | 5' TRAJ35;/5PHOS/AT GTGGTAAAACAATCACTTGAGTGCCGGAC; |
| 249 | 5' TRAJ36;/5PHOS/AT AGGGGAATAACGGTGAGTCTCGTTCCAGT; |
| 250 | 5' TRAJ37;/5PHOS/AT CTGGTTTTACTTGGTAAAGTTGTCCCTTGCC; |
| 251 | 5' TRAJ38;/5PHOS/AT TCGGATTTACTGCCAGGCTTGTTCCC; |
| 252 | 5' TRAJ39;/5PHOS/AT GGGGTTTGACCATTAACCTTGTTCCCC; |
| 253 | 5' TRAJ40;/5PHOS/AT TTGCTAAAACCTTCAGCCTGGTGCCTG; |
| 254 | 5' TRAJ41;/5PHOS/AT GGGGTGTGACCAACAGCGAGGTG; |
| 255 | 5' TRAJ42;/5PHOS/AT TTGGTTTAACAGAGAGTTTAGTGCCTTTTCCAAAGA; |
| 256 | 5' TRAJ43;/5PHOS/AT TTGGTTTTACTGTCAGTCTGGTCCCTGCTC; |
| 257 | 5' TRAJ44;/5PHOS/AT CGAGCGTGACCTGAAGTCTTGTTCCAGT; |
| 258 | 5' TRAJ45;/5PHOS/AT AGGGCTGGATGATTAGATGAGTCCCTTTG; |
| 259 | 5' TRAJ46;/5PHOS/AT TGGGCCTAACTGCTAAACGAGTCCCG; |
| 260 | 5' TRAJ47;/5PHOS/AT AGGACTTGACTCTCAGAATGGTTCCTGCG; |
| 261 | 5' TRAJ48;/5PHOS/AT TGGGTATGATGGTGAGTCTTGTTCCAGTCC; |
| 262 | 5' TRAJ49;/5PHOS/AT TTGGAATGACCGTCAAACTTGTCCCTGT; |
| 263 | 5' TRAJ50;/5PHOS/AT TTGGAATGACTGATAAGCTTGTCCCTGGC; |
| 264 | 5' TRAJ51;/5PHOS/AT TTGGCTTCACAGTTAGTCATGTCTCCTTTCC; |
| 265 | 5' TRAJ52;/5PHOS/AT TTGGATGGACAGTCAAGATGGTCCCTTG; |
| 266 | 5' TRAJ53;/5PHOS/AT TTGGATTCACGGTTAAGAGAGTTCCTTTTCC; |

| **SEQ ID NO:** | **Table 3-7: Beta V Inner Primers** |
|---|---|
| 267 | 5'TCRbV2;GAACCTGAAGTCACCCAGACTCCCAGC; |
| 268 | 5' TCRbV3-1;GCTGTTTCCCAGACTCCAAAATACCTGGTC; |
| 269 | 5' TCRbV4-1;GAAGTTACCCAGACACCAAAACACCTGGTC; |
| 270 | 5' TCRbV5-1;GGAGTCACTCAAACTCCAAGATATCTGATCAAAAC; |
| 271 | 5' TCRbV6-1 ;GGTGTCACTCAGACCCCAAAATTCCAG; |
| 272 | 5' TCRbV7-1;GGAGTCTCCCAGTCCCTGAGACACAAGG; |
| 273 | 5' TCRbV4-2;GGAGTTACGCAGACACCAAGACACCTGG; |
| 274 | 5' TCRbV6-2;GGTGTCACTCAGACCCCAAAATTCCG; |
| 275 | 5' TCRbV4-3;GGAGTTACGCAGACACCAAGACACCTGG; |
| 276 | 5' TCRbV6-3;GGTGTCACTCAGACCCCAAAATTCCG; |
| 277 | 5' TCRbV7-2;GGAGTCTCCCAGTCCCCCAGTAACAAG; |
| 278 | 5' TCRbV6-4;GGGATCACCCAGGCACCAACATCTC; |
| 279 | 5' TCRbV7-3;GGAGTCTCCCAGACCCCCAGTAACAAG; |
| 280 | 5' TCRbV5-3;GGAGTCACCCAAAGTCCCACACACCT; |
| 281 | 5' TCRbV9;GGAGTCACACAAACCCCAAAGCACCT; |
| 282 | 5' TCRbV10-1;GAAATCACCCAGAGCCCAAGACACAAGA; |
| 283 | 5' TCRbV11-1;GAAGTTGCCCAGTCCCCCAGATATAAGATTA; |
| 284 | 5' TCRbV10-2;GGAATCACCCAGAGCCCAAGATACAAGAT; |
| 285 | 5' TCRbV11-2;GGAGTTGCCCAGTCTCCCAGATATAAGATTATAGAG; |
| 286 | 5' TCRbV6-5;GGTGTCACTCAGACCCCAAAATTCCAG; |
| 287 | 5' TCRbV7-4;GGAGTCTCCCAGTCCCCAAGGTACAAAG; |
| 288 | 5' TCRbV5-4;GGAGTCACCCAAAGTCCCACACACCT; |
| 289 | 5' TCRbV6-6;GGTGTCACTCAGACCCCAAAATTCCG; |
| 290 | 5' TCRbV7-5;GGAGTCTCCCAGTCCCCAAGGTACGA; |
| 291 | 5' TCRbV5-5;GGAGTCACCCAAAGTCCCACACACCT; |
| 292 | 5' TCRbV6-7;GGTGTCACTCAGACCCCAAAATTCCAC; |
| 293 | 5' TCRbV7-6;GGAGTCTCCCAGTCTCCCAGGTACAAAGTC; |
| 294 | 5' TCRbV5-6;GGAGTCACCCAAAGTCCCACACACCT; |
| 295 | 5' TCRbV6-8;GGTGTCACTCAGACCCCAAAATTCCACAT; |
| 296 | 5' TCRbV7-7;GGAGTCTCCCAGTCTCCCAGGTACAAAGTC; |
| 297 | 5' TCRbV5-7;GGAGTCACCCAAAGTCCCACACACCT; |
| 298 | 5' TCRbV6-9;GGTGTCACTCAGACCCCAAAATTCCACAT; |
| 299 | 5' TCRbV7-8;GGAGTCTCCCAGTCCCCTAGGTACAAAGTC; |
| 300 | 5' TCRbV5-8;GGAGTCACACAAAGTCCCACACACCTGA; |
| 301 | 5' TCRbV7-9;GGAGTCTCCCAGAACCCCAGACACAAG; |
| 302 | 5' TCRbV13;GGAGTCATCCAGTCCCCAAGACATCTGAT; |
| 303 | 5' TCRbV10-3;GGAATCACCCAGAGCCCAAGACACAAG; |
| 304 | 5' TCRbV11-3;GGAGTGGTTCAGTCTCCCAGATATAAGATTATAGAGAA; |
| 305 | 5' TCRbV12-3;GGAGTTATCCAGTCACCCCGCCATG; |
| 306 | 5' TCRbV12-4;GGAGTTATCCAGTCACCCCGGCAC; |
| 307 | 5' TCRbV12-5;AGAGTCACCCAGACACCAAGGCACAAG; |
| 308 | 5' TCRbV14;GGAGTTACTCAGTTCCCCAGCCACAGC; |
| 309 | 5' TCRbV15;ATGGTCATCCAGAACCCAAGATACCAGGTT; |
| 310 | 5' TCRbV17;GAGCCTGGAGTCAGCCAGACCCC; |
| 311 | 5' TCRbV18;GGCGTCATGCAGAACCCAAGACAC; |
| 312 | 5' TCRbV19;GGAATCACTCAGTCCCCAAAGTACCTGTTCA; |
| 313 | 5' TCRbV20-1;GCTGTCGTCTCTCAACATCCGAGCTG; |
| 314 | 5' TCRbV22;ATTCCAGCTCACTGGGGCTGGATG; |
| 315 | 5' TCRbV23-1;AAAGTCACACAGACTCCAGGACATTTGGTCA; |
| 316 | 5' TCRbV24-1;GATGTTACCCAGACCCCAAGGAATAGGATC; |
| 317 | 5' TCRbV25-1;GACATCTACCAGACCCCAAGATACCTTGTTATAGG; |
| 318 | 5'TCRbV26;GTAGTTACACAATTCCCAAGACACAGAATCATTGG; |
| 319 | 5' TCRbV27;CAAGTGACCCAGAACCCAAGATACCTCATC |

| **SEQ ID NO:** | **Table 3-8: Beta J Inner Primers** |
|---|---|
| 320 | 3' TRBJ1-1;/5PHOS/TCCT CTACAACTGTGAGTCTGGTGCCTTGTCCAAA; |
| 321 | 3' TRBJ1-2;/5PHOS/TCCT CTACAACGGTTAACCTGGTCCCCGAAC; |
| 322 | 3' TRBJ1-3;/5PHOS/TCCT CTACAACAGTGAGCCAACTTCCCTCTCCAA; |
| 323 | 3' TRBJ1-4;/5PHOS/TCCT CCAAGACAGAGAGCTGGGTTCCACTGC; |
| 324 | 3' TRBJ1-5;/5PHOS/TCCT CTAGGATGGAGAGTCGAGTCCCATCACCA; |
| 325 | 3' TRBJ1-6;/5PHOS/TCCT CTGTCACAGTGAGCCTGGTCCCGTTC; |
| 326 | 3' TRBJ2-1;/5PHOS/TCCT CTAGCACGGTGAGCCGTGTCCCTG; |
| 327 | 3' TRBJ2-2;/5PHOS/TCCT CCAGTACGGTCAGCCTAGAGCCTTCTCC; |
| 328 | 3' TRBJ2-2P;/5PHOS/TCCT CCAGAACCAGGAGTCCTCCGCCC; |
| 329 | 3' TRBJ2-3;/5PHOS/TCCT CGAGCACTGTCAGCCGGGTGC; |
| 330 | 3' TRBJ2-4;/5PHOS/TCCT CCAGCACTGAGAGCCGGGTCCC; |
| 331 | 3' TRBJ2-5;/5PHOS/TCCT CGAGCACCAGGAGCCGCGTG; |
| 332 | 3' TRBJ2-6;/5PHOS/TCCT CCAGCACGGTCAGCCTGCTGC; |
| 333 | 3' TRBJ2-7;/5PHOS/TCCT CTGTGACCGTGAGCCTGGTGCC; |

| **SEQ ID NO:** | **Table3- 9: Alpha V Cloning Primers** | |
|---|---|---|
| | **Primer** | **Sequence (5' - 3')** |
| 334 | 5' TCRaV1-2 In-Fusion | TACAGGAGGGCTCGG CA GGACAAAACATTGACCAGCCCACTGAGAT |
| 335 | 5' TCRaV2 In-Fusion | TACAGGAGGGCTCGG CA AAGGACCAAGTGTTTCAGCCTTCCACAGTG |
| 336 | 5' TCRaV3 In-Fusion | TACAGGAGGGCTCGG CA CAGTCAGTGGCTCAGCCGGAAGATC |
| 337 | 5' TCRaV4 In-Fusion | TACAGGAGGGCTCGG CA AAGACCACCCAGCCCATCTCCATG |
| 338 | 5' TCRaV5 In-Fusion | TACAGGAGGGCTCGG CA AGGATGTGGAGCAGAGTCTTTTCCTGAGTG |
| 339 | 5' TCRaV6 In-Fusion | TACAGGAGGGCTCGG CA CAAAAGATAGAACAGAATTCCGAGGCCCTG |
| 340 | 5' TCRaV7 In-Fusion | TACAGGAGGGCTCGG CA GAAAACCAGGTGGAGCACAGCCCTC |
| 341 | 5' TCRaV8-1 In-Fusion | TACAGGAGGGCTCGG CA CAGTCTGTGAGCCAGCATAACCACCAC |
| 342 | 5' TCRaV8-2 In-Fusion | TACAGGAGGGCTCGG CA CAGTCGGTGACCCAGCTTGACAGC |
| 343 | 5' TCRaV8-3 In-Fusion | TACAGGAGGGCTCGG CA CAGTCAGTGACCCAGCCTGACATCCAC |
| 344 | 5' TCRaV8-4 In-Fusion | TACAGGAGGGCTCGG CA CAGTCGGTGACCCAGCTTGGCAG |
| 345 | 5' TCRaV9-1 In-Fusion | TACAGGAGGGCTCGG CA GATTCAGTGGTCCAGACAGAAGGCCAAGT |
| 346 | 5' TCRaV10 In-Fusion | TACAGGAGGGCTCGG CA AAAAACCAAGTGGAGCAGAGTCCTCAGTCC |
| 347 | 5' TCRaV12-1In-Fusion | TACAGGAGGGCTCGG CA CAACGGAAGGAGGTGGAGCAGGATC |
| 348 | 5'TCRaV12-2 In-Fusion | TACAGGAGGGCTCGG CA CAACAGAAGGAGGTGGAGCAGAATTCTGG |
| 349 | 5'TCRaV13-1 In-Fusion | TACAGGAGGGCTCGG CA GAGAATGTGGAGCAGCATCCTTCAACC |
| 350 | 5'TCRaV13-2 In-Fusion | TACAGGAGGGCTCGG CA GAGAGTGTGGGGCTGCATCTTCCTACC |
| 351 | 5'TCRaV14D4In-Fusion | TACAGGAGGGCTCGGCACAGAAGATAACTCAAACCCAACCAGGAATGTTC |
| 352 | 5' TCRaV9-2 In-Fusion | TACAGGAGGGCTCGG CA AATTCAGTGACCCAGATGGAAGGGCC |
| 353 | 5'TCRaV12-3 In-Fusion | TACAGGAGGGCTCGG CA CAACAGAAGGAGGTGGAGCAGGATCCT |
| 354 | 5' TCRaV8-6 In-Fusion | TACAGGAGGGCTCGG CA CAGTCTGTGACCCAGCTTGACAGCCA |
| 355 | 5' TCRav16 In-Fusion | TACAGGAGGGCTCGG CA CAGAGAGTGACTCAGCCCGAGAAGCTC |
| 356 | 5' TCRaV17 In-Fusion | TACAGGAGGGCTCGG CA CAACAGGGAGAAGAGGATCCTCAGGCC |
| 357 | 5' TCRaV18 In-Fusion | TACAGGAGGGCTCGG CA GACTCGGTTACCCAGACAGAAGGCCC |
| 358 | 5' TCRaV19 In-Fusion | TACAGGAGGGCTCGGCACAGAAGGTAACTCAAGCGCAGACTGAAATTTCT |
| 359 | 5' TCRaV20 In-Fusion | TACAGGAGGGCTCGG CA GAAGACCAGGTGACGCAGAGTCCCG |
| 360 | 5' TCRaV21 In-Fusion | TACAGGAGGGCTCGG CA AAACAGGAGGTGACGCAGATTCCTGC |
| 361 | 5' TCRaV22 In-Fusion | TACAGGAGGGCTCGG CA ATACAAGTGGAGCAGAGTCCTCCAGACCTGA |
| 362 | 5'TCRaV23DV6In-Fusion | TACAGGAGGGCTCGG CA CAACAGAAGGAGAAAAGTGACCAGCAGCA |
| 363 | 5' TCRaV24 In-Fusion | TACAGGAGGGCTCGGCATACTGAACGTGGAACAAAGTCCTCAGTCACTG |
| 364 | 5' TCRaV25 In-Fusion | TACAGGAGGGCTCGG CA CAACAGGTAATGCAAATTCCTCAGTACCAGC |
| 365 | 5'TCRaV26-1 In-Fusion | TACAGGAGGGCTCGG CA AAGACCACCCAGCCCCCCTCC |
| 366 | 5' TCRaV8-7 In-Fusion | TACAGGAGGGCTCGG CA CAGTCGGTGACCCAGCTTGATGGC |
| 367 | 5' TCRaV27 In-Fusion | TACAGGAGGGCTCGG CA CAGCTGCTGGAGCAGAGCCCTCAGT |
| 368 | 5'TCRaV29DV5In-Fusion | TACAGGAGGGCTCGGCA CAACAGAAGAATGATGACCAGCAAGTTAAGCAA |
| 369 | 5' TCRaV30 In-Fusion | TACAGGAGGGCTCGG CA CAACAACCAGTGCAGAGTCCTCAAGCC |
| 370 | 5'TCRaV26-2 In-Fusion | TACAGGAGGGCTCGGCA AGACCACACAGCCAAATTCAATGGAGAGTAAC |
| 371 | 5' TCRaV34 In-Fusion | TACAGGAGGGCTCGG CA CAAGAACTGGAGCAGAGTCCTCAGTCCTTG |
| 372 | 5' TCRaV35 In-Fusion | TACAGGAGGGCTCGGCACAACAGCTGAATCAGAGTCCTCAATCTATGTTT ATC |
| 373 | 5'TCRaV36DV7 In-Fusion | TACAGGAGGGCTCGGCA AAGACAAGGTGGTACAAAGCCCTCTATCTCTG |
| 374 | 5'TCRaV382DV8InFusi on | TACAGGAGGGCTCGGCA AGACAGTCACTCAGTCTCAACCAGAGATGTCT |
| 375 | 5' TCRaV39 In-Fusion | TACAGGAGGGCTCGG CA GAGCTGAAAGTGGAACAAAACCCTCTGTTC |
| 376 | 5' TCRaV40 In-Fusion | TACAGGAGGGCTCGG CA AATTCAGTCAAGCAGACGGGCCAAATAAC |
| 377 | 5' TCRaV41 In-Fusion | TACAGGAGGGCTCGG CA GCCAAAAATGAAGTGGAGCAGAGTCCTC |

| **SEQ ID NO:** | **Table 3-10: Alpha J Cloning Primers** | |
|---|---|---|
| | **Primer** | **Sequence (5' - 3')** |
| 378 | 5' TRAJ1 In-Fusion | GTCAGGGTTCTGGATAT GGGGAGAAGTGGAAACTCTGGTTCC |
| 379 | 5' TRAJ2 In-Fusion | GTCAGGGTTCTGGATAT CAGATATAATGAATACATGGGTCCCTTTCCCA |
| 380 | 5' TRAJ3 In-Fusion | GTCAGGGTTCTGGATAT TTGGCCGGATGCTGAGTCTGGTC |
| 381 | 5' TRAJ4 In-Fusion | GTCAGGGTTCTGGATAT ATGGGTGTACAGCCAGCCTGGTCCC |
| 382 | 5' TRAJ5 In-Fusion | GTCAGGGTTCTGGATAT TTGGTTGCACTTGGAGTCTTGTTCCACTC |
| 383 | 5' TRAJ6 In-Fusion | GTCAGGGTTCTGGATAT ACGGATGAACAATAAGGCTGGTTCCTCTTC |
| 384 | 5' TRAJ7 In-Fusion | GTCAGGGTTCTGGATAT TTGGTATGACCACCACTTGGTTCCCCTT |
| 385 | 5' TRAJ8 In-Fusion | GTCAGGGTTCTGGATAT TTGGACTGACCAGAAGTCGGGTGCC |
| 386 | 5' TRAJ9 In-Fusion | GTCAGGGTTCTGGATAT TTGCTTTAACAAATAGTCTTGTTCCTGCTCCAAAG |
| 387 | 5' TRAJ10 In-Fusion | GTCAGGGTTCTGGATAT TGAGTTCCACTTTTAGCTGAGTGCCTGTCC |
| 388 | 5' TRAJ11 In-Fusion | GTCAGGGTTCTGGATAT CTGGAGAGACTAGAAGCATAGTCCCCTTCCC |
| 389 | 5' TRAJ12 In-Fusion | GTCAGGGTTCTGGATAT CAGGCCTGACCAGCAGTCTGGTCC |
| 390 | 5' TRAJ13 In-Fusion | GTCAGGGTTCTGGATAT TTGGGATGACTTGGAGCTTTGTTCCAAT |
| 391 | 5' TRAJ14 In-Fusion | GTCAGGGTTCTGGATAT CAGGTTTTACTGATAATCTTGTCCCACTCCCA |
| 392 | 5' TRAJ15 In-Fusion | GTCAGGGTTCTGGATAT TGGAACTCACTGATAAGGTGGGTTCCCTTC |
| 393 | 5' TRAJ16 In-Fusion | GTCAGGGTTCTGGATAT TAAGATCCACCTTTAACATGGTTCCCCTTG |
| 394 | 5' TRAJ17 In-Fusion | GTCAGGGTTCTGGATAT TTGGTTTAACTAGCACCCTGGTTCCTCCTC |
| 395 | 5' TRAJ18 In-Fusion | GTCAGGGTTCTGGATAT CAGGCCAGACAGTCAACTGAGTTCCTCTTC |
| 396 | 5' TRAJ19 In-Fusion | GTCAGGGTTCTGGATAT TTGGAGTGACATTATGTTTGGATCCCTTTCC |
| 397 | 5' TRAJ20 In-Fusion | GTCAGGGTTCTGGATAT TTGCTCTTACAGTTACTGTGGTTCCGGCTC |
| 398 | 5' TRAJ21 In-Fusion | GTCAGGGTTCTGGATAT TTGGTTTTACATTGAGTTTGGTCCCAGATCC |
| 399 | 5' TRAJ22 In-Fusion | GTCAGGGTTCTGGATAT CAGGTAAAACAGTCAATTGTGTCCCAGATCC |
| 400 | 5' TRAJ23 In-Fusion | GTCAGGGTTCTGGATAT TGGGTTTCACAGATAACTCCGTTCCCTGT |
| 401 | 5' TRAJ24 In-Fusion | GTCAGGGTTCTGGATAT CTGGGGTGACCACAACCTGGGTC |
| 402 | 5' TRAJ25 In-Fusion | GTCAGGGTTCTGGATAT CTGGGGTGACCACAACCTGGGTC |
| 403 | 5' TRAJ26 In-Fusion | GTCAGGGTTCTGGATAT AGGGCAGCACGGACAATCTGGTTC |
| 404 | 5' TRAJ27 In-Fusion | GTCAGGGTTCTGGATAT TTGGCTTCACAGTGAGCGTAGTCCCATC |
| 405 | 5' TRAJ28 In-Fusion | GTCAGGGTTCTGGATAT TTGGTATGACCGAGAGTTTGGTCCCCTT |
| 406 | 5' TRAJ29 In-Fusion | GTCAGGGTTCTGGATAT TTGCAATCACAGAAAGTCTTGTGCCCTTTC |
| 407 | 5' TRAJ30 In-Fusion | GTCAGGGTTCTGGATAT TGGGGAGAATATGAAGTCGTGTCCCTTTTC |
| 408 | 5' TRAJ31 In-Fusion | GTCAGGGTTCTGGATAT TGGGCTTCACCACCAGCTGAGTTC |
| 409 | 5' TRAJ32 In-Fusion | GTCAGGGTTCTGGATAT TTGGCTGGACAGCAAGCAGAGTGC |
| 410 | 5' TRAJ33 In-Fusion | GTCAGGGTTCTGGATAT CTGGCTTTATAATTAGCTTGGTCCCAGCG |
| 411 | 5' TRAJ34 In-Fusion | GTCAGGGTTCTGGATAT TTGGAAAGACTTGTAATCTGGTCCCAGTCC |
| 412 | 5' TRAJ35 In-Fusion | GTCAGGGTTCTGGATAT GTGGTAAAACAATCACTTGAGTGCCGGAC |
| 413 | 5' TRAJ36 In-Fusion | GTCAGGGTTCTGGATAT AGGGGAATAACGGTGAGTCTCGTTCCAGT |
| 414 | 5' TRAJ37 In-Fusion | GTCAGGGTTCTGGATAT CTGGTTTTACTTGGTAAAGTTGTCCCTTGCC |
| 415 | 5' TRAJ38 In-Fusion | GTCAGGGTTCTGGATAT TCGGATTTACTGCCAGGCTTGTTCCC |
| 416 | 5' TRAJ39 In-Fusion | GTCAGGGTTCTGGATAT GGGGTTTGACCATTAACCTTGTTCCCC |
| 417 | 5' TRAJ40 In-Fusion | GTCAGGGTTCTGGATAT TTGCTAAAACCTTCAGCCTGGTGCCTG |
| 418 | 5' TRAJ41 In-Fusion | GTCAGGGTTCTGGATAT GGGGTGTGACCAACAGCGAGGTG |
| 419 | 5' TRAJ42 In-Fusion | GTCAGGGTTCTGGATATTTGGTTTAACAGAGAGTTTAGTGCCTTTTCCAAAGA |
| 420 | 5' TRAJ43 In-Fusion | GTCAGGGTTCTGGATAT TTGGTTTTACTGTCAGTCTGGTCCCTGCTC |
| 421 | 5' TRAJ44 In-Fusion | GTCAGGGTTCTGGATAT CGAGCGTGACCTGAAGTCTTGTTCCAGT |
| 422 | 5' TRAJ45 In-Fusion | GTCAGGGTTCTGGATAT AGGGCTGGATGATTAGATGAGTCCCTTTG |
| 423 | 5' TRAJ46 In-Fusion | GTCAGGGTTCTGGATAT TGGGCCTAACTGCTAAACGAGTCCCG |
| 424 | 5' TRAJ47 In-Fusion | GTCAGGGTTCTGGATAT AGGACTTGACTCTCAGAATGGTTCCTGCG |
| 425 | 5' TRAJ48 In-Fusion | GTCAGGGTTCTGGATAT TGGGTATGATGGTGAGTCTTGTTCCAGTCC |
| 426 | 5' TRAJ49 In-Fusion | GTCAGGGTTCTGGATAT TTGGAATGACCGTCAAACTTGTCCCTGT |
| 427 | 5' TRAJ50 In-Fusion | GTCAGGGTTCTGGATAT TTGGAATGACTGATAAGCTTGTCCCTGGC |
| 428 | 5' TRAJ51 In-Fusion | GTCAGGGTTCTGGATAT TTGGCTTCACAGTTAGTCATGTCTCCTTTCC |
| 429 | 5' TRAJ52 In-Fusion | GTCAGGGTTCTGGATAT TTGGATGGACAGTCAAGATGGTCCCTTG |
| 430 | 5' TRAJ53 In-Fusion | GTCAGGGTTCTGGATAT TTGGATTCACGGTTAAGAGAGTTCCTTTTCC |
| 431 | 5' TRAJ54 In-Fusion | GTCAGGGTTCTGGATAT TTGGGTTGATAGTCAGCCTGGTTCCTTG |
| 432 | 5' TRAJ55 In-Fusion | GTCAGGGTTCTGGATAT TTGGATTTATTTTTGTACTCATCCCCTTTCCC |
| 433 | 5' TRAJ56 In-Fusion | GTCAGGGTTCTGGATATCTGGTCTAACACTCAGAGTTATTCCTTTTCCAAATGT C |
| 434 | 5' TRAJ57 In-Fusion | GTCAGGGTTCTGGATAT ATGGGTTTACTGTCAGTTTCGTTCCCTTTCC |
| 435 | 5' TRAJ58 In-Fusion | GTCAGGGTTCTGGATAT CAGGATTCACTGTGAGCTGTGTTCCTTCC |
| 436 | 5' TRAJ59 In-Fusion | GTCAGGGTTCTGGATAT TCACTCTCACTTGCGTCCCCATTCC |
| 437 | 5' TRAJ60 In-Fusion | GTCAGGGTTCTGGATAT CCAGGCTCACAATTAACTCAGTCCCCTTC |
| 438 | 5' TRAJ61 In-Fusion | GTCAGGGTTCTGGATAT TGAGTTTCATGATTCCTCTAGTGTTGGCTCC |

| **SEQ ID NO:** | **Table 3-11: Beta V Cloning Primers** | |
|---|---|---|
| | **Primer** | **Sequence (5' - 3')** |
| 439 | 5' TCRbV2 In-Fusion | CAAGAGGGCTCGGCA GAACCTGAAGTCACCCAGACTCCCAGC |
| 440 | 5' TCRbV3-1 In-Fusion | CAAGAGGGCTCGGCA GCTGTTTCCCAGACTCCAAAATACCTGGTC |
| 441 | 5' TCRbV4-1 In-Fusion | CAAGAGGGCTCGGCA GAAGTTACCCAGACACCAAAACACCTGGTC |
| 442 | 5' TCRbV5-1 In-Fusion | CAAGAGGGCTCGGCA GGAGTCACTCAAACTCCAAGATATCTGATCAAAAC |
| 443 | 5' TCRbV6-1 In-Fusion | CAAGAGGGCTCGGCA GGTGTCACTCAGACCCCAAAATTCCAG |
| 444 | 5' TCRbV7-1 In-Fusion | CAAGAGGGCTCGGCA GGAGTCTCCCAGTCCCTGAGACACAAGG |
| 445 | 5' TCRbV4-2 In-Fusion | CAAGAGGGCTCGGCA GGAGTTACGCAGACACCAAGACACCTGG |
| 446 | 5' TCRbV6-2 In-Fusion | CAAGAGGGCTCGGCA GGTGTCACTCAGACCCCAAAATTCCG |
| 447 | 5' TCRbV4-3 In-Fusion | CAAGAGGGCTCGGCA GGAGTTACGCAGACACCAAGACACCTGG |
| 448 | 5' TCRbV6-3 In-Fusion | CAAGAGGGCTCGGCA GGTGTCACTCAGACCCCAAAATTCCG |
| 449 | 5' TCRbV7-2 In-Fusion | CAAGAGGGCTCGGCA GGAGTCTCCCAGTCCCCCAGTAACAAG |
| 450 | 5' TCRbV6-4 In-Fusion | CAAGAGGGCTCGGCA GGGATCACCCAGGCACCAACATCTC |
| 451 | 5' TCRbV7-3 In-Fusion | CAAGAGGGCTCGGCA GGAGTCTCCCAGACCCCCAGTAACAAG |
| 452 | 5' TCRbV5-3 In-Fusion | CAAGAGGGCTCGGCA GGAGTCACCCAAAGTCCCACACACCT |
| 453 | 5' TCRbV9 In-Fusion | CAAGAGGGCTCGGCA GGAGTCACACAAACCCCAAAGCACCT |
| 454 | 5'TCRbV10-1In-Fusion | CAAGAGGGCTCGGCA GAAATCACCCAGAGCCCAAGACACAAGA |
| 455 | 5'TCRbV11-1In-Fusion | CAAGAGGGCTCGGCA GAAGTTGCCCAGTCCCCCAGATATAAGATTA |
| 456 | 5'TCRbV10-2In-Fusion | CAAGAGGGCTCGGCA GGAATCACCCAGAGCCCAAGATACAAGAT |
| 457 | 5'TCRbV311-2In-Fusion | CAAGAGGGCTCGGCA GGAGTTGCCCAGTCTCCCAGATATAAGATTATAGAG |
| 458 | 5' TCRbV6-5 In-Fusion | CAAGAGGGCTCGGCA GGTGTCACTCAGACCCCAAAATTCCAG |
| 459 | 5' TCRbV7-4 In-Fusion | CAAGAGGGCTCGGCA GGAGTCTCCCAGTCCCCAAGGTACAAAG |
| 460 | 5' TCRbV5-4 In-Fusion | CAAGAGGGCTCGGCA GGAGTCACCCAAAGTCCCACACACCT |
| 461 | 5' TCRbV6-6 In-Fusion | CAAGAGGGCTCGGCA GGTGTCACTCAGACCCCAAAATTCCG |
| 462 | 5' TCRbV7-5 In-Fusion | CAAGAGGGCTCGGCA GGAGTCTCCCAGTCCCCAAGGTACGA |
| 463 | 5' TCRbV5-5 In-Fusion | CAAGAGGGCTCGGCA GGAGTCACCCAAAGTCCCACACACCT |
| 464 | 5' TCRbV6-7 In-Fusion | CAAGAGGGCTCGGCA GGTGTCACTCAGACCCCAAAATTCCAC |
| 465 | 5' TCRbV7-6 In-Fusion | CAAGAGGGCTCGGCA GGAGTCTCCCAGTCTCCCAGGTACAAAGTC |
| 466 | 5' TCRbV5-6 In-Fusion | CAAGAGGGCTCGGCA GGAGTCACCCAAAGTCCCACACACCT |
| 467 | 5' TCRbV6-8 In-Fusion | CAAGAGGGCTCGGCA GGTGTCACTCAGACCCCAAAATTCCACAT |
| 468 | 5' TCRbV7-7 In-Fusion | CAAGAGGGCTCGGCA GGAGTCTCCCAGTCTCCCAGGTACAAAGTC |
| 469 | 5' TCRbV5-7 In-Fusion | CAAGAGGGCTCGGCA GGAGTCACCCAAAGTCCCACACACCT |
| 470 | 5' TCRbV6-9 In-Fusion | CAAGAGGGCTCGGCA GGTGTCACTCAGACCCCAAAATTCCACAT |
| 471 | 5' TCRbV7-8 In-Fusion | CAAGAGGGCTCGGCA GGAGTCTCCCAGTCCCCTAGGTACAAAGTC |
| 472 | 5' TCRbV5-8 In-Fusion | CAAGAGGGCTCGGCA GGAGTCACACAAAGTCCCACACACCTGA |
| 473 | 5' TCRbV7-9 In-Fusion | CAAGAGGGCTCGGCA GGAGTCTCCCAGAACCCCAGACACAAG |
| 474 | 5' TCRbV13 In-Fusion | CAAGAGGGCTCGGCA GGAGTCATCCAGTCCCCAAGACATCTGAT |
| 475 | 5'TCRbV10-3In-Fusion | CAAGAGGGCTCGGCA GGAATCACCCAGAGCCCAAGACACAAG |
| 476 | 5'TCRbV11-3In-Fusion | CAAGAGGGCTCGGCAGGAGTGGTTCAGTCTCCCAGATATAAGATTATA GAGAA |
| 477 | 5'TCRbV12-3In-Fusion | CAAGAGGGCTCGGCA GGAGTTATCCAGTCACCCCGCCATG |
| 478 | 5'TCRbV12-4In-Fusion | CAAGAGGGCTCGGCA GGAGTTATCCAGTCACCCCGGCAC |
| 479 | 5'TCRbV12-5In-Fusion | CAAGAGGGCTCGGCA AGAGTCACCCAGACACCAAGGCACAAG |
| 480 | 5' TCRbV14 In-Fusion | CAAGAGGGCTCGGCA GGAGTTACTCAGTTCCCCAGCCACAGC |
| 481 | 5' TCRbV15 In-Fusion | CAAGAGGGCTCGGCA ATGGTCATCCAGAACCCAAGATACCAGGTT |
| 482 | 5' TCRbV17 In-Fusion | CAAGAGGGCTCGGCA GAGCCTGGAGTCAGCCAGACCCC |
| 483 | 5' TCRbV18 In-Fusion | CAAGAGGGCTCGGCA GGCGTCATGCAGAACCCAAGACAC |
| 484 | 5' TCRbV19 In-Fusion | CAAGAGGGCTCGGCA GGAATCACTCAGTCCCCAAAGTACCTGTTCA |
| 485 | 5'TCRbV20-1In-Fusion | CAAGAGGGCTCGGCA GCTGTCGTCTCTCAACATCCGAGCTG |
| 486 | 5' TCRbV22 In-Fusion | CAAGAGGGCTCGGCA ATTCCAGCTCACTGGGGCTGGATG |
| 487 | 5'TCRbV23-1In-Fusion | CAAGAGGGCTCGGCA AAAGTCACACAGACTCCAGGACATTTGGTCA |
| 488 | 5'TCRbV24-1In-Fusion | CAAGAGGGCTCGGCA GATGTTACCCAGACCCCAAGGAATAGGATC |
| 489 | 5'TCRbV25-1In-Fusion | CAAGAGGGCTCGGCA GACATCTACCAGACCCCAAGATACCTTGTTATAGG |
| 490 | 5' TCRbV26 In-Fusion | CAAGAGGGCTCGGCA GTAGTTACACAATTCCCAAGACACAGAATCATTGG |
| 491 | 5' TCRbV27 In-Fusion | CAAGAGGGCTCGGCA CAAGTGACCCAGAACCCAAGATACCTCATC |
| 492 | 5' TCRbV28 In-Fusion | CAAGAGGGCTCGGCA TCGAGATATCTAGTCAAAAGGACGGAGAGAAA |
| 493 | 5'TCRbV29-1In-Fusion | CAAGAGGGCTCGGCA GCTGTCATCTCTCAAAAGCCAAGCAGG |

| **SEQ ID NO:** | **Table 3-12: Beta J Cloning Primers** | |
|---|---|---|
| | **Primer** | **Sequence (5' - 3')** |
| 494 | 3'TRBJ1-1In-Fusion | AACACCTTGTTCAGGTCCT CTACAACTGTGAGTCTGGTGCCTTGTCCAAA |
| 495 | 3' TRBJ1-2 In-Fusion | AACACCTTGTTCAGGTCCT CTACAACGGTTAACCTGGTCCCCGAAC |
| 496 | 3' TRBJ1-3 In-Fusion | AACACCTTGTTCAGGTCCT CTACAACAGTGAGCCAACTTCCCTCTCCAA |
| 497 | 3' TRBJ1-4 In-Fusion | AACACCTTGTTCAGGTCCT CCAAGACAGAGAGCTGGGTTCCACTGC |
| 498 | 3' TRBJ1-5 In-Fusion | AACACCTTGTTCAGGTCCT CTAGGATGGAGAGTCGAGTCCCATCACCA |
| 499 | 3' TRBJ1-6 In-Fusion | AACACCTTGTTCAGGTCCT CTGTCACAGTGAGCCTGGTCCCGTTC |
| 500 | 3' TRBJ2-1 In-Fusion | AACACCTTGTTCAGGTCCT CTAGCACGGTGAGCCGTGTCCCTG |
| 501 | 3' TRBJ2-2 In-Fusion | AACACCTTGTTCAGGTCCT CCAGTACGGTCAGCCTAGAGCCTTCTCC |
| 502 | 3' TRBJ2-2P In-Fusion | AACACCTTGTTCAGGTCCT CCAGAACCAGGAGTCCTCCGCCC |
| 503 | 3' TRBJ2-3 In-Fusion | AACACCTTGTTCAGGTCCT CGAGCACTGTCAGCCGGGTGC |
| 504 | 3' TRBJ2-4 In-Fusion | AACACCTTGTTCAGGTCCT CCAGCACTGAGAGCCGGGTCCC |
| 505 | 3' TRBJ2-5 In-Fusion | AACACCTTGTTCAGGTCCT CGAGCACCAGGAGCCGCGTG |
| 506 | 3' TRBJ2-6 In-Fusion | AACACCTTGTTCAGGTCCT CCAGCACGGTCAGCCTGCTGC |
| 507 | 3' TRBJ2-7 In-Fusion | AACACCTTGTTCAGGTCCT CTGTGACCGTGAGCCTGGTGCC |

| **SEQ ID NO:** | **Table 3-13: Homologous Primers** | |
|---|---|---|
| | **Primer** | **Sequence (5' - 3')** |
| 508 | 5' Alpha Ext-Universal In-Fusion | CGTGGT TACAGGAGGGCTCGGCA |
| 509 | 3' Alpha Ext-Universal In-Fusion | CACGGCAGG GTCAGGGTTCTGGATAT |
| 510 | 5' Beta Ext-Universal In-Fusion | TGGCTC CAAGAGGGCTCGGCA |
| 511 | 3' Beta Ext-Universal In-Fusion | GGGTGGG AACACCTTGTTCAGGTCCT |

| **Table 3-14: TCR Isolation PCR Protocols** | | |
|---|---|---|
| **Protocol for Outer Primers (round 1)** | | |
| Step | Temperature | Time |
| 1 | 95°C | 30sec |
| 2 | 77°C | 45sec |
| 3 | 77°C | 30sec |
| 4 | 95°C | 30sec |
| 5 | 76°C | 45sec |
| 6 | 76°C | 30sec |
| 7 | 95°C | 30sec |
| 8 | 75°C | 45sec |
| 9 | 75°C | 30sec |
| 10 | 95°C | 30sec |
| 11 | 74°C | 45sec |
| 12 | 74°C | 30sec |
| 13 | 95°C | 30sec |
| 14 | 73°C | 45sec |
| 15 | 73°C | 30sec |
| 16 | 95°C | 30sec |
| 17 | 72°C | 45sec |
| 18 | 72°C | 30sec |
| 19 | 95°C | 30sec |
| 20 | 71°C | 45sec |
| 21 | 71°C | 30sec |
| 22 | 95°C | 30sec |
| 23 | 70°C | 45sec |
| 24 | 70°C | 30sec |
| 25 | 95°C | 30sec |
| 26 | 69°C | 45sec |
| 27 | 70°C | 30sec |
| 28 | 95°C | 30sec |
| 29 | 68°C | 45sec |
| 30 | 70°C | 30sec |
| 31 | 95°C | 30sec |
| 32 | 67°C | 45sec |
| 33 | 70°C | 30sec |
| 34 | 95°C | 30sec |
| 35 | 66°C | 45sec |
| 36 | 70°C | 30sec |

| **Protocol for Inner Primers (round 2)** | | |
|---|---|---|
| Step | Temperature | Time |
| 1 | 95°C | 30sec |
| 2 | 77°C | 4:00min |
| 3 | 77°C | 30sec |
| 4 | 95°C | 30sec |
| 5 | 76°C | 4:00min |
| 6 | 76°C | 30sec |
| 7 | 95°C | 30sec |
| 8 | 75°C | 4:00min |
| 9 | 75°C | 30sec |
| 10 | 95°C | 30sec |
| 11 | 74°C | 4:00min |
| 12 | 74°C | 30sec |
| 13 | 95°C | 30sec |
| 14 | 73°C | 4:00min |
| 15 | 73°C | 30sec |
| 16 | 95°C | 30sec |
| 17 | 72°C | 4:00min |
| 18 | 72°C | 30sec |
| 19 | 95°C | 30sec |
| 20 | 71°C | 4:00min |
| 21 | 71°C | 30sec |
| 22 | 95°C | 30sec |
| 23 | 70°C | 4:00min |
| 24 | 70°C | 30sec |
| 25 | 95°C | 30sec |
| 26 | 69°C | 4:00min |
| 27 | 70°C | 30sec |
| 28 | 95°C | 30sec |
| 29 | 68°C | 4:00min |
| 30 | 70°C | 30sec |
| 31 | 95°C | 30sec |
| 32 | 67°C | 4:00min |
| 33 | 70°C | 30sec |
| 34 | 95°C | 30sec |
| 35 | 66°C | 4:00min |
| 36 | 70°C | 30sec |

| **Protocol for Cloning Primers (round 3)** | | |
|---|---|---|
| Step | Temperature | Time |
| 1 | 95°C | 30sec |
| 2 | 77°C | 4:00min |
| 3 | 77°C | 30sec |
| 4 | 95°C | 30sec |
| 5 | 76°C | 4:00min |
| 6 | 76°C | 30sec |
| 7 | 95°C | 30sec |
| 8 | 75°C | 4:00min |
| 9 | 75°C | 30sec |
| 10 | 95°C | 30sec |
| 11 | 74°C | 4:00min |
| 12 | 74°C | 30sec |
| 13 | 95°C | 30sec |
| 14 | 73°C | 4:00min |
| 15 | 73°C | 30sec |
| 16 | 95°C | 30sec |
| 17 | 72°C | 4:00min |
| 18 | 72°C | 30sec |
| 19 | 95°C | 30sec |
| 20 | 71°C | 4:00min |
| 21 | 71°C | 30sec |
| 22 | 95°C | 30sec |
| 23 | 70°C | 4:00min |
| 24 | 70°C | 30sec |
| 25 | 95°C | 30sec |
| 26 | 69°C | 4:00min |
| 27 | 70°C | 30sec |
| 28 | 95°C | 30sec |
| 29 | 68°C | 4:00min |
| 30 | 70°C | 30sec |
| 31 | 95°C | 30sec |
| 32 | 67°C | 4:00min |
| 33 | 70°C | 30sec |

| **Protocol for Homologous Primers (round 4)** | | |
|---|---|---|
| Step | Temperature | Time |
| 1 | 95°C | 2:00min |
| 2 | 95°C | 20sec |
| 3 | 65°C | 10sec |
| 4 | 70°C | 10sec |

| Go to Step 4 and Repeat 45 Cycles | | |
|---|---|---|
| 5 | 4°C | Hold |

### Example 4: NA-17 TCR Isolation

NA-17 is a melanoma-specific antigen. T Cells were obtained from a patient sample and stained with an NY-ESO specific tetramer. Genomic DNA from eight NA-17⁺ CD3⁺ cells was amplified and used as template for a TCR isolation protocol. These cells yielded four distinct alpha chains and five distinct beta chains: TRAV12-1J11, TRAV12-3J9, TRAV16J28, TRAV17J10, TRBV12-3J1-6, TRBV27J1-2, TRBV27J1-6, TRBV5-5J1-3, TRBV5-6J1-1. Some chains were found to be expressed in multiple cells. Without subscribing to any one theory, these results may indicate that the cells were clonal or that individual wells contained multiple cells, making pairing ambiguous.

## Claims

1. A method of isolating DNA encoding the variable regions of a T cell receptor (TCR) alpha or beta chain, said method comprising: (a) isolating genomic DNA from a single T cell, wherein said isolating step comprises whole genome amplification; and (b) amplifying a gene segment encompassing the TCR alpha or beta chain variable region by an enrichment amplification reaction to produce a first enrichment product comprising the V and J regions of the TCR alpha or beta chain from the single cell, the amplification reaction comprising incubating the isolated genomic DNA with a set of outer forward primers and a set of outer reverse primers, wherein each outer forward primer anneals to a sequence 5 to 40 base pairs upstream of the signal sequence junction of the V region, the set of outer forward primers comprising at least one primer for each of the possible V regions of the TCR alpha chain or TCR beta chain, and wherein each outer reverse primer anneals to a sequence 5 to 40 base pairs downstream of the exon/intron junction of the J region, the set of outer reverse primers comprising at least one primer for each of the possible J regions of the TCR alpha chain or TCR beta chain.

2. The method of claim 1, wherein the first enrichment product is further amplified in an isolation amplification reaction to produce a second isolation product, the isolation amplification reaction comprising incubating the first enrichment product with a set of inner forward primers and set of inner reverse primers, wherein each inner forward primer anneals to a sequence at the start of the first amino acid downstream of the signal sequence of the V region, the set of inner forward primers comprising at least one primer for each of the possible V regions of the TCR alpha chain or TCR beta chain, and wherein each inner reverse primer anneals to a sequence at the downstream end of the J region, the set of inner reverse primers comprising at least one primer for each of the possible J regions of the TCR alpha chain or TCR beta chain.

3. The method of claim 2, additionally comprising sequencing the isolation product.

4. The method of claim 2, wherein the second isolation product is further amplified in a cloning amplification reaction to produce a third cloning product, the cloning amplification reaction comprising incubating the second isolation product with a set of cloning forward primers and a set of cloning reverse primers, wherein each cloning forward primer anneals to a sequence that overlaps the sequence of an inner forward primer, the set of cloning forward primers comprising at least one primer for each of the possible V regions of the TCR alpha chain or TCR beta chain, and wherein each cloning reverse primer anneals to a sequence that overlaps the sequence of an inner reverse primer, the set of cloning reverse primers comprising at least one primer for each of the possible J regions of the TCR alpha chain or TCR beta chain, and wherein each cloning forward primer and each cloning reverse primer also comprises a vector region that is homologous to a vector.

5. The method of claim 4, wherein the vector region comprises about 15 bases of homology to the vector.

6. The method of claim 4 or 5, additionally comprising inserting the cloning product into the vector.

7. The method of claim 6, wherein the vector is an expression vector.

8. The method of claim 4 or 5 wherein the third cloning product is further amplified in a homologous amplification reaction to produce a fourth homologous product, the homologous amplification reaction comprising incubating the third cloning product with a set of homologous primers that are homologous to the vector region of the cloning primers.

9. The method of any one of the preceding claims wherein the amplification reaction comprises a PCR reaction.

10. The method of claim 9, wherein the PCR reaction utilizes a touchdown PCR protocol.

11. The method of any one of the preceding claims additionally comprising screening the isolated genomic DNA obtained in step (a) for the presence of T cell receptor constant regions prior to step (b).

## Patentansprüche

1. Verfahren zum Isolieren von DNA, welche variable Regionen einer Alpha- oder Beta-Kette eines T-Zell-Rezeptors (TCR) kodiert, wobei das Verfahren umfasst: (a) Isolieren genomischer DNA aus einer einzelnen T-Zelle, wobei der Isolationsschritt eine Amplifikation des gesamten Genoms umfasst; und (b) Amplifizieren eines Gensegmentes, welches die variable Region der Alpha- oder Beta-Kette des TCR einschließt, durch eine Anreicherungsamplifikationsreaktion zum Erzeugen eines ersten Anreicherungsproduktes, das die V- und J-Region der Alpha- oder Beta-Kette des TCR umfasst, aus der einzelnen Zelle, wobei die Amplifikationsreaktion ein Inkubieren der isolierten genomischen DNA mit einem Satz äußerer Vorwärtsprimer und einem Satz äußerer Rückwärtsprimer umfasst, wobei sich jeder Vorwärtsprimer durch Annealing an eine Sequenz 5 bis 40 Basenpaare stromaufwärts der Signalsequenz-Übergangsstelle der V-Region anlagert, der Satz äußerer Vorwärtsprimer mindestens einen Primer für jede der möglichen V-Regionen der TCR-Alpha-Kette oder TCR-Beta-Kette umfasst, und wobei sich jeder Rückwärtsprimer durch Annealing an eine Sequenz 5 bis 40 Basenpaare stromabwärts der Exon-/Intron-Übergangsstelle der J-Region anlagert, der Satz äußerer Rückwärtsprimer mindestens einen Primer für jede der möglichen J-Regionen der TCR-Alpha-Kette oder TCR-Beta-Kette umfasst.

2. Verfahren nach Anspruch 1, wobei das erste Anreicherungsprodukt in einer Isolationsamplifikationsreaktion weiter amplifiziert wird, um ein zweites Isolationsprodukt zu erzeugen, wobei die Isolationsamplifikationsreaktion ein Inkubieren des ersten Anreicherungsproduktes mit einem Satz innerer Vorwärtsprimer und einem Satz innerer Rückwärtsprimer umfasst, wobei sich jeder innere Vorwärtsprimer durch Annealing an eine Sequenz am Anfang der ersten Aminosäure stromabwärts der Signalsequenz der V-Region anlagert, der Satz innerer Vorwärtsprimer mindestens einen Primer für jede der möglichen V-Regionen der TCR-Alpha-Kette oder TCR-Beta-Kette umfasst, und wobei sich jeder innere Rückwärtsprimer durch Annealing an eine Sequenz an dem stromabwärts gelegenen Ende der J-Region anlagert, der Satz innerer Rückwärtsprimer mindestens einen Primer für jede der möglichen J-Regionen der TCR-Alpha-Kette oder TCR-Beta-Kette umfasst.

3. Verfahren nach Anspruch 2, zusätzlich die Sequenzierung des Isolationsproduktes umfassend.

4. Verfahren nach Anspruch 2, wobei das zweite Isolationsprodukt in einer Klonierungsamplifikationsreaktion weiter amplifiziert wird, um ein drittes Klonierungsprodukt zu erzeugen, wobei die Klonierungsamplifikationsreaktion ein Inkubieren des zweiten Isolationsproduktes mit einem Satz Klonierungsvorwärtsprimern und einem Satz Klonierungsrückwärtsprimern umfasst, wobei sich jeder Klonierungsvorwärtsprimer durch Annealing an eine Sequenz, welche die Sequenz eines inneren Vorwärtsprimers überlagert, anlagert, der Satz Klonierungsvorwärtsprimer mindestens einen Primer für jede der möglichen V-Regionen der TCR-Alpha-Kette oder TCR-Beta-Kette umfasst, und wobei sich jeder Klonierungsrückwärtsprimer durch Annealing an eine Sequenz, welche die Sequenz eines inneren Rückwärtsprimers überlagert, anlagert, der Satz Klonierungsrückwärtsprimer mindestens einen Primer für jede der möglichen J-Regionen der TCR-Alpha-Kette oder TCR-Beta-Kette umfasst, und wobei jeder Klonierungsvorwärtsprimer und jeder Klonierungsrückwärtsprimer zudem eine Vektorregion, die gegenüber einem Vektor homolog ist, umfasst.

5. Verfahren nach Anspruch 4, wobei die Vektorregion etwa 15 Homologiebasen gegenüber dem Vektor umfasst.

6. Verfahren nach Anspruch 4 oder 5, zusätzlich ein Einfügen des Klonierungsproduktes in den Vektor umfassend.

7. Verfahren nach Anspruch 6, wobei der Vektor ein Expressionsvektor ist.

8. Verfahren nach Anspruch 4 oder 5, wobei das dritte Klonierungsprodukt in einer homologen Amplifikationsreaktion weiter amplifiziert wird, um ein viertes homologes Produkt zu erzeugen, wobei die homologe Amplifikationsreaktion ein Inkubieren des dritten Klonierungsproduktes mit einem Satz homologer Primer, welche gegenüber der Vektorregion des Klonierungsprimers homolog sind, umfasst.

9. Verfahren nach einem der vorausgehenden Ansprüche, wobei die Amplifikationsreaktion eine PCR-Reaktion umfasst.

10. Verfahren nach Anspruch 9, wobei die PCR-Reaktion ein Touchdown-PCR-Protokoll verwendet.

11. Verfahren nach einem der vorausgehenden Ansprüche, zusätzlich ein Screenen der in Schritt (a) erhaltenen isolierten genomischen DNA auf das Vorhandensein konstanter T-Zell-Rezeptor-Regionen vor Schritt (b) umfassend.

## Revendications

1. Procédé permettant l'ADN encodant les zones variables d'une chaîne alpha ou bêta de récepteur de cellule T (RCT), ledit procédé comprenant: (a) l'isolation de l'ADN génomique provenant d'une seule cellule T, où ladite étape d'isolation comprend l'amplification du génome complet ; et (b) l'amplification d'un segment génique englobant la zone variable de chaîne alpha ou bêta RCT par une réaction d'amplification d'enrichissement pour obtenir un premier produit enrichissement comprenant les zones V et J de la chaîne alpha ou bêta de la simple cellule, la réaction d'amplification comprenant l'incubation de l'ADN génomique isolé avec un ensemble d'amorces vers l'avant externes et un ensemble d'amorces vers l'arrière externes, où chaque amorce vers l'avant externe est trempée pour produire une séquence de 5 à 40 paires de bases en amont de la jonction de signal de séquence de la zone V, l'ensemble d'amorces vers l'avant externes comprenant au moins une amorce pour chacune des zones V possibles de la chaîne alpha RCT ou de la chaîne bêta RCT, et où chaque amorce vers l'arrière externe est trempée pour produire une séquence de 5 à 40 paires de bases en aval de la jonction exon/intron de la zone J, l'ensemble d'amorces vers l'arrière externes comprenant au moins une amorce pour chacune des zones J possibles de la chaîne alpha RCT ou de la chaîne bêta RCT.

2. Procédé selon la revendication 1, où le premier produit d'enrichissement est encore amplifié dans une réaction d'amplification d'isolation pour obtenir un deuxième produit d'isolation, la réaction d'amplification d'isolation comprenant l'incubation du premier produit d'enrichissement avec un ensemble d'amorces vers l'avant internes et un ensemble d'amorces vers l'arrière internes, où chaque amorce vers l'avant interne est trempée pour produire une séquence au début du premier acide aminé de la séquence de signal de la zone V, l'ensemble d'amorces vers l'avant internes comprenant au moins une amorce pour chacune des zones V possibles de la chaîne alpha RCT ou de la chaîne bêta RCT, et où chaque amorce vers l'arrière interne est trempée pour produire une séquence à l'extrémité en aval de la zone J, l'ensemble d'amorces vers l'arrière internes comprenant au moins une amorce pour chacune des zones J possibles de la chaîne alpha RCT ou de la chaîne bêta RCT.

3. Procédé selon la revendication 2, comprenant en outre le séquençage du produit d'isolation.

4. Procédé selon la revendication 2, où le deuxième produit d'isolation est encore amplifié dans une réaction d'amplification de clonage pour obtenir un troisième produit de clonage, la réaction d'amplification de clonage comprenant l'incubation du deuxième produit d'isolation avec un ensemble d'amorces vers l'avant de clonage et un ensemble d'amorces vers l'arrière de clonage, où chaque amorce vers l'avant de clonage est trempée pour obtenir une séquence chevauchant la séquence d'une amorce vers l'avant interne, l'ensemble d'amorces vers l'avant de clonage comprenant au moins une amorce pour chacune des zones V possibles de la chaîne alpha RCT ou de la chaîne bêta RCT, et où chaque amorce vers l'arrière de clonage est trempée pour obtenir une séquence chevauchant la séquence d'une amorce vers l'arrière interne, l'ensemble d'amorces vers l'arrière de clonage comprenant au moins une amorce pour chacune des zones J possibles de la chaîne alpha RCT ou de la chaîne bêta RCT, et où chaque amorce vers l'avant de clonage et chaque amorce vers l'arrière de clonage comprend également un zone vecteur homologue à un vecteur.

5. Procédé selon la revendication 4, où la zone vecteur comprend environ 15 bases d'homologie au vecteur.

6. Procédé selon les revendications 4 ou 5, comprenant en outre l'insertion du produit de clonage dans le vecteur.

7. Procédé selon la revendication 6, où le vecteur est un vecteur d'expression.

8. Procédé selon les revendications 4 ou 5, où le troisième produit de clonage est encore amplifié dans une réaction d'amplification homologue pour obtenir un quatrième produit homologue, la réaction d'amplification homologue comprenant l'incubation du troisième produit de clonage avec un ensemble d'amorces homologues qui sont des homologues de la zone vecteur des amorces de clonage.

9. Procédé selon l'une quelconque des revendications précédentes, où la réaction d'amplification comprend une réaction RCP.

10. Procédé selon la revendication 9, où la réaction RCP utilise un protocole RCP « Touchdown ».

11. Procédé selon l'une quelconque des revendications précédentes en outre comprenant le dépistage de l'ADN génomique isolé obtenu à l'étape (a) pour rechercher la présence de zones constantes de récepteur de cellule T avant de passer à l'étape (b).


### SEQUENCE LISTING

<110> Balazs, Alejandro Benjamin Tsai, Jonathan Michael Baltimore, David
<120> ISOLATION OF UNKNOWN REARRANGED T-CELL
   RECEPTORS FROM SINGLE CELLS
<130> CALTE.062VPC
<150> 61/221,505
   <151> 2009-06-29
<160> 511
<170> FastSEQ for Windows Version 4.0
<210> 1
   <211> 25
   <212> DNA
   <213> Artificial Sequence
<220>
   <223> primer
<400> 1
   cagaaccctg accctgccgt gtacc 25
<210> 2
   <211> 26
   <212> DNA
   <213> Artificial Sequence
<220>
   <223> primer
<400> 2
   gccattcctg aagcaaggaa acagcc 26
<210> 3
   <211> 22
   <212> DNA
   <213> Artificial Sequence
<220>
   <223> primer
<400> 3
   ggccacactg gtgtgcctgg cc 22
<210> 4
   <211> 22
   <212> DNA
   <213> Artificial Sequence
<220>
   <223> primer
<400> 4
   cggcgctgac gatctgggtg ac 22
<210> 5
   <211> 25
   <212> DNA
   <213> Artificial Sequence
<220>
   <223> primer
<400> 5
   ccaatggctc aggaactggg aatgc 25
<210> 6
   <211> 38
   <212> DNA
   <213> Artificial Sequence
<220>
   <223> primer
<400> 6
   ggaaaaaatg caaaacaggt agtcttaaat aagcattc 38
<210> 7
   <211> 19
   <212> DNA
   <213> Artificial Sequence
<220>
   <223> primer
<400> 7
   gaccccccca atcccgccc 19
<210> 8
   <211> 29
   <212> DNA
   <213> Artificial Sequence
<220>
   <223> primer
<400> 8
   cagacacagc aaaagagcct agaacctgg 29
<210> 9
   <211> 34
   <212> DNA
   <213> Artificial Sequence
<220>
   <223> primer
<400> 9
   gataatatag ctctcttggc tggagattgc aggt 34
<210> 10
   <211> 35
   <212> DNA
   <213> Artificial Sequence
<220>
   <223> primer
<400> 10
   taacacctat caaactaaac agaatggctt tttgg 35
<210> 11
   <211> 39
   <212> DNA
   <213> Artificial Sequence
<220>
   <223> primer
<400> 11
   aagaaacaaa caataaaagc tttgtttggc tacataatt 39
<210> 12
   <211> 29
   <212> DNA
   <213> Artificial Sequence
<220>
   <223> primer
<400> 12
   aagacctggg ttccagccactttcctact 29
<210> 13
   <211> 34
   <212> DNA
   <213> Artificial Sequence
<220>
   <223> primer
<400> 13
   ctcctagctc ctgaggctca ggacccctgg cttc 34
<210> 14
   <211> 32
   <212> DNA
   <213> Artificial Sequence
<220>
   <223> primer
<400> 14
   acacctcttg gtcttggtct cttcagacac tt 32
<210> 15
   <211> 22
   <212> DNA
   <213> Artificial Sequence
<220>
   <223> primer
<400> 15
   tgtccgctct gctcagggcc ct 22
<210> 16
   <211> 32
   <212> DNA
   <213> Artificial Sequence
<220>
   <223> primer
<400> 16
   ttttcctcac actaagaaga caagacccaa gg 32
<210> 17
   <211> 44
   <212> DNA
   <213> Artificial Sequence
<220>
   <223> primer
<400> 17
   tagtgttaaa aaaaaagaga agatgttgaa tacacaagtc aact 44
<210> 18
   <211> 38
   <212> DNA
   <213> Artificial Sequence
<220>
   <223> primer
<400> 18
   atttcttttt ggattgaaaa ttttaatcct cagtgaac 38
<210> 19
   <211> 41
   <212> DNA
   <213> Artificial Sequence
<220>
   <223> primer
<400> 19
   aaatatccat tctaggtgca ttttttaagg gtttaaaatt t 41
<210> 20
   <211> 40
   <212> DNA
   <213> Artificial Sequence
<220>
   <223> primer
<400> 20
   acaacctgat gatagaagta actcttataa ctggaggttg 40
<210> 21
   <211> 32
   <212> DNA
   <213> Artificial Sequence
<220>
   <223> primer
<400> 21
   cgatgatgga agtagctctt atggctggag at 32
<210> 22
   <211> 38
   <212> DNA
   <213> Artificial Sequence
<220>
   <223> primer
<400> 22
   cacctcacag tacagagtcc tgaaaataaa gaagaaga 38
<210> 23
   <211> 37
   <212> DNA
   <213> Artificial Sequence
<220>
   <223> primer
<400> 23
   ttcttcatgt taaggatcaa gaccattatt tgggtaa 37
<210> 24
   <211> 40
   <212> DNA
   <213> Artificial Sequence
<220>
   <223> primer
<400> 24
   aaatgagaaa cgtttgttat tatttttttt tcgtgtttaa 40
<210> 25
   <211> 31
   <212> DNA
   <213> Artificial Sequence
<220>
   <223> primer
<400> 25
   tgaggctcag cgcccttggc ttctgtccgc c 31
<210> 26
   <211> 31
   <212> DNA
   <213> Artificial Sequence
<220>
   <223> primer
<400> 26
   cagagtgtct atgtggctga atcgtttcca g 31
<210> 27
   <211> 27
   <212> DNA
   <213> Artificial Sequence
<220>
   <223> primer
<400> 27
   tcatctgtga ctgaggagcc ttgctcc 27
<210> 28
   <211> 32
   <212> DNA
   <213> Artificial Sequence
<220>
   <223> primer
<400> 28
   acctttcggt ttggatatct ctcaacaaaa cc 32
<210> 29
   <211> 27
   <212> DNA
   <213> Artificial Sequence
<220>
   <223> primer
<400> 29
   agacggagca cggaacattt cactcag 27
<210> 30
   <211> 37
   <212> DNA
   <213> Artificial Sequence
<220>
   <223> primer
<400> 30
   aagaaggttg gaattatcgt aatttgtttc taggctg 37
<210> 31
   <211> 40
   <212> DNA
   <213> Artificial Sequence
<220>
   <223> primer
<400> 31
   cttgtgagcc attctccata tttcagatat aagatttcag 40
<210> 32
   <211> 39
   <212> DNA
   <213> Artificial Sequence
<220>
   <223> primer
<400> 32
   ccaaggttta gttaaatata tcttatggtg aaaatgccc 39
<210> 33
   <211> 26
   <212> DNA
   <213> Artificial Sequence
<220>
   <223> primer
<400> 33
   gttgggaaga ctggaagacc acctgg 26
<210> 34
   <211> 32
   <212> DNA
   <213> Artificial Sequence
<220>
   <223> primer
<400> 34
   ttccacagat tttggctgaa aaacgttttt ct 32
<210> 35
   <211> 30
   <212> DNA
   <213> Artificial Sequence
<220>
   <223> primer
<400> 35
   gtaccaggca acccatttag gagaagttgg 30
<210> 36
   <211> 34
   <212> DNA
   <213> Artificial Sequence
<220>
   <223> primer
<400> 36
   aacctagaat cagacacaaa aactgaactc tggg 34
<210> 37
   <211> 34
   <212> DNA
   <213> Artificial Sequence
<220>
   <223> primer
<400> 37
   cccactcagg agatcttcta gaatagagct ctca 34
<210> 38
   <211> 27
   <212> DNA
   <213> Artificial Sequence
<220>
   <223> primer
<400> 38
   aggagcagct aaagtcaggg gccatgt 27
<210> 39
   <211> 30
   <212> DNA
   <213> Artificial Sequence
<220>
   <223> primer
<400> 39
   tgcagctttc taggcaggag acaagacaat 30
<210> 40
   <211> 30
   <212> DNA
   <213> Artificial Sequence
<220>
   <223> primer
<400> 40
   gttaaggaag cccattcaga agctgactgg 30
<210> 41
   <211> 27
   <212> DNA
   <213> Artificial Sequence
<220>
   <223> primer
<400> 41
   gacacagagt ctgagttctg gggcctg 27
<210> 42
   <211> 29
   <212> DNA
   <213> Artificial Sequence
<220>
   <223> primer
<400> 42
   gcaaagtaac ttctgctggg gaagctcat 29
<210> 43
   <211> 34
   <212> DNA
   <213> Artificial Sequence
<220>
   <223> primer
<400> 43
   gtgtcactct aagcccaaga gagtttcttg aagc 34
<210> 44
   <211> 30
   <212> DNA
   <213> Artificial Sequence
<220>
   <223> primer
<400> 44
   ttaaaggtag tgaatcacgt tttgcccagg 30
<210> 45
   <211> 30
   <212> DNA
   <213> Artificial Sequence
<220>
   <223> primer
<400> 45
   aacccatcag agcaggagac ttttcactct 30
<210> 46
   <211> 35
   <212> DNA
   <213> Artificial Sequence
<220>
   <223> primer
<400> 46
   atactcaagg ttcagatcag aagaggaggc ttctc 35
<210> 47
   <211> 35
   <212> DNA
   <213> Artificial Sequence
<220>
   <223> primer
<400> 47
   caactttcaa ggctcctaaa tctgagtttt cagtg 35
<210> 48
   <211> 31
   <212> DNA
   <213> Artificial Sequence
<220>
   <223> primer
<400> 48
   aactgtgaat cctcacttca acagtgatgc c 31
<210> 49
   <211> 33
   <212> DNA
   <213> Artificial Sequence
<220>
   <223> primer
<400> 49
   atatattccg aaatcctcca acagagacct gtg 33
<210> 50
   <211> 25
   <212> DNA
   <213> Artificial Sequence
<220>
   <223> primer
<400> 50
   ggtcccaccg aggctttagt gagca 25
<210> 51
   <211> 30
   <212> DNA
   <213> Artificial Sequence
<220>
   <223> primer
<400> 51
   agagaaagga ttagtgacac tggcccatgg 30
<210> 52
   <211> 33
   <212> DNA
   <213> Artificial Sequence
<220>
   <223> primer
<400> 52
   gcattttgga caaagaagaa atagttgtcc gtc 33
<210> 53
   <211> 36
   <212> DNA
   <213> Artificial Sequence
<220>
   <223> primer
<400> 53
   ctgttttctc atagacaagt ggtcagttct ttttgc 36
<210> 54
   <211> 34
   <212> DNA
   <213> Artificial Sequence
<220>
   <223> primer
<400> 54
   ttcatcatct aagaaagcag agtagggcct ttct 34
<210> 55
   <211> 43
   <212> DNA
   <213> Artificial Sequence
<220>
   <223> primer
<400> 55
   tctcactact acagtatcct tccataatat aatctgtctg caa 43
<210> 56
   <211> 24
   <212> DNA
   <213> Artificial Sequence
<220>
   <223> primer
<400> 56
   tctccagcac agggtagcga tggg 24
<210> 57
   <211> 41
   <212> DNA
   <213> Artificial Sequence
<220>
   <223> primer
<400> 57
   accagaataa ttatatccat atatgcccaa tattgaggat a 41
<210> 58
   <211> 37
   <212> DNA
   <213> Artificial Sequence
<220>
   <223> primer
<400> 58
   cccctaaaaa gaaaaaaatc aatgaaaaca gatgttc 37
<210> 59
   <211> 36
   <212> DNA
   <213> Artificial Sequence
<220>
   <223> primer
<400> 59
   caccttttct tccacttatt gtcaccagaa tacatt 36
<210> 60
   <211> 34
   <212> DNA
   <213> Artificial Sequence
<220>
   <223> primer
<400> 60
   caatacatat ggaagcctta aaccagataa gggg 34
<210> 61
   <211> 34
   <212> DNA
   <213> Artificial Sequence
<220>
   <223> primer
<400> 61
   tttcctgaga catgaagaca ttttaccctc aatc 34
<210> 62
   <211> 33
   <212> DNA
   <213> Artificial Sequence
<220>
   <223> primer
<400> 62
   caaatgttac ggtctgagag aagacaacac aag 33
<210> 63
   <211> 36
   <212> DNA
   <213> Artificial Sequence
<220>
   <223> primer
<400> 63
   agtaagttta gtgggtctca gtagccacat taagcc 36
<210> 64
   <211> 38
   <212> DNA
   <213> Artificial Sequence
<220>
   <223> primer
<400> 64
   tcacctgtgc aatatatgac tacaggataa gtacaagc 38
<210> 65
   <211> 44
   <212> DNA
   <213> Artificial Sequence
<220>
   <223> primer
<400> 65
   cttagatttc caaaaaagct tattacttgt ctcaaaaact aatc 44
<210> 66
   <211> 29
   <212> DNA
   <213> Artificial Sequence
<220>
   <223> primer
<400> 66
   gcacattgaa ttgcaaattg atgacaagg 29
<210> 67
   <211> 40
   <212> DNA
   <213> Artificial Sequence
<220>
   <223> primer
<400> 67
   gagttaattc atctcccctt ttaatttctc cacagtaata 40
<210> 68
   <211> 25
   <212> DNA
   <213> Artificial Sequence
<220>
   <223> primer
<400> 68
   gaagaaactt tgctcccctg gccct 25
<210> 69
   <211> 31
   <212> DNA
   <213> Artificial Sequence
<220>
   <223> primer
<400> 69
   tgctgaaaaa cctacccacc attttgctta a 31
<210> 70
   <211> 40
   <212> DNA
   <213> Artificial Sequence
<220>
   <223> primer
<400> 70
   aatacagact gaaaagaaga atttagcata atgtgttggt 40
<210> 71
   <211> 47
   <212> DNA
   <213> Artificial Sequence
<220>
   <223> primer
<400> 71
   cccattaagt tacatgtaca gaatacattt gtagattagt aaatcag 47
<210> 72
   <211> 32
   <212> DNA
   <213> Artificial Sequence
<220>
   <223> primer
<400> 72
   ggtctaaatc agcccttaat ccacagacat tg 32
<210> 73
   <211> 27
   <212> DNA
   <213> Artificial Sequence
<220>
   <223> primer
<400> 73
   gcatgcaggg catgccaaat actaagg 27
<210> 74
   <211> 35
   <212> DNA
   <213> Artificial Sequence
<220>
   <223> primer
<400> 74
   aaagagggca agttttcctc ttggagataa tcata 35
<210> 75
   <211> 28
   <212> DNA
   <213> Artificial Sequence
<220>
   <223> primer
<400> 75
   agcttctccc cacatcaagc actggact 28
<210> 76
   <211> 31
   <212> DNA
   <213> Artificial Sequence
<220>
   <223> primer
<400> 76
   ttcaaactaa tgatttgatt gattgcccct g 31
<210> 77
   <211> 29
   <212> DNA
   <213> Artificial Sequence
<220>
   <223> primer
<400> 77
   agcttctgca tgatggaaga caggcttct 29
<210> 78
   <211> 31
   <212> DNA
   <213> Artificial Sequence
<220>
   <223> primer
<400> 78
   aaataattca agggaagaag ccattgctga g 31
<210> 79
   <211> 32
   <212> DNA
   <213> Artificial Sequence
<220>
   <223> primer
<400> 79
   cactctcagc agtttgaact cagtgggagt ta 32
<210> 80
   <211> 35
   <212> DNA
   <213> Artificial Sequence
<220>
   <223> primer
<400> 80
   aaatctccac taacttcacg ggatttattt gtttg 35
<210> 81
   <211> 27
   <212> DNA
   <213> Artificial Sequence
<220>
   <223> primer
<400> 81
   cgcttcctac ttgccatgga cacagaa 27
<210> 82
   <211> 36
   <212> DNA
   <213> Artificial Sequence
<220>
   <223> primer
<400> 82
   tgctacactt tgtgcattat tcaactagtg tctcct 36
<210> 83
   <211> 42
   <212> DNA
   <213> Artificial Sequence
<220>
   <223> primer
<400> 83
   ttcatttaaa aaaaaaagaa aaagaaaaag aaaacacctt tt 42
<210> 84
   <211> 41
   <212> DNA
   <213> Artificial Sequence
<220>
   <223> primer
<400> 84
   cataagaaga actgttctat atgatttacg gacataacag c 41
<210> 85
   <211> 36
   <212> DNA
   <213> Artificial Sequence
<220>
   <223> primer
<400> 85
   gtgtctggga tgtgagaact tgtcattaca gactaa 36
<210> 86
   <211> 40
   <212> DNA
   <213> Artificial Sequence
<220>
   <223> primer
<400> 86
   gacagagaag attaaacaaa aaatgaacag agtggataac 40
<210> 87
   <211> 33
   <212> DNA
   <213> Artificial Sequence
<220>
   <223> primer
<400> 87
   ggcagtttct gagatatttc aaactgcaca gac 33
<210> 88
   <211> 32
   <212> DNA
   <213> Artificial Sequence
<220>
   <223> primer
<400> 88
   tcagtgctac ggcttccttt tgaaattaga gc 32
<210> 89
   <211> 32
   <212> DNA
   <213> Artificial Sequence
<220>
   <223> primer
<400> 89
   gtccctcaaa catgaacacc aacaaccttt aa 32
<210> 90
   <211> 29
   <212> DNA
   <213> Artificial Sequence
<220>
   <223> primer
<400> 90
   caacaggtcc cattggattt ctttccaga 29
<210> 91
   <211> 34
   <212> DNA
   <213> Artificial Sequence
<220>
   <223> primer
<400> 91
   attctgttgc ccagagtgac aaagtactga tgat 34
<210> 92
   <211> 26
   <212> DNA
   <213> Artificial Sequence
<220>
   <223> primer
<400> 92
   cagcaccatt gctcactcag gtcagc 26
<210> 93
   <211> 32
   <212> DNA
   <213> Artificial Sequence
<220>
   <223> primer
<400> 93
   tgcagtatcc cctgttttaa aggaacacac ag 32
<210> 94
   <211> 29
   <212> DNA
   <213> Artificial Sequence
<220>
   <223> primer
<400> 94
   ggtttagaaa tgtccccatg aggactgca 29
<210> 95
   <211> 37
   <212> DNA
   <213> Artificial Sequence
<220>
   <223> primer
<400> 95
   ctcaaatgtc aggctagaac aaataatagg aaaaggc 37
<210> 96
   <211> 37
   <212> DNA
   <213> Artificial Sequence
<220>
   <223> primer
<400> 96
   agccagaaaa agtttattta atatgcaatg aaaccca 37
<210> 97
   <211> 34
   <212> DNA
   <213> Artificial Sequence
<220>
   <223> primer
<400> 97
   atgtctacta tgatccccag aatcttatgc aggc 34
<210> 98
   <211> 33
   <212> DNA
   <213> Artificial Sequence
<220>
   <223> primer
<400> 98
   ctctttctgc agtttaaagg gtttgctcaa cac 33
<210> 99
   <211> 30
   <212> DNA
   <213> Artificial Sequence
<220>
   <223> primer
<400> 99
   ctaatgtatg agactgttag ccccagcgca 30
<210> 100
   <211> 28
   <212> DNA
   <213> Artificial Sequence
<220>
   <223> primer
<400> 100
   cggggaaggg agcaaaagta cataagga 28
<210> 101
   <211> 29
   <212> DNA
   <213> Artificial Sequence
<220>
   <223> primer
<400> 101
   ctgacactgg ggtgacattc cagaatttc 29
<210> 102
   <211> 35
   <212> DNA
   <213> Artificial Sequence
<220>
   <223> primer
<400> 102
   ggaggggcaa gtaattaaat cagaagtgtt tgaat 35
<210> 103
   <211> 26
   <212> DNA
   <213> Artificial Sequence
<220>
   <223> primer
<400> 103
   ccacaggacc agatgcctga gctagg 26
<210> 104
   <211> 25
   <212> DNA
   <213> Artificial Sequence
<220>
   <223> primer
<400> 104
   cactgcagac cagaatcctg ccctg 25
<210> 105
   <211> 22
   <212> DNA
   <213> Artificial Sequence
<220>
   <223> primer
<400> 105
   cagcacctcg cccaaaggac cc 22
<210> 106
   <211> 24
   <212> DNA
   <213> Artificial Sequence
<220>
   <223> primer
<400> 106
   ggaggaccaa gccctgagca caga 24
<210> 107
   <211> 28
   <212> DNA
   <213> Artificial Sequence
<220>
   <223> primer
<400> 107
   tatcaccgat gcacagaccc agaagacc 28
<210> 108
   <211> 34
   <212> DNA
   <213> Artificial Sequence
<220>
   <223> primer
<400> 108
   tcctactcac agtgactctg atctggtaaa gctc 34
<210> 109
   <211> 25
   <212> DNA
   <213> Artificial Sequence
<220>
   <223> primer
<400> 109
   tcacccagag gaccccagtc agagg 25
<210> 110
   <211> 26
   <212> DNA
   <213> Artificial Sequence
<220>
   <223> primer
<400> 110
   tcccttttca ccaatgcaca gaccca 26
<210> 111
   <211> 26
   <212> DNA
   <213> Artificial Sequence
<220>
   <223> primer
<400> 111
   acctcaccca gaggacccca gtcaga 26
<210> 112
   <211> 27
   <212> DNA
   <213> Artificial Sequence
<220>
   <223> primer
<400> 112
   ccttttcacc aatgcacaga cccagag 27
<210> 113
   <211> 31
   <212> DNA
   <213> Artificial Sequence
<220>
   <223> primer
<400> 113
   ctcacagtga tcctgatctg gtaaagctcc c 31
<210> 114
   <211> 29
   <212> DNA
   <213> Artificial Sequence
<220>
   <223> primer
<400> 114
   gcctttcatc aacacacaga cccagaaga 29
<210> 115
   <211> 28
   <212> DNA
   <213> Artificial Sequence
<220>
   <223> primer
<400> 115
   tcctgctcac agtgaccctg atctggta 28
<210> 116
   <211> 24
   <212> DNA
   <213> Artificial Sequence
<220>
   <223> primer
<400> 116
   cccaggagga ccaagccctg aatc 24
<210> 117
   <211> 35
   <212> DNA
   <213> Artificial Sequence
<220>
   <223> primer
<400> 117
   ggagcttagg aacttcagaa tgcttactac agaga 35
<210> 118
   <211> 24
   <212> DNA
   <213> Artificial Sequence
<220>
   <223> primer
<400> 118
   cttcagtctg cccacagcag ggct 24
<210> 119
   <211> 28
   <212> DNA
   <213> Artificial Sequence
<220>
   <223> primer
<400> 119
   ctcctctgct cctgttcaca aggaccct 28
<210> 120
   <211> 22
   <212> DNA
   <213> Artificial Sequence
<220>
   <223> primer
<400> 120
   aatttgccca cagcagggct gg 22
<210> 121
   <211> 26
   <212> DNA
   <213> Artificial Sequence
<220>
   <223> primer
<400> 121
   ttttgctcac agtgaccctg attggg 26
<210> 122
   <211> 28
   <212> DNA
   <213> Artificial Sequence
<220>
   <223> primer
<400> 122
   ctgctcccct ttcatcaatg cacagata 28
<210> 123
   <211> 30
   <212> DNA
   <213> Artificial Sequence
<220>
   <223> primer
<400> 123
   gctcctgctc atagtgacac tgacctggta 30
<210> 124
   <211> 24
   <212> DNA
   <213> Artificial Sequence
<220>
   <223> primer
<400> 124
   ccccaggagg accaagccct gaat 24
<210> 125
   <211> 31
   <212> DNA
   <213> Artificial Sequence
<220>
   <223> primer
<400> 125
   cctttcatca atgcacagat acagaagacc c 31
<210> 126
   <211> 30
   <212> DNA
   <213> Artificial Sequence
<220>
   <223> primer
<400> 126
   gctcctgctc acagtgacac tgatctggta 30
<210> 127
   <211> 24
   <212> DNA
   <213> Artificial Sequence
<220>
   <223> primer
<400> 127
   cccaggagga ccaagccctg aatc 24
<210> 128
   <211> 26
   <212> DNA
   <213> Artificial Sequence
<220>
   <223> primer
<400> 128
   cccctttcat caatgcacag acccag 26
<210> 129
   <211> 28
   <212> DNA
   <213> Artificial Sequence
<220>
   <223> primer
<400> 129
   tgctgctgct cacagtgaca ctgatctg 28
<210> 130
   <211> 24
   <212> DNA
   <213> Artificial Sequence
<220>
   <223> primer
<400> 130
   ttccccagga gaaccaagcc ctga 24
<210> 131
   <211> 30
   <212> DNA
   <213> Artificial Sequence
<220>
   <223> primer
<400> 131
   cccttttatc aatgcacaga cccagaagac 30
<210> 132
   <211> 27
   <212> DNA
   <213> Artificial Sequence
<220>
   <223> primer
<400> 132
   tccgctcctg ctcacagtga cactgat 27
<210> 133
   <211> 23
   <212> DNA
   <213> Artificial Sequence
<220>
   <223> primer
<400> 133
   tttcccagga ggaccaagcc ctg 23
<210> 134
   <211> 29
   <212> DNA
   <213> Artificial Sequence
<220>
   <223> primer
<400> 134
   cctttcatca atgcacagac ccagaagac 29
<210> 135
   <211> 33
   <212> DNA
   <213> Artificial Sequence
<220>
   <223> primer
<400> 135
   ttctgttcac agtgacactg atctggtaaa gcc 33
<210> 136
   <211> 26
   <212> DNA
   <213> Artificial Sequence
<220>
   <223> primer
<400> 136
   ccaggaagac caagccctga atcagg 26
<210> 137
   <211> 30
   <212> DNA
   <213> Artificial Sequence
<220>
   <223> primer
<400> 137
   ctgctcacag tgaccctgat ctggtaaagc 30
<210> 138
   <211> 25
   <212> DNA
   <213> Artificial Sequence
<220>
   <223> primer
<400> 138
   caaaagccct gctttctcac cccag 25
<210> 139
   <211> 23
   <212> DNA
   <213> Artificial Sequence
<220>
   <223> primer
<400> 139
   ctatttcccc aggcagggct ggg 23
<210> 140
   <211> 27
   <212> DNA
   <213> Artificial Sequence
<220>
   <223> primer
<400> 140
   ctgctcctgc tcacagtgac cctgatc 27
<210> 141
   <211> 30
   <212> DNA
   <213> Artificial Sequence
<220>
   <223> primer
<400> 141
   ctcacagagg gcctggtcta gaatattcca 30
<210> 142
   <211> 28
   <212> DNA
   <213> Artificial Sequence
<220>
   <223> primer
<400> 142
   ttctttgctc atgttcacag agggcctg 28
<210> 143
   <211> 22
   <212> DNA
   <213> Artificial Sequence
<220>
   <223> primer
<400> 143
   ttcgtgccca caagggcctc at 22
<210> 144
   <211> 34
   <212> DNA
   <213> Artificial Sequence
<220>
   <223> primer
<400> 144
   ctcatacttg taagctcctt catctggaaa tgtg 34
<210> 145
   <211> 30
   <212> DNA
   <213> Artificial Sequence
<220>
   <223> primer
<400> 145
   cagagcctga gacagacaga tgcttcattc 30
<210> 146
   <211> 26
   <212> DNA
   <213> Artificial Sequence
<220>
   <223> primer
<400> 146
   tactgcacat cagaacccat cgctgg 26
<210> 147
   <211> 22
   <212> DNA
   <213> Artificial Sequence
<220>
   <223> primer
<400> 147
   tgcagcaagt gcctttgccc tg 22
<210> 148
   <211> 29
   <212> DNA
   <213> Artificial Sequence
<220>
   <223> primer
<400> 148
   cattctcttc caacaagtgc ttggagctc 29
<210> 149
   <211> 25
   <212> DNA
   <213> Artificial Sequence
<220>
   <223> primer
<400> 149
   gaggcagtgg tcacaactct cccca 25
<210> 150
   <211> 35
   <212> DNA
   <213> Artificial Sequence
<220>
   <223> primer
<400> 150
   tctctctctc ttagagcctg tgtctgtaac ttcag 35
<210> 151
   <211> 27
   <212> DNA
   <213> Artificial Sequence
<220>
   <223> primer
<400> 151
   cagaaagggg atgaaaaagc ctcatcc 27
<210> 152
   <211> 25
   <212> DNA
   <213> Artificial Sequence
<220>
   <223> primer
<400> 152
   atgccctgct tccctcaaca tccag 25
<210> 153
   <211> 24
   <212> DNA
   <213> Artificial Sequence
<220>
   <223> primer
<400> 153
   cccatcctgc ttccccacta ctgg 24
<210> 154
   <211> 31
   <212> DNA
   <213> Artificial Sequence
<220>
   <223> primer
<400> 154
   atcagggact aaattcatca cagcaccaag c 31
<210> 155
   <211> 24
   <212> DNA
   <213> Artificial Sequence
<220>
   <223> primer
<400> 155
   acagaaacca cctggagccc ccag 24
<210> 156
   <211> 24
   <212> DNA
   <213> Artificial Sequence
<220>
   <223> primer
<400> 156
   tggacccact tttccctgtg acgg 24
<210> 157
   <211> 28
   <212> DNA
   <213> Artificial Sequence
<220>
   <223> primer
<400> 157
   catttcccag gacagagtcc tccctcat 28
<210> 158
   <211> 25
   <212> DNA
   <213> Artificial Sequence
<220>
   <223> primer
<400> 158
   ctggattcca gccccttttt gcaag 25
<210> 159
   <211> 28
   <212> DNA
   <213> Artificial Sequence
<220>
   <223> primer
<400> 159
   ggtcctcctg gaactccgac cttatgat 28
<210> 160
   <211> 29
   <212> DNA
   <213> Artificial Sequence
<220>
   <223> primer
<400> 160
   aagcagagaa ctctgccttc aagggacaa 29
<210> 161
   <211> 27
   <212> DNA
   <213> Artificial Sequence
<220>
   <223> primer
<400> 161
   aactgatcat tgcagtcaaa cccaggc 27
<210> 162
   <211> 21
   <212> DNA
   <213> Artificial Sequence
<220>
   <223> primer
<400> 162
   cgtgcaggct gggctgctca c 21
<210> 163
   <211> 19
   <212> DNA
   <213> Artificial Sequence
<220>
   <223> primer
<400> 163
   gcccatcccg ccctctcgg 19
<210> 164
   <211> 24
   <212> DNA
   <213> Artificial Sequence
<220>
   <223> primer
<400> 164
   cagactcagc tcgggtcctt ccca 24
<210> 165
   <211> 18
   <212> DNA
   <213> Artificial Sequence
<220>
   <223> primer
<400> 165
   gggcgccccc tccccagt 18
<210> 166
   <211> 24
   <212> DNA
   <213> Artificial Sequence
<220>
   <223> primer
<400> 166
   gcacaaaaac ccgagcgcag tctc 24
<210> 167
   <211> 22
   <212> DNA
   <213> Artificial Sequence
<220>
   <223> primer
<400> 167
   caaaaaccag acccaagccg cc 22
<210> 168
   <211> 21
   <212> DNA
   <213> Artificial Sequence
<220>
   <223> primer
<400> 168
   cgccgccttc cacctgaatc c 21
<210> 169
   <211> 19
   <212> DNA
   <213> Artificial Sequence
<220>
   <223> primer
<400> 169
   cgactccggg gaccgaggg 19
<210> 170
   <211> 29
   <212> DNA
   <213> Artificial Sequence
<220>
   <223> primer
<400> 170
   ggacaaaaca ttgaccagcc cactgagat 29
<210> 171
   <211> 30
   <212> DNA
   <213> Artificial Sequence
<220>
   <223> primer
<400> 171
   aaggaccaag tgtttcagcc ttccacagtg 30
<210> 172
   <211> 25
   <212> DNA
   <213> Artificial Sequence
<220>
   <223> primer
<400> 172
   cagtcagtgg ctcagccgga agatc 25
<210> 173
   <211> 24
   <212> DNA
   <213> Artificial Sequence
<220>
   <223> primer
<400> 173
   aagaccaccc agcccatctc catg 24
<210> 174
   <211> 31
   <212> DNA
   <213> Artificial Sequence
<220>
   <223> primer
<400> 174
   gaggatgtgg agcagagtct tttcctgagt g 31
<210> 175
   <211> 30
   <212> DNA
   <213> Artificial Sequence
<220>
   <223> primer
<400> 175
   caaaagatag aacagaattc cgaggccctg 30
<210> 176
   <211> 25
   <212> DNA
   <213> Artificial Sequence
<220>
   <223> primer
<400> 176
   gaaaaccagg tggagcacag ccctc 25
<210> 177
   <211> 27
   <212> DNA
   <213> Artificial Sequence
<220>
   <223> primer
<400> 177
   cagtctgtga gccagcataa ccaccac 27
<210> 178
   <211> 24
   <212> DNA
   <213> Artificial Sequence
<220>
   <223> primer
<400> 178
   cagtcggtga cccagcttga cagc 24
<210> 179
   <211> 27
   <212> DNA
   <213> Artificial Sequence
<220>
   <223> primer
<400> 179
   cagtcagtga cccagcctga catccac 27
<210> 180
   <211> 23
   <212> DNA
   <213> Artificial Sequence
<220>
   <223> primer
<400> 180
   cagtcggtga cccagcttgg cag 23
<210> 181
   <211> 29
   <212> DNA
   <213> Artificial Sequence
<220>
   <223> primer
<400> 181
   gattcagtgg tccagacaga aggccaagt 29
<210> 182
   <211> 30
   <212> DNA
   <213> Artificial Sequence
<220>
   <223> primer
<400> 182
   aaaaaccaag tggagcagag tcctcagtcc 30
<210> 183
   <211> 25
   <212> DNA
   <213> Artificial Sequence
<220>
   <223> primer
<400> 183
   caacggaagg aggtggagca ggatc 25
<210> 184
   <211> 29
   <212> DNA
   <213> Artificial Sequence
<220>
   <223> primer
<400> 184
   caacagaagg aggtggagca gaattctgg 29
<210> 185
   <211> 27
   <212> DNA
   <213> Artificial Sequence
<220>
   <223> primer
<400> 185
   gagaatgtgg agcagcatcc ttcaacc 27
<210> 186
   <211> 27
   <212> DNA
   <213> Artificial Sequence
<220>
   <223> primer
<400> 186
   gagagtgtgg ggctgcatct tcctacc 27
<210> 187
   <211> 33
   <212> DNA
   <213> Artificial Sequence
<220>
   <223> primer
<400> 187
   cagaagataa ctcaaaccca accaggaatg ttc 33
<210> 188
   <211> 26
   <212> DNA
   <213> Artificial Sequence
<220>
   <223> primer
<400> 188
   aattcagtga cccagatgga agggcc 26
<210> 189
   <211> 27
   <212> DNA
   <213> Artificial Sequence
<220>
   <223> primer
<400> 189
   caacagaagg aggtggagca ggatcct 27
<210> 190
   <211> 26
   <212> DNA
   <213> Artificial Sequence
<220>
   <223> primer
<400> 190
   cagtctgtga cccagcttga cagcca 26
<210> 191
   <211> 27
   <212> DNA
   <213> Artificial Sequence
<220>
   <223> primer
<400> 191
   cagagagtga ctcagcccga gaagctc 27
<210> 192
   <211> 27
   <212> DNA
   <213> Artificial Sequence
<220>
   <223> primer
<400> 192
   caacagggag aagaggatcc tcaggcc 27
<210> 193
   <211> 26
   <212> DNA
   <213> Artificial Sequence
<220>
   <223> primer
<400> 193
   gactcggtta cccagacaga aggccc 26
<210> 194
   <211> 33
   <212> DNA
   <213> Artificial Sequence
<220>
   <223> primer
<400> 194
   cagaaggtaa ctcaagcgca gactgaaatt tct 33
<210> 195
   <211> 25
   <212> DNA
   <213> Artificial Sequence
<220>
   <223> primer
<400> 195
   gaagaccagg tgacgcagag tcccg 25
<210> 196
   <211> 26
   <212> DNA
   <213> Artificial Sequence
<220>
   <223> primer
<400> 196
   aaacaggagg tgacgcagat tcctgc 26
<210> 197
   <211> 31
   <212> DNA
   <213> Artificial Sequence
<220>
   <223> primer
<400> 197
   atacaagtgg agcagagtcc tccagacctg a 31
<210> 198
   <211> 29
   <212> DNA
   <213> Artificial Sequence
<220>
   <223> primer
<400> 198
   caacagaagg agaaaagtga ccagcagca 29
<210> 199
   <211> 33
   <212> DNA
   <213> Artificial Sequence
<220>
   <223> primer
<400> 199
   atactgaacg tggaacaaag tcctcagtca ctg 33
<210> 200
   <211> 31
   <212> DNA
   <213> Artificial Sequence
<220>
   <223> primer
<400> 200
   caacaggtaa tgcaaattcc tcagtaccag c 31
<210> 201
   <211> 21
   <212> DNA
   <213> Artificial Sequence
<220>
   <223> primer
<400> 201
   aagaccaccc agcccccctc c 21
<210> 202
   <211> 24
   <212> DNA
   <213> Artificial Sequence
<220>
   <223> primer
<400> 202
   cagtcggtga cccagcttga tggc 24
<210> 203
   <211> 25
   <212> DNA
   <213> Artificial Sequence
<220>
   <223> primer
<400> 203
   cagctgctgg agcagagccc tcagt 25
<210> 204
   <211> 33
   <212> DNA
   <213> Artificial Sequence
<220>
   <223> primer
<400> 204
   caacagaaga atgatgacca gcaagttaag caa 33
<210> 205
   <211> 27
   <212> DNA
   <213> Artificial Sequence
<220>
   <223> primer
<400> 205
   caacaaccag tgcagagtcc tcaagcc 27
<210> 206
   <211> 33
   <212> DNA
   <213> Artificial Sequence
<220>
   <223> primer
<400> 206
   aagaccacac agccaaattc aatggagagt aac 33
<210> 207
   <211> 30
   <212> DNA
   <213> Artificial Sequence
<220>
   <223> primer
<400> 207
   caagaactgg agcagagtcc tcagtccttg 30
<210> 208
   <211> 36
   <212> DNA
   <213> Artificial Sequence
<220>
   <223> primer
<400> 208
   caacagctga atcagagtcc tcaatctatg tttatc 36
<210> 209
   <211> 33
   <212> DNA
   <213> Artificial Sequence
<220>
   <223> primer
<400> 209
   gaagacaagg tggtacaaag ccctctatct ctg 33
<210> 210
   <211> 33
   <212> DNA
   <213> Artificial Sequence
<220>
   <223> primer
<400> 210
   cagacagtca ctcagtctca accagagatg tct 33
<210> 211
   <211> 30
   <212> DNA
   <213> Artificial Sequence
<220>
   <223> primer
<400> 211
   gagctgaaag tggaacaaaa ccctctgttc 30
<210> 212
   <211> 29
   <212> DNA
   <213> Artificial Sequence
<220>
   <223> primer
<400> 212
   aattcagtca agcagacggg ccaaataac 29
<210> 213
   <211> 28
   <212> DNA
   <213> Artificial Sequence
<220>
   <223> primer
<400> 213
   gccaaaaatg aagtggagca gagtcctc 28
<210> 214
   <211> 27
   <212> DNA
   <213> Artificial Sequence
<220>
   <223> primer
<400> 214
   atggggagaa gtggaaactc tggttcc 27
<210> 215
   <211> 34
   <212> DNA
   <213> Artificial Sequence
<220>
   <223> primer
<400> 215
   atcagatata atgaatacat gggtcccttt ccca 34
<210> 216
   <211> 25
   <212> DNA
   <213> Artificial Sequence
<220>
   <223> primer
<400> 216
   atttggccgg atgctgagtc tggtc 25
<210> 217
   <211> 27
   <212> DNA
   <213> Artificial Sequence
<220>
   <223> primer
<400> 217
   atatgggtgt acagccagcc tggtccc 27
<210> 218
   <211> 31
   <212> DNA
   <213> Artificial Sequence
<220>
   <223> primer
<400> 218
   atttggttgc acttggagtc ttgttccact c 31
<210> 219
   <211> 32
   <212> DNA
   <213> Artificial Sequence
<220>
   <223> primer
<400> 219
   atacggatga acaataaggc tggttcctct tc 32
<210> 220
   <211> 30
   <212> DNA
   <213> Artificial Sequence
<220>
   <223> primer
<400> 220
   atttggtatg accaccactt ggttcccctt 30
<210> 221
   <211> 27
   <212> DNA
   <213> Artificial Sequence
<220>
   <223> primer
<400> 221
   atttggactg accagaagtc gggtgcc 27
<210> 222
   <211> 37
   <212> DNA
   <213> Artificial Sequence
<220>
   <223> primer
<400> 222
   atttgcttta acaaatagtc ttgttcctgc tccaaag 37
<210> 223
   <211> 32
   <212> DNA
   <213> Artificial Sequence
<220>
   <223> primer
<400> 223
   attgagttcc acttttagct gagtgcctgt cc 32
<210> 224
   <211> 33
   <212> DNA
   <213> Artificial Sequence
<220>
   <223> primer
<400> 224
   atctggagag actagaagca tagtcccctt ccc 33
<210> 225
   <211> 26
   <212> DNA
   <213> Artificial Sequence
<220>
   <223> primer
<400> 225
   atcaggcctg accagcagtc tggtcc 26
<210> 226
   <211> 30
   <212> DNA
   <213> Artificial Sequence
<220>
   <223> primer
<400> 226
   atttgggatg acttggagct ttgttccaat 30
<210> 227
   <211> 34
   <212> DNA
   <213> Artificial Sequence
<220>
   <223> primer
<400> 227
   atcaggtttt actgataatc ttgtcccact ccca 34
<210> 228
   <211> 32
   <212> DNA
   <213> Artificial Sequence
<220>
   <223> primer
<400> 228
   attggaactc actgataagg tgggttccct tc 32
<210> 229
   <211> 32
   <212> DNA
   <213> Artificial Sequence
<220>
   <223> primer
<400> 229
   attaagatcc acctttaaca tggttcccct tg 32
<210> 230
   <211> 32
   <212> DNA
   <213> Artificial Sequence
<220>
   <223> primer
<400> 230
   atttggttta actagcaccc tggttcctcc tc 32
<210> 231
   <211> 32
   <212> DNA
   <213> Artificial Sequence
<220>
   <223> primer
<400> 231
   atcaggccag acagtcaact gagttcctct tc 32
<210> 232
   <211> 33
   <212> DNA
   <213> Artificial Sequence
<220>
   <223> primer
<400> 232
   atttggagtg acattatgtt tggatccctt tcc 33
<210> 233
   <211> 32
   <212> DNA
   <213> Artificial Sequence
<220>
   <223> primer
<400> 233
   atttgctctt acagttactg tggttccggc tc 32
<210> 234
   <211> 33
   <212> DNA
   <213> Artificial Sequence
<220>
   <223> primer
<400> 234
   atttggtttt acattgagtt tggtcccaga tcc 33
<210> 235
   <211> 33
   <212> DNA
   <213> Artificial Sequence
<220>
   <223> primer
<400> 235
   atcaggtaaa acagtcaatt gtgtcccaga tcc 33
<210> 236
   <211> 31
   <212> DNA
   <213> Artificial Sequence
<220>
   <223> primer
<400> 236
   attgggtttc acagataact ccgttccctg t 31
<210> 237
   <211> 25
   <212> DNA
   <213> Artificial Sequence
<220>
   <223> primer
<400> 237
   atctggggtg accacaacct gggtc 25
<210> 238
   <211> 25
   <212> DNA
   <213> Artificial Sequence
<220>
   <223> primer
<400> 238
   atctggggtg accacaacct gggtc 25
<210> 239
   <211> 26
   <212> DNA
   <213> Artificial Sequence
<220>
   <223> primer
<400> 239
   atagggcagc acggacaatc tggttc 26
<210> 240
   <211> 30
   <212> DNA
   <213> Artificial Sequence
<220>
   <223> primer
<400> 240
   atttggcttc acagtgagcg tagtcccatc 30
<210> 241
   <211> 30
   <212> DNA
   <213> Artificial Sequence
<220>
   <223> primer
<400> 241
   atttggtatg accgagagtt tggtcccctt 30
<210> 242
   <211> 32
   <212> DNA
   <213> Artificial Sequence
<220>
   <223> primer
<400> 242
   atttgcaatc acagaaagtc ttgtgccctt tc 32
<210> 243
   <211> 32
   <212> DNA
   <213> Artificial Sequence
<220>
   <223> primer
<400> 243
   attggggaga atatgaagtc gtgtcccttt tc 32
<210> 244
   <211> 26
   <212> DNA
   <213> Artificial Sequence
<220>
   <223> primer
<400> 244
   attgggcttc accaccagct gagttc 26
<210> 245
   <211> 26
   <212> DNA
   <213> Artificial Sequence
<220>
   <223> primer
<400> 245
   atttggctgg acagcaagca gagtgc 26
<210> 246
   <211> 31
   <212> DNA
   <213> Artificial Sequence
<220>
   <223> primer
<400> 246
   atctggcttt ataattagct tggtcccagc g 31
<210> 247
   <211> 32
   <212> DNA
   <213> Artificial Sequence
<220>
   <223> primer
<400> 247
   atttggaaag acttgtaatc tggtcccagt cc 32
<210> 248
   <211> 31
   <212> DNA
   <213> Artificial Sequence
<220>
   <223> primer
<400> 248
   atgtggtaaa acaatcactt gagtgccgga c 31
<210> 249
   <211> 31
   <212> DNA
   <213> Artificial Sequence
<220>
   <223> primer
<400> 249
   ataggggaat aacggtgagt ctcgttccag t 31
<210> 250
   <211> 33
   <212> DNA
   <213> Artificial Sequence
<220>
   <223> primer
<400> 250
   atctggtttt acttggtaaa gttgtccctt gcc 33
<210> 251
   <211> 28
   <212> DNA
   <213> Artificial Sequence
<220>
   <223> primer
<400> 251
   attcggattt actgccaggc ttgttccc 28
<210> 252
   <211> 29
   <212> DNA
   <213> Artificial Sequence
<220>
   <223> primer
<400> 252
   atggggtttg accattaacc ttgttcccc 29
<210> 253
   <211> 29
   <212> DNA
   <213> Artificial Sequence
<220>
   <223> primer
<400> 253
   atttgctaaa accttcagcc tggtgcctg 29
<210> 254
   <211> 25
   <212> DNA
   <213> Artificial Sequence
<220>
   <223> primer
<400> 254
   atggggtgtg accaacagcg aggtg 25
<210> 255
   <211> 38
   <212> DNA
   <213> Artificial Sequence
<220>
   <223> primer
<400> 255
   atttggttta acagagagtt tagtgccttt tccaaaga 38
<210> 256
   <211> 32
   <212> DNA
   <213> Artificial Sequence
<220>
   <223> primer
<400> 256
   atttggtttt actgtcagtc tggtccctgc tc 32
<210> 257
   <211> 30
   <212> DNA
   <213> Artificial Sequence
<220>
   <223> primer
<400> 257
   atcgagcgtg acctgaagtc ttgttccagt 30
<210> 258
   <211> 31
   <212> DNA
   <213> Artificial Sequence
<220>
   <223> primer
<400> 258
   atagggctgg atgattagat gagtcccttt g 31
<210> 259
   <211> 28
   <212> DNA
   <213> Artificial Sequence
<220>
   <223> primer
<400> 259
   attgggccta actgctaaac gagtcccg 28
<210> 260
   <211> 31
   <212> DNA
   <213> Artificial Sequence
<220>
   <223> primer
<400> 260
   ataggacttg actctcagaa tggttcctgc g 31
<210> 261
   <211> 32
   <212> DNA
   <213> Artificial Sequence
<220>
   <223> primer
<400> 261
   attgggtatg atggtgagtc ttgttccagt cc 32
<210> 262
   <211> 30
   <212> DNA
   <213> Artificial Sequence
<220>
   <223> primer
<400> 262
   atttggaatg accgtcaaac ttgtccctgt 30
<210> 263
   <211> 31
   <212> DNA
   <213> Artificial Sequence
<220>
   <223> primer
<400> 263
   atttggaatg actgataagc ttgtccctgg c 31
<210> 264
   <211> 33
   <212> DNA
   <213> Artificial Sequence
<220>
   <223> primer
<400> 264
   atttggcttc acagttagtc atgtctcctt tcc 33
<210> 265
   <211> 30
   <212> DNA
   <213> Artificial Sequence
<220>
   <223> primer
<400> 265
   atttggatgg acagtcaaga tggtcccttg 30
<210> 266
   <211> 33
   <212> DNA
   <213> Artificial Sequence
<220>
   <223> primer
<400> 266
   atttggattc acggttaaga gagttccttt tcc 33
<210> 267
   <211> 27
   <212> DNA
   <213> Artificial Sequence
<220>
   <223> primer
<400> 267
   gaacctgaag tcacccagac tcccagc 27
<210> 268
   <211> 30
   <212> DNA
   <213> Artificial Sequence
<220>
   <223> primer
<400> 268
   gctgtttccc agactccaaa atacctggtc 30
<210> 269
   <211> 30
   <212> DNA
   <213> Artificial Sequence
<220>
   <223> primer
<400> 269
   gaagttaccc agacaccaaa acacctggtc 30
<210> 270
   <211> 35
   <212> DNA
   <213> Artificial Sequence
<220>
   <223> primer
<400> 270
   ggagtcactc aaactccaag atatctgatc aaaac 35
<210> 271
   <211> 27
   <212> DNA
   <213> Artificial Sequence
<220>
   <223> primer
<400> 271
   ggtgtcactc agaccccaaa attccag 27
<210> 272
   <211> 28
   <212> DNA
   <213> Artificial Sequence
<220>
   <223> primer
<400> 272
   ggagtctccc agtccctgag acacaagg 28
<210> 273
   <211> 28
   <212> DNA
   <213> Artificial Sequence
<220>
   <223> primer
<400> 273
   ggagttacgc agacaccaag acacctgg 28
<210> 274
   <211> 26
   <212> DNA
   <213> Artificial Sequence
<220>
   <223> primer
<400> 274
   ggtgtcactc agaccccaaa attccg 26
<210> 275
   <211> 28
   <212> DNA
   <213> Artificial Sequence
<220>
   <223> primer
<400> 275
   ggagttacgc agacaccaag acacctgg 28
<210> 276
   <211> 26
   <212> DNA
   <213> Artificial Sequence
<220>
   <223> primer
<400> 276
   ggtgtcactc agaccccaaa attccg 26
<210> 277
   <211> 27
   <212> DNA
   <213> Artificial Sequence
<220>
   <223> primer
<400> 277
   ggagtctccc agtcccccag taacaag 27
<210> 278
   <211> 25
   <212> DNA
   <213> Artificial Sequence
<220>
   <223> primer
<400> 278
   gggatcaccc aggcaccaac atctc 25
<210> 279
   <211> 27
   <212> DNA
   <213> Artificial Sequence
<220>
   <223> primer
<400> 279
   ggagtctccc agacccccag taacaag 27
<210> 280
   <211> 26
   <212> DNA
   <213> Artificial Sequence
<220>
   <223> primer
<400> 280
   ggagtcaccc aaagtcccac acacct 26
<210> 281
   <211> 26
   <212> DNA
   <213> Artificial Sequence
<220>
   <223> primer
<400> 281
   ggagtcacac aaaccccaaa gcacct 26
<210> 282
   <211> 28
   <212> DNA
   <213> Artificial Sequence
<220>
   <223> primer
<400> 282
   gaaatcaccc agagcccaag acacaaga 28
<210> 283
   <211> 31
   <212> DNA
   <213> Artificial Sequence
<220>
   <223> primer
<400> 283
   gaagttgccc agtcccccag atataagatt a 31
<210> 284
   <211> 29
   <212> DNA
   <213> Artificial Sequence
<220>
   <223> primer
<400> 284
   ggaatcaccc agagcccaag atacaagat 29
<210> 285
   <211> 36
   <212> DNA
   <213> Artificial Sequence
<220>
   <223> primer
<400> 285
   ggagttgccc agtctcccag atataagatt atagag 36
<210> 286
   <211> 27
   <212> DNA
   <213> Artificial Sequence
<220>
   <223> primer
<400> 286
   ggtgtcactc agaccccaaa attccag 27
<210> 287
   <211> 28
   <212> DNA
   <213> Artificial Sequence
<220>
   <223> primer
<400> 287
   ggagtctccc agtccccaag gtacaaag 28
<210> 288
   <211> 26
   <212> DNA
   <213> Artificial Sequence
<220>
   <223> primer
<400> 288
   ggagtcaccc aaagtcccac acacct 26
<210> 289
   <211> 26
   <212> DNA
   <213> Artificial Sequence
<220>
   <223> primer
<400> 289
   ggtgtcactc agaccccaaa attccg 26
<210> 290
   <211> 26
   <212> DNA
   <213> Artificial Sequence
<220>
   <223> primer
<400> 290
   ggagtctccc agtccccaag gtacga 26
<210> 291
   <211> 26
   <212> DNA
   <213> Artificial Sequence
<220>
   <223> primer
<400> 291
   ggagtcaccc aaagtcccac acacct 26
<210> 292
   <211> 27
   <212> DNA
   <213> Artificial Sequence
<220>
   <223> primer
<400> 292
   ggtgtcactc agaccccaaa attccac 27
<210> 293
   <211> 30
   <212> DNA
   <213> Artificial Sequence
<220>
   <223> primer
<400> 293
   ggagtctccc agtctcccag gtacaaagtc 30
<210> 294
   <211> 26
   <212> DNA
   <213> Artificial Sequence
<220>
   <223> primer
<400> 294
   ggagtcaccc aaagtcccac acacct 26
<210> 295
   <211> 29
   <212> DNA
   <213> Artificial Sequence
<220>
   <223> primer
<400> 295
   ggtgtcactc agaccccaaa attccacat 29
<210> 296
   <211> 30
   <212> DNA
   <213> Artificial Sequence
<220>
   <223> primer
<400> 296
   ggagtctccc agtctcccag gtacaaagtc 30
<210> 297
   <211> 26
   <212> DNA
   <213> Artificial Sequence
<220>
   <223> primer
<400> 297
   ggagtcaccc aaagtcccac acacct 26
<210> 298
   <211> 29
   <212> DNA
   <213> Artificial Sequence
<220>
   <223> primer
<400> 298
   ggtgtcactc agaccccaaa attccacat 29
<210> 299
   <211> 30
   <212> DNA
   <213> Artificial Sequence
<220>
   <223> primer
<400> 299
   ggagtctccc agtcccctag gtacaaagtc 30
<210> 300
   <211> 28
   <212> DNA
   <213> Artificial Sequence
<220>
   <223> primer
<400> 300
   ggagtcacac aaagtcccac acacctga 28
<210> 301
   <211> 27
   <212> DNA
   <213> Artificial Sequence
<220>
   <223> primer
<400> 301
   ggagtctccc agaaccccag acacaag 27
<210> 302
   <211> 29
   <212> DNA
   <213> Artificial Sequence
<220>
   <223> primer
<400> 302
   ggagtcatcc agtccccaag acatctgat 29
<210> 303
   <211> 27
   <212> DNA
   <213> Artificial Sequence
<220>
   <223> primer
<400> 303
   ggaatcaccc agagcccaag acacaag 27
<210> 304
   <211> 38
   <212> DNA
   <213> Artificial Sequence
<220>
   <223> primer
<400> 304
   ggagtggttc agtctcccag atataagatt atagagaa 38
<210> 305
   <211> 25
   <212> DNA
   <213> Artificial Sequence
<220>
   <223> primer
<400> 305
   ggagttatcc agtcaccccg ccatg 25
<210> 306
   <211> 24
   <212> DNA
   <213> Artificial Sequence
<220>
   <223> primer
<400> 306
   ggagttatcc agtcaccccg gcac 24
<210> 307
   <211> 27
   <212> DNA
   <213> Artificial Sequence
<220>
   <223> primer
<400> 307
   agagtcaccc agacaccaag gcacaag 27
<210> 308
   <211> 27
   <212> DNA
   <213> Artificial Sequence
<220>
   <223> primer
<400> 308
   ggagttactc agttccccag ccacagc 27
<210> 309
   <211> 30
   <212> DNA
   <213> Artificial Sequence
<220>
   <223> primer
<400> 309
   atggtcatcc agaacccaag ataccaggtt 30
<210> 310
   <211> 23
   <212> DNA
   <213> Artificial Sequence
<220>
   <223> primer
<400> 310
   gagcctggag tcagccagac ccc 23
<210> 311
   <211> 24
   <212> DNA
   <213> Artificial Sequence
<220>
   <223> primer
<400> 311
   ggcgtcatgc agaacccaag acac 24
<210> 312
   <211> 31
   <212> DNA
   <213> Artificial Sequence
<220>
   <223> primer
<400> 312
   ggaatcactc agtccccaaa gtacctgttc a 31
<210> 313
   <211> 26
   <212> DNA
   <213> Artificial Sequence
<220>
   <223> primer
<400> 313
   gctgtcgtct ctcaacatcc gagctg 26
<210> 314
   <211> 24
   <212> DNA
   <213> Artificial Sequence
<220>
   <223> primer
<400> 314
   attccagctc actggggctg gatg 24
<210> 315
   <211> 31
   <212> DNA
   <213> Artificial Sequence
<220>
   <223> primer
<400> 315
   aaagtcacac agactccagg acatttggtc a 31
<210> 316
   <211> 30
   <212> DNA
   <213> Artificial Sequence
<220>
   <223> primer
<400> 316
   gatgttaccc agaccccaag gaataggatc 30
<210> 317
   <211> 35
   <212> DNA
   <213> Artificial Sequence
<220>
   <223> primer
<400> 317
   gacatctacc agaccccaag ataccttgtt atagg 35
<210> 318
   <211> 35
   <212> DNA
   <213> Artificial Sequence
<220>
   <223> primer
<400> 318
   gtagttacac aattcccaag acacagaatc attgg 35
<210> 319
   <211> 30
   <212> DNA
   <213> Artificial Sequence
<220>
   <223> primer
<400> 319
   caagtgaccc agaacccaag atacctcatc 30
<210> 320
   <211> 35
   <212> DNA
   <213> Artificial Sequence
<220>
   <223> primer
<400> 320
   tcctctacaa ctgtgagtct ggtgccttgt ccaaa 35
<210> 321
   <211> 31
   <212> DNA
   <213> Artificial Sequence
<220>
   <223> primer
<400> 321
   tcctctacaa cggttaacct ggtccccgaa c 31
<210> 322
   <211> 34
   <212> DNA
   <213> Artificial Sequence
<220>
   <223> primer
<400> 322
   tcctctacaa cagtgagcca acttccctct ccaa 34
<210> 323
   <211> 31
   <212> DNA
   <213> Artificial Sequence
<220>
   <223> primer
<400> 323
   tcctccaaga cagagagctg ggttccactg c 31
<210> 324
   <211> 33
   <212> DNA
   <213> Artificial Sequence
<220>
   <223> primer
<400> 324
   tcctctagga tggagagtcg agtcccatca cca 33
<210> 325
   <211> 30
   <212> DNA
   <213> Artificial Sequence
<220>
   <223> primer
<400> 325
   tcctctgtca cagtgagcct ggtcccgttc 30
<210> 326
   <211> 28
   <212> DNA
   <213> Artificial Sequence
<220>
   <223> primer
<400> 326
   tcctctagca cggtgagccg tgtccctg 28
<210> 327
   <211> 32
   <212> DNA
   <213> Artificial Sequence
<220>
   <223> primer
<400> 327
   tcctccagta cggtcagcct agagccttct cc 32
<210> 328
   <211> 27
   <212> DNA
   <213> Artificial Sequence
<220>
   <223> primer
<400> 328
   tcctccagaa ccaggagtcc tccgccc 27
<210> 329
   <211> 25
   <212> DNA
   <213> Artificial Sequence
<220>
   <223> primer
<400> 329
   tcctcgagca ctgtcagccg ggtgc 25
<210> 330
   <211> 26
   <212> DNA
   <213> Artificial Sequence
<220>
   <223> primer
<400> 330
   tcctccagca ctgagagccg ggtccc 26
<210> 331
   <211> 24
   <212> DNA
   <213> Artificial Sequence
<220>
   <223> primer
<400> 331
   tcctcgagca ccaggagccg cgtg 24
<210> 332
   <211> 25
   <212> DNA
   <213> Artificial Sequence
<220>
   <223> primer
<400> 332
   tcctccagca cggtcagcct gctgc 25
<210> 333
   <211> 26
   <212> DNA
   <213> Artificial Sequence
<220>
   <223> primer
<400> 333
   tcctctgtga ccgtgagcct ggtgcc 26
<210> 334
   <211> 46
   <212> DNA
   <213> Artificial Sequence
<220>
   <223> primer
<400> 334
   tacaggaggg ctcggcagga caaaacattg accagcccac tgagat 46
<210> 335
   <211> 47
   <212> DNA
   <213> Artificial Sequence
<220>
   <223> primer
<400> 335
   tacaggaggg ctcggcaaag gaccaagtgt ttcagccttc cacagtg 47
<210> 336
   <211> 42
   <212> DNA
   <213> Artificial Sequence
<220>
   <223> primer
<400> 336
   tacaggaggg ctcggcacag tcagtggctc agccggaaga tc 42
<210> 337
   <211> 41
   <212> DNA
   <213> Artificial Sequence
<220>
   <223> primer
<400> 337
   tacaggaggg ctcggcaaag accacccagc ccatctccat g 41
<210> 338
   <211> 47
   <212> DNA
   <213> Artificial Sequence
<220>
   <223> primer
<400> 338
   tacaggaggg ctcggcaagg atgtggagca gagtcttttc ctgagtg 47
<210> 339
   <211> 47
   <212> DNA
   <213> Artificial Sequence
<220>
   <223> primer
<400> 339
   tacaggaggg ctcggcacaa aagatagaac agaattccga ggccctg 47
<210> 340
   <211> 42
   <212> DNA
   <213> Artificial Sequence
<220>
   <223> primer
<400> 340
   tacaggaggg ctcggcagaa aaccaggtgg agcacagccc tc 42
<210> 341
   <211> 44
   <212> DNA
   <213> Artificial Sequence
<220>
   <223> primer
<400> 341
   tacaggaggg ctcggcacag tctgtgagcc agcataacca ccac 44
<210> 342
   <211> 41
   <212> DNA
   <213> Artificial Sequence
<220>
   <223> primer
<400> 342
   tacaggaggg ctcggcacag tcggtgaccc agcttgacag c 41
<210> 343
   <211> 44
   <212> DNA
   <213> Artificial Sequence
<220>
   <223> primer
<400> 343
   tacaggaggg ctcggcacag tcagtgaccc agcctgacat ccac 44
<210> 344
   <211> 40
   <212> DNA
   <213> Artificial Sequence
<220>
   <223> primer
<400> 344
   tacaggaggg ctcggcacag tcggtgaccc agcttggcag 40
<210> 345
   <211> 46
   <212> DNA
   <213> Artificial Sequence
<220>
   <223> primer
<400> 345
   tacaggaggg ctcggcagat tcagtggtcc agacagaagg ccaagt 46
<210> 346
   <211> 47
   <212> DNA
   <213> Artificial Sequence
<220>
   <223> primer
<400> 346
   tacaggaggg ctcggcaaaa aaccaagtgg agcagagtcc tcagtcc 47
<210> 347
   <211> 42
   <212> DNA
   <213> Artificial Sequence
<220>
   <223> primer
<400> 347
   tacaggaggg ctcggcacaa cggaaggagg tggagcagga tc 42
<210> 348
   <211> 46
   <212> DNA
   <213> Artificial Sequence
<220>
   <223> primer
<400> 348
   tacaggaggg ctcggcacaa cagaaggagg tggagcagaa ttctgg 46
<210> 349
   <211> 44
   <212> DNA
   <213> Artificial Sequence
<220>
   <223> primer
<400> 349
   tacaggaggg ctcggcagag aatgtggagc agcatccttc aacc 44
<210> 350
   <211> 44
   <212> DNA
   <213> Artificial Sequence
<220>
   <223> primer
<400> 350
   tacaggaggg ctcggcagag agtgtggggc tgcatcttcc tacc 44
<210> 351
   <211> 50
   <212> DNA
   <213> Artificial Sequence
<220>
   <223> primer
<400> 351
   tacaggaggg ctcggcacag aagataactc aaacccaacc aggaatgttc 50
<210> 352
   <211> 43
   <212> DNA
   <213> Artificial Sequence
<220>
   <223> primer
<400> 352
   tacaggaggg ctcggcaaat tcagtgaccc agatggaagg gcc 43
<210> 353
   <211> 44
   <212> DNA
   <213> Artificial Sequence
<220>
   <223> primer
<400> 353
   tacaggaggg ctcggcacaa cagaaggagg tggagcagga tcct 44
<210> 354
   <211> 43
   <212> DNA
   <213> Artificial Sequence
<220>
   <223> primer
<400> 354
   tacaggaggg ctcggcacag tctgtgaccc agcttgacag cca 43
<210> 355
   <211> 44
   <212> DNA
   <213> Artificial Sequence
<220>
   <223> primer
<400> 355
   tacaggaggg ctcggcacag agagtgactc agcccgagaa gctc 44
<210> 356
   <211> 44
   <212> DNA
   <213> Artificial Sequence
<220>
   <223> primer
<400> 356
   tacaggaggg ctcggcacaa cagggagaag aggatcctca ggcc 44
<210> 357
   <211> 43
   <212> DNA
   <213> Artificial Sequence
<220>
   <223> primer
<400> 357
   tacaggaggg ctcggcagac tcggttaccc agacagaagg ccc 43
<210> 358
   <211> 50
   <212> DNA
   <213> Artificial Sequence
<220>
   <223> primer
<400> 358
   tacaggaggg ctcggcacag aaggtaactc aagcgcagac tgaaatttct 50
<210> 359
   <211> 42
   <212> DNA
   <213> Artificial Sequence
<220>
   <223> primer
<400> 359
   tacaggaggg ctcggcagaa gaccaggtga cgcagagtcc cg 42
<210> 360
   <211> 43
   <212> DNA
   <213> Artificial Sequence
<220>
   <223> primer
<400> 360
   tacaggaggg ctcggcaaaa caggaggtga cgcagattcc tgc 43
<210> 361
   <211> 48
   <212> DNA
   <213> Artificial Sequence
<220>
   <223> primer
<400> 361
   tacaggaggg ctcggcaata caagtggagc agagtcctcc agacctga 48
<210> 362
   <211> 46
   <212> DNA
   <213> Artificial Sequence
<220>
   <223> primer
<400> 362
   tacaggaggg ctcggcacaa cagaaggaga aaagtgacca gcagca 46
<210> 363
   <211> 49
   <212> DNA
   <213> Artificial Sequence
<220>
   <223> primer
<400> 363
   tacaggaggg ctcggcatac tgaacgtgga acaaagtcct cagtcactg 49
<210> 364
   <211> 48
   <212> DNA
   <213> Artificial Sequence
<220>
   <223> primer
<400> 364
   tacaggaggg ctcggcacaa caggtaatgc aaattcctca gtaccagc 48
<210> 365
   <211> 38
   <212> DNA
   <213> Artificial Sequence
<220>
   <223> primer
<400> 365
   tacaggaggg ctcggcaaag accacccagc ccccctcc 38
<210> 366
   <211> 41
   <212> DNA
   <213> Artificial Sequence
<220>
   <223> primer
<400> 366
   tacaggaggg ctcggcacag tcggtgaccc agcttgatgg c 41
<210> 367
   <211> 42
   <212> DNA
   <213> Artificial Sequence
<220>
   <223> primer
<400> 367
   tacaggaggg ctcggcacag ctgctggagc agagccctca gt 42
<210> 368
   <211> 50
   <212> DNA
   <213> Artificial Sequence
<220>
   <223> primer
<400> 368
   tacaggaggg ctcggcacaa cagaagaatg atgaccagca agttaagcaa 50
<210> 369
   <211> 44
   <212> DNA
   <213> Artificial Sequence
<220>
   <223> primer
<400> 369
   tacaggaggg ctcggcacaa caaccagtgc agagtcctca agcc 44
<210> 370
   <211> 49
   <212> DNA
   <213> Artificial Sequence
<220>
   <223> primer
<400> 370
   tacaggaggg ctcggcaaga ccacacagcc aaattcaatg gagagtaac 49
<210> 371
   <211> 47
   <212> DNA
   <213> Artificial Sequence
<220>
   <223> primer
<400> 371
   tacaggaggg ctcggcacaa gaactggagc agagtcctca gtccttg 47
<210> 372
   <211> 53
   <212> DNA
   <213> Artificial Sequence
<220>
   <223> primer
<400> 372
   tacaggaggg ctcggcacaa cagctgaatc agagtcctca atctatgttt atc 53
<210> 373
   <211> 49
   <212> DNA
   <213> Artificial Sequence
<220>
   <223> primer
<400> 373
   tacaggaggg ctcggcaaag acaaggtggt acaaagccct ctatctctg 49
<210> 374
   <211> 49
   <212> DNA
   <213> Artificial Sequence
<220>
   <223> primer
<400> 374
   tacaggaggg ctcggcaaga cagtcactca gtctcaacca gagatgtct 49
<210> 375
   <211> 47
   <212> DNA
   <213> Artificial Sequence
<220>
   <223> primer
<400> 375
   tacaggaggg ctcggcagag ctgaaagtgg aacaaaaccc tctgttc 47
<210> 376
   <211> 46
   <212> DNA
   <213> Artificial Sequence
<220>
   <223> primer
<400> 376
   tacaggaggg ctcggcaaat tcagtcaagc agacgggcca aataac 46
<210> 377
   <211> 45
   <212> DNA
   <213> Artificial Sequence
<220>
   <223> primer
<400> 377
   tacaggaggg ctcggcagcc aaaaatgaag tggagcagag tcctc 45
<210> 378
   <211> 42
   <212> DNA
   <213> Artificial Sequence
<220>
   <223> primer
<400> 378
   gtcagggttc tggatatggg gagaagtgga aactctggtt cc 42
<210> 379
   <211> 49
   <212> DNA
   <213> Artificial Sequence
<220>
   <223> primer
<400> 379
   gtcagggttc tggatatcag atataatgaa tacatgggtc cctttccca 49
<210> 380
   <211> 40
   <212> DNA
   <213> Artificial Sequence
<220>
   <223> primer
<400> 380
   gtcagggttc tggatatttg gccggatgct gagtctggtc 40
<210> 381
   <211> 42
   <212> DNA
   <213> Artificial Sequence
<220>
   <223> primer
<400> 381
   gtcagggttc tggatatatg ggtgtacagc cagcctggtc cc 42
<210> 382
   <211> 46
   <212> DNA
   <213> Artificial Sequence
<220>
   <223> primer
<400> 382
   gtcagggttc tggatatttg gttgcacttg gagtcttgtt ccactc 46
<210> 383
   <211> 47
   <212> DNA
   <213> Artificial Sequence
<220>
   <223> primer
<400> 383
   gtcagggttc tggatatacg gatgaacaat aaggctggtt cctcttc 47
<210> 384
   <211> 45
   <212> DNA
   <213> Artificial Sequence
<220>
   <223> primer
<400> 384
   gtcagggttc tggatatttg gtatgaccac cacttggttc ccctt 45
<210> 385
   <211> 42
   <212> DNA
   <213> Artificial Sequence
<220>
   <223> primer
<400> 385
   gtcagggttc tggatatttg gactgaccag aagtcgggtg cc 42
<210> 386
   <211> 52
   <212> DNA
   <213> Artificial Sequence
<220>
   <223> primer
<400> 386
   gtcagggttc tggatatttg ctttaacaaa tagtcttgtt cctgctccaa ag 52
<210> 387
   <211> 47
   <212> DNA
   <213> Artificial Sequence
<220>
   <223> primer
<400> 387
   gtcagggttc tggatattga gttccacttt tagctgagtg cctgtcc 47
<210> 388
   <211> 48
   <212> DNA
   <213> Artificial Sequence
<220>
   <223> primer
<400> 388
   gtcagggttc tggatatctg gagagactag aagcatagtc cccttccc 48
<210> 389
   <211> 41
   <212> DNA
   <213> Artificial Sequence
<220>
   <223> primer
<400> 389
   gtcagggttc tggatatcag gcctgaccag cagtctggtc c 41
<210> 390
   <211> 45
   <212> DNA
   <213> Artificial Sequence
<220>
   <223> primer
<400> 390
   gtcagggttc tggatatttg ggatgacttg gagctttgtt ccaat 45
<210> 391
   <211> 49
   <212> DNA
   <213> Artificial Sequence
<220>
   <223> primer
<400> 391
   gtcagggttc tggatatcag gttttactga taatcttgtc ccactccca 49
<210> 392
   <211> 47
   <212> DNA
   <213> Artificial Sequence
<220>
   <223> primer
<400> 392
   gtcagggttc tggatattgg aactcactga taaggtgggt tcccttc 47
<210> 393
   <211> 47
   <212> DNA
   <213> Artificial Sequence
<220>
   <223> primer
<400> 393
   gtcagggttc tggatattaa gatccacctt taacatggtt ccccttg 47
<210> 394
   <211> 47
   <212> DNA
   <213> Artificial Sequence
<220>
   <223> primer
<400> 394
   gtcagggttc tggatatttg gtttaactag caccctggtt cctcctc 47
<210> 395
   <211> 47
   <212> DNA
   <213> Artificial Sequence
<220>
   <223> primer
<400> 395
   gtcagggttc tggatatcag gccagacagt caactgagtt cctcttc 47
<210> 396
   <211> 48
   <212> DNA
   <213> Artificial Sequence
<220>
   <223> primer
<400> 396
   gtcagggttc tggatatttg gagtgacatt atgtttggat ccctttcc 48
<210> 397
   <211> 47
   <212> DNA
   <213> Artificial Sequence
<220>
   <223> primer
<400> 397
   gtcagggttc tggatatttg ctcttacagt tactgtggtt ccggctc 47
<210> 398
   <211> 48
   <212> DNA
   <213> Artificial Sequence
<220>
   <223> primer
<400> 398
   gtcagggttc tggatatttg gttttacatt gagtttggtc ccagatcc 48
<210> 399
   <211> 48
   <212> DNA
   <213> Artificial Sequence
<220>
   <223> primer
<400> 399
   gtcagggttc tggatatcag gtaaaacagt caattgtgtc ccagatcc 48
<210> 400
   <211> 46
   <212> DNA
   <213> Artificial Sequence
<220>
   <223> primer
<400> 400
   gtcagggttc tggatattgg gtttcacaga taactccgtt ccctgt 46
<210> 401
   <211> 40
   <212> DNA
   <213> Artificial Sequence
<220>
   <223> primer
<400> 401
   gtcagggttc tggatatctg gggtgaccac aacctgggtc 40
<210> 402
   <211> 40
   <212> DNA
   <213> Artificial Sequence
<220>
   <223> primer
<400> 402
   gtcagggttc tggatatctg gggtgaccac aacctgggtc 40
<210> 403
   <211> 41
   <212> DNA
   <213> Artificial Sequence
<220>
   <223> primer
<400> 403
   gtcagggttc tggatatagg gcagcacgga caatctggtt c 41
<210> 404
   <211> 45
   <212> DNA
   <213> Artificial Sequence
<220>
   <223> primer
<400> 404
   gtcagggttc tggatatttg gcttcacagt gagcgtagtc ccatc 45
<210> 405
   <211> 45
   <212> DNA
   <213> Artificial Sequence
<220>
   <223> primer
<400> 405
   gtcagggttc tggatatttg gtatgaccga gagtttggtc ccctt 45
<210> 406
   <211> 47
   <212> DNA
   <213> Artificial Sequence
<220>
   <223> primer
<400> 406
   gtcagggttc tggatatttg caatcacaga aagtcttgtg ccctttc 47
<210> 407
   <211> 47
   <212> DNA
   <213> Artificial Sequence
<220>
   <223> primer
<400> 407
   gtcagggttc tggatattgg ggagaatatg aagtcgtgtc ccttttc 47
<210> 408
   <211> 41
   <212> DNA
   <213> Artificial Sequence
<220>
   <223> primer
<400> 408
   gtcagggttc tggatattgg gcttcaccac cagctgagtt c 41
<210> 409
   <211> 41
   <212> DNA
   <213> Artificial Sequence
<220>
   <223> primer
<400> 409
   gtcagggttc tggatatttg gctggacagc aagcagagtg c 41
<210> 410
   <211> 46
   <212> DNA
   <213> Artificial Sequence
<220>
   <223> primer
<400> 410
   gtcagggttc tggatatctg gctttataat tagcttggtc ccagcg 46
<210> 411
   <211> 47
   <212> DNA
   <213> Artificial Sequence
<220>
   <223> primer
<400> 411
   gtcagggttc tggatatttg gaaagacttg taatctggtc ccagtcc 47
<210> 412
   <211> 46
   <212> DNA
   <213> Artificial Sequence
<220>
   <223> primer
<400> 412
   gtcagggttc tggatatgtg gtaaaacaat cacttgagtg ccggac 46
<210> 413
   <211> 46
   <212> DNA
   <213> Artificial Sequence
<220>
   <223> primer
<400> 413
   gtcagggttc tggatatagg ggaataacgg tgagtctcgt tccagt 46
<210> 414
   <211> 48
   <212> DNA
   <213> Artificial Sequence
<220>
   <223> primer
<400> 414
   gtcagggttc tggatatctg gttttacttg gtaaagttgt cccttgcc 48
<210> 415
   <211> 43
   <212> DNA
   <213> Artificial Sequence
<220>
   <223> primer
<400> 415
   gtcagggttc tggatattcg gatttactgc caggcttgtt ccc 43
<210> 416
   <211> 44
   <212> DNA
   <213> Artificial Sequence
<220>
   <223> primer
<400> 416
   gtcagggttc tggatatggg gtttgaccat taaccttgtt cccc 44
<210> 417
   <211> 44
   <212> DNA
   <213> Artificial Sequence
<220>
   <223> primer
<400> 417
   gtcagggttc tggatatttg ctaaaacctt cagcctggtg cctg 44
<210> 418
   <211> 40
   <212> DNA
   <213> Artificial Sequence
<220>
   <223> primer
<400> 418
   gtcagggttc tggatatggg gtgtgaccaa cagcgaggtg 40
<210> 419
   <211> 53
   <212> DNA
   <213> Artificial Sequence
<220>
   <223> primer
<400> 419
   gtcagggttc tggatatttg gtttaacaga gagtttagtg ccttttccaa aga 53
<210> 420
   <211> 47
   <212> DNA
   <213> Artificial Sequence
<220>
   <223> primer
<400> 420
   gtcagggttc tggatatttg gttttactgt cagtctggtc cctgctc 47
<210> 421
   <211> 45
   <212> DNA
   <213> Artificial Sequence
<220>
   <223> primer
<400> 421
   gtcagggttc tggatatcga gcgtgacctg aagtcttgtt ccagt 45
<210> 422
   <211> 46
   <212> DNA
   <213> Artificial Sequence
<220>
   <223> primer
<400> 422
   gtcagggttc tggatatagg gctggatgat tagatgagtc cctttg 46
<210> 423
   <211> 43
   <212> DNA
   <213> Artificial Sequence
<220>
   <223> primer
<400> 423
   gtcagggttc tggatattgg gcctaactgc taaacgagtc ccg 43
<210> 424
   <211> 46
   <212> DNA
   <213> Artificial Sequence
<220>
   <223> primer
<400> 424
   gtcagggttc tggatatagg acttgactct cagaatggtt cctgcg 46
<210> 425
   <211> 47
   <212> DNA
   <213> Artificial Sequence
<220>
   <223> primer
<400> 425
   gtcagggttc tggatattgg gtatgatggt gagtcttgtt ccagtcc 47
<210> 426
   <211> 45
   <212> DNA
   <213> Artificial Sequence
<220>
   <223> primer
<400> 426
   gtcagggttc tggatatttg gaatgaccgt caaacttgtc cctgt 45
<210> 427
   <211> 46
   <212> DNA
   <213> Artificial Sequence
<220>
   <223> primer
<400> 427
   gtcagggttc tggatatttg gaatgactga taagcttgtc cctggc 46
<210> 428
   <211> 48
   <212> DNA
   <213> Artificial Sequence
<220>
   <223> primer
<400> 428
   gtcagggttc tggatatttg gcttcacagt tagtcatgtc tcctttcc 48
<210> 429
   <211> 45
   <212> DNA
   <213> Artificial Sequence
<220>
   <223> primer
<400> 429
   gtcagggttc tggatatttg gatggacagt caagatggtc ccttg 45
<210> 430
   <211> 48
   <212> DNA
   <213> Artificial Sequence
<220>
   <223> primer
<400> 430
   gtcagggttc tggatatttg gattcacggt taagagagtt ccttttcc 48
<210> 431
   <211> 45
   <212> DNA
   <213> Artificial Sequence
<220>
   <223> primer
<400> 431
   gtcagggttc tggatatttg ggttgatagt cagcctggtt ccttg 45
<210> 432
   <211> 49
   <212> DNA
   <213> Artificial Sequence
<220>
   <223> primer
<400> 432
   gtcagggttc tggatatttg gatttatttt tgtactcatc ccctttccc 49
<210> 433
   <211> 55
   <212> DNA
   <213> Artificial Sequence
<220>
   <223> primer
<400> 433
   gtcagggttc tggatatctg gtctaacact cagagttatt ccttttccaa atgtc 55
<210> 434
   <211> 48
   <212> DNA
   <213> Artificial Sequence
<220>
   <223> primer
<400> 434
   gtcagggttc tggatatatg ggtttactgt cagtttcgtt ccctttcc 48
<210> 435
   <211> 46
   <212> DNA
   <213> Artificial Sequence
<220>
   <223> primer
<400> 435
   gtcagggttc tggatatcag gattcactgt gagctgtgtt ccttcc 46
<210> 436
   <211> 42
   <212> DNA
   <213> Artificial Sequence
<220>
   <223> primer
<400> 436
   gtcagggttc tggatattca ctctcacttg cgtccccatt cc 42
<210> 437
   <211> 46
   <212> DNA
   <213> Artificial Sequence
<220>
   <223> primer
<400> 437
   gtcagggttc tggatatcca ggctcacaat taactcagtc cccttc 46
<210> 438
   <211> 48
   <212> DNA
   <213> Artificial Sequence
<220>
   <223> primer
<400> 438
   gtcagggttc tggatattga gtttcatgat tcctctagtg ttggctcc 48
<210> 439
   <211> 42
   <212> DNA
   <213> Artificial Sequence
<220>
   <223> primer
<400> 439
   caagagggct cggcagaacc tgaagtcacc cagactccca gc 42
<210> 440
   <211> 45
   <212> DNA
   <213> Artificial Sequence
<220>
   <223> primer
<400> 440
   caagagggct cggcagctgt ttcccagact ccaaaatacc tggtc 45
<210> 441
   <211> 45
   <212> DNA
   <213> Artificial Sequence
<220>
   <223> primer
<400> 441
   caagagggct cggcagaagt tacccagaca ccaaaacacc tggtc 45
<210> 442
   <211> 50
   <212> DNA
   <213> Artificial Sequence
<220>
   <223> primer
<400> 442
   caagagggct cggcaggagt cactcaaact ccaagatatc tgatcaaaac 50
<210> 443
   <211> 42
   <212> DNA
   <213> Artificial Sequence
<220>
   <223> primer
<400> 443
   caagagggct cggcaggtgt cactcagacc ccaaaattcc ag 42
<210> 444
   <211> 43
   <212> DNA
   <213> Artificial Sequence
<220>
   <223> primer
<400> 444
   caagagggct cggcaggagt ctcccagtcc ctgagacaca agg 43
<210> 445
   <211> 43
   <212> DNA
   <213> Artificial Sequence
<220>
   <223> primer
<400> 445
   caagagggct cggcaggagt tacgcagaca ccaagacacc tgg 43
<210> 446
   <211> 41
   <212> DNA
   <213> Artificial Sequence
<220>
   <223> primer
<400> 446
   caagagggct cggcaggtgt cactcagacc ccaaaattcc g 41
<210> 447
   <211> 43
   <212> DNA
   <213> Artificial Sequence
<220>
   <223> primer
<400> 447
   caagagggct cggcaggagt tacgcagaca ccaagacacc tgg 43
<210> 448
   <211> 41
   <212> DNA
   <213> Artificial Sequence
<220>
   <223> primer
<400> 448
   caagagggct cggcaggtgt cactcagacc ccaaaattcc g 41
<210> 449
   <211> 42
   <212> DNA
   <213> Artificial Sequence
<220>
   <223> primer
<400> 449
   caagagggct cggcaggagt ctcccagtcc cccagtaaca ag 42
<210> 450
   <211> 40
   <212> DNA
   <213> Artificial Sequence
<220>
   <223> primer
<400> 450
   caagagggct cggcagggat cacccaggca ccaacatctc 40
<210> 451
   <211> 42
   <212> DNA
   <213> Artificial Sequence
<220>
   <223> primer
<400> 451
   caagagggct cggcaggagt ctcccagacc cccagtaaca ag 42
<210> 452
   <211> 41
   <212> DNA
   <213> Artificial Sequence
<220>
   <223> primer
<400> 452
   caagagggct cggcaggagt cacccaaagt cccacacacc t 41
<210> 453
   <211> 41
   <212> DNA
   <213> Artificial Sequence
<220>
   <223> primer
<400> 453
   caagagggct cggcaggagt cacacaaacc ccaaagcacc t 41
<210> 454
   <211> 43
   <212> DNA
   <213> Artificial Sequence
<220>
   <223> primer
<400> 454
   caagagggct cggcagaaat cacccagagc ccaagacaca aga 43
<210> 455
   <211> 46
   <212> DNA
   <213> Artificial Sequence
<220>
   <223> primer
<400> 455
   caagagggct cggcagaagt tgcccagtcc cccagatata agatta 46
<210> 456
   <211> 44
   <212> DNA
   <213> Artificial Sequence
<220>
   <223> primer
<400> 456
   caagagggct cggcaggaat cacccagagc ccaagataca agat 44
<210> 457
   <211> 51
   <212> DNA
   <213> Artificial Sequence
<220>
   <223> primer
<400> 457
   caagagggct cggcaggagt tgcccagtct cccagatata agattataga g 51
<210> 458
   <211> 42
   <212> DNA
   <213> Artificial Sequence
<220>
   <223> primer
<400> 458
   caagagggct cggcaggtgt cactcagacc ccaaaattcc ag 42
<210> 459
   <211> 43
   <212> DNA
   <213> Artificial Sequence
<220>
   <223> primer
<400> 459
   caagagggct cggcaggagt ctcccagtcc ccaaggtaca aag 43
<210> 460
   <211> 41
   <212> DNA
   <213> Artificial Sequence
<220>
   <223> primer
<400> 460
   caagagggct cggcaggagt cacccaaagt cccacacacc t 41
<210> 461
   <211> 41
   <212> DNA
   <213> Artificial Sequence
<220>
   <223> primer
<400> 461
   caagagggct cggcaggtgt cactcagacc ccaaaattcc g 41
<210> 462
   <211> 41
   <212> DNA
   <213> Artificial Sequence
<220>
   <223> primer
<400> 462
   caagagggct cggcaggagt ctcccagtcc ccaaggtacg a 41
<210> 463
   <211> 41
   <212> DNA
   <213> Artificial Sequence
<220>
   <223> primer
<400> 463
   caagagggct cggcaggagt cacccaaagt cccacacacc t 41
<210> 464
   <211> 42
   <212> DNA
   <213> Artificial Sequence
<220>
   <223> primer
<400> 464
   caagagggct cggcaggtgt cactcagacc ccaaaattcc ac 42
<210> 465
   <211> 45
   <212> DNA
   <213> Artificial Sequence
<220>
   <223> primer
<400> 465
   caagagggct cggcaggagt ctcccagtct cccaggtaca aagtc 45
<210> 466
   <211> 41
   <212> DNA
   <213> Artificial Sequence
<220>
   <223> primer
<400> 466
   caagagggct cggcaggagt cacccaaagt cccacacacc t 41
<210> 467
   <211> 44
   <212> DNA
   <213> Artificial Sequence
<220>
   <223> primer
<400> 467
   caagagggct cggcaggtgt cactcagacc ccaaaattcc acat 44
<210> 468
   <211> 45
   <212> DNA
   <213> Artificial Sequence
<220>
   <223> primer
<400> 468
   caagagggct cggcaggagt ctcccagtct cccaggtaca aagtc 45
<210> 469
   <211> 41
   <212> DNA
   <213> Artificial Sequence
<220>
   <223> primer
<400> 469
   caagagggct cggcaggagt cacccaaagt cccacacacc t 41
<210> 470
   <211> 44
   <212> DNA
   <213> Artificial Sequence
<220>
   <223> primer
<400> 470
   caagagggct cggcaggtgt cactcagacc ccaaaattcc acat 44
<210> 471
   <211> 45
   <212> DNA
   <213> Artificial Sequence
<220>
   <223> primer
<400> 471
   caagagggct cggcaggagt ctcccagtcc cctaggtaca aagtc 45
<210> 472
   <211> 43
   <212> DNA
   <213> Artificial Sequence
<220>
   <223> primer
<400> 472
   caagagggct cggcaggagt cacacaaagt cccacacacc tga 43
<210> 473
   <211> 42
   <212> DNA
   <213> Artificial Sequence
<220>
   <223> primer
<400> 473
   caagagggct cggcaggagt ctcccagaac cccagacaca ag 42
<210> 474
   <211> 44
   <212> DNA
   <213> Artificial Sequence
<220>
   <223> primer
<400> 474
   caagagggct cggcaggagt catccagtcc ccaagacatc tgat 44
<210> 475
   <211> 42
   <212> DNA
   <213> Artificial Sequence
<220>
   <223> primer
<400> 475
   caagagggct cggcaggaat cacccagagc ccaagacaca ag 42
<210> 476
   <211> 53
   <212> DNA
   <213> Artificial Sequence
<220>
   <223> primer
<400> 476
   caagagggct cggcaggagt ggttcagtct cccagatata agattataga gaa 53
<210> 477
   <211> 40
   <212> DNA
   <213> Artificial Sequence
<220>
   <223> primer
<400> 477
   caagagggct cggcaggagt tatccagtca ccccgccatg 40
<210> 478
   <211> 39
   <212> DNA
   <213> Artificial Sequence
<220>
   <223> primer
<400> 478
   caagagggct cggcaggagt tatccagtca ccccggcac 39
<210> 479
   <211> 42
   <212> DNA
   <213> Artificial Sequence
<220>
   <223> primer
<400> 479
   caagagggct cggcaagagt cacccagaca ccaaggcaca ag 42
<210> 480
   <211> 42
   <212> DNA
   <213> Artificial Sequence
<220>
   <223> primer
<400> 480
   caagagggct cggcaggagt tactcagttc cccagccaca gc 42
<210> 481
   <211> 45
   <212> DNA
   <213> Artificial Sequence
<220>
   <223> primer
<400> 481
   caagagggct cggcaatggt catccagaac ccaagatacc aggt 45
<210> 482
   <211> 38
   <212> DNA
   <213> Artificial Sequence
<220>
   <223> primer
<400> 482
   caagagggct cggcagagcc tggagtcagc cagacccc 38
<210> 483
   <211> 39
   <212> DNA
   <213> Artificial Sequence
<220>
   <223> primer
<400> 483
   caagagggct cggcaggcgt catgcagaac ccaagacac 39
<210> 484
   <211> 46
   <212> DNA
   <213> Artificial Sequence
<220>
   <223> primer
<400> 484
   caagagggct cggcaggaat cactcagtcc ccaaagtacc tgttca 46
<210> 485
   <211> 41
   <212> DNA
   <213> Artificial Sequence
<220>
   <223> primer
<400> 485
   caagagggct cggcagctgt cgtctctcaa catccgagct g 41
<210> 486
   <211> 39
   <212> DNA
   <213> Artificial Sequence
<220>
   <223> primer
<400> 486
   caagagggct cggcaattcc agctcactgg ggctggatg 39
<210> 487
   <211> 46
   <212> DNA
   <213> Artificial Sequence
<220>
   <223> primer
<400> 487
   caagagggct cggcaaaagt cacacagact ccaggacatt tggtca 46
<210> 488
   <211> 45
   <212> DNA
   <213> Artificial Sequence
<220>
   <223> primer
<400> 488
   caagagggct cggcagatgt tacccagacc ccaaggaata ggatc 45
<210> 489
   <211> 50
   <212> DNA
   <213> Artificial Sequence
<220>
   <223> primer
<400> 489
   caagagggct cggcagacat ctaccagacc ccaagatacc ttgttatagg 50
<210> 490
   <211> 50
   <212> DNA
   <213> Artificial Sequence
<220>
   <223> primer
<400> 490
   caagagggct cggcagtagt tacacaattc ccaagacaca gaatcattgg 50
<210> 491
   <211> 45
   <212> DNA
   <213> Artificial Sequence
<220>
   <223> primer
<400> 491
   caagagggct cggcacaagt gacccagaac ccaagatacc tcatc 45
<210> 492
   <211> 48
   <212> DNA
   <213> Artificial Sequence
<220>
   <223> primer
<400> 492
   caagagggct cggcatcgag atatctagtc aaaaggacgg gagagaaa 48
<210> 493
   <211> 42
   <212> DNA
   <213> Artificial Sequence
<220>
   <223> primer
<400> 493
   caagagggct cggcagctgt catctctcaa aagccaagca gg 42
<210> 494
   <211> 50
   <212> DNA
   <213> Artificial Sequence
<220>
   <223> primer
<400> 494
   aacaccttgt tcaggtcctc tacaactgtg agtctggtgc cttgtccaaa 50
<210> 495
   <211> 46
   <212> DNA
   <213> Artificial Sequence
<220>
   <223> primer
<400> 495
   aacaccttgt tcaggtcctc tacaacggtt aacctggtcc ccgaac 46
<210> 496
   <211> 49
   <212> DNA
   <213> Artificial Sequence
<220>
   <223> primer
<400> 496
   aacaccttgt tcaggtcctc tacaacagtg agccaacttc cctctccaa 49
<210> 497
   <211> 46
   <212> DNA
   <213> Artificial Sequence
<220>
   <223> primer
<400> 497
   aacaccttgt tcaggtcctc caagacagag agctgggttc cactgc 46
<210> 498
   <211> 48
   <212> DNA
   <213> Artificial Sequence
<220>
   <223> primer
<400> 498
   aacaccttgt tcaggtcctc taggatggag agtcgagtcc catcacca 48
<210> 499
   <211> 45
   <212> DNA
   <213> Artificial Sequence
<220>
   <223> primer
<400> 499
   aacaccttgt tcaggtcctc tgtcacagtg agcctggtcc cgttc 45
<210> 500
   <211> 43
   <212> DNA
   <213> Artificial Sequence
<220>
   <223> primer
<400> 500
   aacaccttgt tcaggtcctc tagcacggtg agccgtgtcc ctg 43
<210> 501
   <211> 47
   <212> DNA
   <213> Artificial Sequence
<220>
   <223> primer
<400> 501
   aacaccttgt tcaggtcctc cagtacggtc agcctagagc cttctcc 47
<210> 502
   <211> 42
   <212> DNA
   <213> Artificial Sequence
<220>
   <223> primer
<400> 502
   aacaccttgt tcaggtcctc cagaaccagg agtcctccgc cc 42
<210> 503
   <211> 40
   <212> DNA
   <213> Artificial Sequence
<220>
   <223> primer
<400> 503
   aacaccttgt tcaggtcctc gagcactgtc agccgggtgc 40
<210> 504
   <211> 41
   <212> DNA
   <213> Artificial Sequence
<220>
   <223> primer
<400> 504
   aacaccttgt tcaggtcctc cagcactgag agccgggtcc c 41
<210> 505
   <211> 39
   <212> DNA
   <213> Artificial Sequence
<220>
   <223> primer
<400> 505
   aacaccttgt tcaggtcctc gagcaccagg agccgcgtg 39
<210> 506
   <211> 40
   <212> DNA
   <213> Artificial Sequence
<220>
   <223> primer
<400> 506
   aacaccttgt tcaggtcctc cagcacggtc agcctgctgc 40
<210> 507
   <211> 41
   <212> DNA
   <213> Artificial Sequence
<220>
   <223> primer
<400> 507
   aacaccttgt tcaggtcctc tgtgaccgtg agcctggtgc c 41
<210> 508
   <211> 23
   <212> DNA
   <213> Artificial Sequence
<220>
   <223> primer
<400> 508
   cgtggttaca ggagggctcg gca 23
<210> 509
   <211> 26
   <212> DNA
   <213> Artificial Sequence
<220>
   <223> primer
<400> 509
   cacggcaggg tcagggttct ggatat 26
<210> 510
   <211> 21
   <212> DNA
   <213> Artificial Sequence
<220>
   <223> primer
<400> 510
   tggctccaag agggctcggc a 21
<210> 511
   <211> 26
   <212> DNA
   <213> Artificial Sequence
<220>
   <223> primer
<400> 511
   gggtgggaac accttgttca ggtcct 26